# EUROPEAN PATENT APPLICATION

(11) **EP 4 353 721 A1**
(43) Date of publication of application: **17.04.2024**
(21) Application number: 22806793.0
(22) Date of filing: 11.05.2022
(51) Int. Cl.: C07D 471/04, A61K 31/437, A61P 35/00

(54) **NITROGEN-CONTAINING HETEROCYCLIC COMPOUND**

(30) Priority: 11.05.2021 CN 202110511570; 23.09.2021 CN 202111114252; 25.01.2022 CN 202210086937
(71) Applicant: Evopoint Biosciences Co., Ltd., Suzhou, Jiangsu 215000 (CN)
(72) Inventor: WU, Yuchuan, Beijing 100176 (CN); LIU, Xiao, Beijing 100176 (CN); XIE, Yonghua, Beijing 100176 (CN); CHEN, Xi, Beijing 100176 (CN); HAO, Rui, Beijing 100176 (CN); HU, Yonghan, Suzhou, Jiangsu 215000 (CN)
(74) Representative: Cabinet Laurent & Charras
(86) International application number: PCT/CN2022/092182
(87) International publication number: WO 2022/237830

(57) **Abstract**

A compound as represented by formula I or a pharmaceutically acceptable form thereof, a pharmaceutical composition containing same, and the medical use thereof for preventing and/or treating HPK1-related diseases.

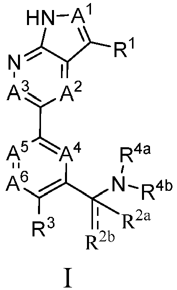

## Description

### TECHNICAL FIELD

The present disclosure belongs to the field of pharmaceutical chemistry, and relates to a nitrogen-containing heterocyclic compound or a pharmaceutically acceptable form thereof, a pharmaceutical composition comprising the same, and medicinal use thereof in preventing and/or treating HPK1-related diseases.

### BACKGROUND

Hematopoietic progenitor kinase 1 (HPK1, also known as MAP4K1), a member of the MAP4K family, is a serine/threonine kinase. HPK1 is mainly expressed in immune cells and plays a role in regulating the function of the immune cells.

In T cells, the activation of a T cell receptor (TCR) signaling pathway causes HPK1 in cytoplasm to be recruited to a cell membrane for binding to an adaptor protein SLP76 and phosphorylating the same, promoting the binding of the SLP76 to E3 ligase 14-3-3, causing the degradation of SLP76/LAT signalosomes, thereby negatively regulating the T cell receptor (TCR) pathway, thus inhibiting the activation of T cells and the function of effector T cells. Compared with wild type, the HPK1-knockout (HPK1^{-/-}) and HPK1 kinase-dead (HPK1 KD) T cells show stronger proliferation ability and higher cytokine expression levels. The mRNA and protein expression levels of HPK1 in CD4+ T cells from patients with systemic lupus erythematosus are also significantly reduced.

HPK1 can control the anti-tumor immune mechanism in a T cell-dependent manner. In HPK1^{-/-} and HPK1 KD tumor-bearing mice, T cells have stronger tumor cell-killing ability, while tumor cells expressing an immunosuppressive molecule PGE2 grow more slowly in HPK1^{-/-} and HPK1 KD mice than in wild-type mice. The analysis of tumor microenvironment of the HPK1 KD mice shows that the main immune cell biomarkers (such as CD4, CD8, IFNγ, Granzyme B, etc.) involved in anti-tumor immunity are significantly improved, the expression of genes related to pro-inflammatory pathways including chemokine CXCL14, etc. is also significantly increased, but the expression of genes related to Th2 and Treg is decreased.

The expression of HPK1 exhibits significant and positive correlation with T cell exhaustion marker PD-1 in 25 human cancers, and has positive correlation with other T cell exhaustion markers such as TIGIT, CTLA-4, LAG3, etc. in various tumors. Reduced expression of HPK1 in low-grade glioma (LGG) and kidney renal clear cell carcinoma (KIRC) is associated with prolonged survival of patients, while HPK1 expansion in pancreatic adenocarcinoma (PAAD) and spreading breast cancer (BRAC) is associated with poor prognosis of patients.

In addition, HPK1 is also a negative regulator for activation of B cells and dendritic cells, and plays an important role in the function maintenance of Treg cells. In conclusion, HPK1 has multifaceted anti-tumor immune promotion effects, being a potential therapeutic target for tumor immunotherapy and autoimmune diseases.

### SUMMARY

Through extensive research, the present inventors found that a nitrogen-containing heterocyclic compound has potential value in preventing and/or treating HPK1-related diseases.

In a first aspect, the present disclosure provides a compound having a structure of formula I or a pharmaceutically acceptable form thereof: 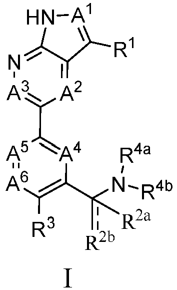 wherein,
A¹ and A³ are each independently selected from N or CR¹⁻¹, and R¹⁻¹ is selected from H, halogen, CN or C₁₋₄ alkyl, wherein the C₁₋₄ alkyl is optionally substituted with one or more halogens;
A² is selected from N or CR²⁻¹, and R²⁻¹ is selected from H, OH, halogen, CN, C₁₋₄ alkyl, C₁₋₆ alkoxy or C₃₋₆ cycloalkyl, wherein the C₁₋₄ alkyl, the C₁₋₆ alkoxy or the C₃₋₆ cycloalkyl is optionally substituted with one or more R³⁻¹, each R³⁻¹ is independently selected from oxo, halogen, OH, CN, C₁₋₆ alkyl, C₁₋₆ alkoxy, C₂₋₆ alkenyl or NR³⁻¹⁻¹R³⁻¹⁻², and R³⁻¹⁻¹ and R³⁻¹⁻² are each independently selected from H or C₁₋₆ alkyl;
A⁴ is selected from N or CR⁴⁻¹, and R⁴⁻¹ is selected from H, halogen, CN, OH, NH₂, -NH-CH₃, -N(CH₃)₂, C₁₋₄ alkyl, C₁₋₄ alkoxy or C₃₋₆ cycloalkyl, wherein the C₁₋₄ alkyl, the C₁₋₄ alkoxy or the C₃₋₆ cycloalkyl is optionally substituted with one or more halogens;
A⁵ is selected from N or CR⁵⁻¹, and R⁵⁻¹ is selected from H, halogen, CN, OH, NH₂, -NH-CH₃, -N(CH₃)₂, C₁₋₄ alkyl, C₁₋₄ alkoxy, C₃₋₆ cycloalkyl, C₂₋₆ alkenyl or C₂₋₆ alkynyl, wherein the C₁₋₄ alkyl, the C₁₋₄ alkoxy, the C₃₋₆ cycloalkyl, the C₂₋₆ alkenyl or the C₂₋₆ alkynyl is optionally substituted with one or more halogens;
A⁶ is selected from N or CR⁶⁻¹, and R⁶⁻¹ is selected from H, halogen, OH, CN, NO₂, oxo, C₁₋₈ alkyl, C₂₋₈ alkenyl, C₂₋₈ alkynyl, C₃₋₈ cycloalkyl, 3- to 8-membered heterocyclyl, 5- to 10-membered heteroaryl, C₆₋₁₀ aryl, C₁₋₆ alkoxy, -OR⁶⁻², -SR⁶⁻², -S(=O)R⁶⁻², -S(=O)₂R⁶⁻², NH₂, - NHR⁶⁻², -NR⁶⁻²R⁶⁻³, -C(=O)R⁶⁻², -C(=O)OR⁶⁻², -C(=O)NHR⁶⁻², -C(=O)NR⁶⁻²R⁶⁻³, -NR⁶⁻²C(=O)R⁶⁻² or -NR⁶⁻²C(=O)NR⁶⁻²R⁶⁻³, wherein R⁶⁻² and R⁶⁻³ are each independently selected from H, C₁₋₈ alkyl, C₂₋₈ alkynyl, C₃₋₈ cycloalkyl, 3- to 8-membered heterocyclyl, 5- to 10-membered heteroaryl or C₆₋₁₀ aryl, wherein the C₁₋₈ alkyl, the C₂₋₈ alkenyl, the C₂₋₈ alkynyl, the C₃₋₈ cycloalkyl, the 3- to 8-membered heterocyclyl, the 5- to 10-membered heteroaryl, the C₆₋₁₀ aryl or the C₁₋₆ alkoxy is optionally substituted with one or more substituents independently selected from H, halogen, CN, oxo, -NR⁶⁻¹⁻¹R⁶⁻¹⁻², -OR⁶⁻¹⁻¹, -SR⁶⁻¹⁻¹, -S(=O)R⁶⁻¹⁻¹ or -S(=O)₂R⁶⁻¹⁻¹, and R⁶⁻¹⁻¹ and R⁶⁻¹⁻² are each independently selected from H, C₁₋₄ alkyl, -C₁₋₄ alkylene-C₁₋₄ alkoxy, C₂₋₆ alkenyl or C₂₋₆ alkynyl;
R¹ is selected from H, halogen, CN, -NR^{6e}R^{6f}, -C(=O)OR⁶⁻², -C(=O)NR^{6g}R^{6h}, C₁₋₄ alkyl, C₃₋₈ cycloalkyl or 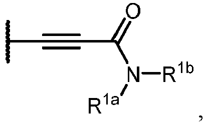 wherein the C₁₋₄ alkyl or the C₃₋₈ cycloalkyl is optionally substituted with one or more substituents independently selected from CN, OH, halogen, CF₃, - NR^{6e}R^{6f} or -C(=O)NR^{6g}R^{6h};
R^{1a} and R^{1b} are each independently selected from H, C₁₋₈ alkyl, C₂₋₈ alkenyl, C₂₋₈ alkynyl, C₃₋₈ cycloalkyl, 3- to 8-membered heterocyclyl, 5- to 10-membered heteroaryl, C₆₋₁₀ aryl, -OR^{1c}, -NR^{1c}R^{1d}, -S(=O)₂R^{1c}, -S(=O)₂NR^{1c}R^{1d}, -C(=O)R^{1c}, -C(=O)NR^{1c}R^{1d}, -NR^{1c}C(=O)R^{1d} or - NR^{1c}S(=O)₂R^{1d};
R^{1c} and R^{1d} are each independently selected from H, C₁₋₈ alkyl, C₂₋₈ alkynyl, C₃₋₈ cycloalkyl, 3- to 8-membered heterocyclyl, 5- to 10-membered heteroaryl or C₆₋₁₀ aryl, wherein the C₁₋₈ alkyl, the C₂₋₈ alkynyl, the C₃₋₈ cycloalkyl, the 3- to 8-membered heterocyclyl, the 5- to 10-membered heteroaryl or the C₆₋₁₀ aryl is optionally substituted with one or more substituents independently selected from H, halogen, CN, oxo, -NR⁶⁻¹⁻¹R⁶⁻¹⁻², -OR⁶⁻¹⁻¹, -SR⁶⁻¹⁻¹, -S(=O)R⁶⁻¹⁻¹ or -S(=O)₂R⁶⁻¹⁻¹, and R⁶⁻¹⁻¹ and R⁶⁻¹⁻² are each independently selected from H, C₁₋₄ alkyl, -C₁₋₄ alkylene-C₁₋₄ alkoxy, C₂₋₆ alkenyl or C₂₋₆ alkynyl;
or, R^{1a} and R^{1b}, together with the atoms to which they are attached, form a 5- to 8-membered ring comprising 1 or 2 heteroatoms independently selected from N, O or S;
R^{6e}, R^{6f}, R^{6g} and R^{6h} are each independently selected from H, -S(=O)₂R⁷, C₃₋₆ cycloalkyl or C₁₋₆ alkyl, wherein the C₃₋₆ cycloalkyl is optionally substituted with one or more substituents independently selected from -OR⁵, Het^{g} or Het^{e}, wherein the C₁₋₆ alkyl is optionally substituted with one or more substituents independently selected from -OR⁵, -NR^{9a}C(=O)R^{9b}, Het^{g} or Het^{e}, and two substituents on the same carbon atom of the C₁₋₆ alkyl optionally together form a C₃₋₆ cycloalkyl or a 3- to 6-membered heterocyclyl comprising 1 or 2 heteroatoms selected from N, O or S;
or, each of pair R^{6e}/R^{6f} and pair R^{6g}/R^{6h}, together with the N atom to which they are attached, form a 5- to 8-membered heterocyclyl;
each Het^{e} is independently a 5- to 12-membered heteroaryl comprising 1 N atom and optionally 1 or 2 additional heteroatoms independently selected from N, O or S, wherein the heteroaryl is optionally substituted with a substituent selected from Het^{f} or C₁₋₄ alkyl, wherein the C₁₋₄ alkyl is optionally substituted with Het^{f};
each Het^{g} is independently a 4- to 12-membered heterocyclyl comprising 1, 2 or 3 heteroatoms independently selected from N, O or S, wherein the heterocyclyl is optionally substituted with a substituent selected from oxo, Het^{f} or C₁₋₄ alkyl, wherein the C₁₋₄ alkyl is optionally substituted with Het^{f};
each Het^{f} is independently a 4- to 12-membered heterocyclyl comprising 1, 2 or 3 heteroatoms independently selected from N, O or S, wherein the heterocyclyl is optionally substituted with C₁₋₄ alkyl;
R^{9a} is selected from H or C₁₋₄ alkyl;
R^{9b} is selected from H or C₁₋₄ alkyl, wherein the C₁₋₄ alkyl is optionally substituted with 1, 2 or 3 halogen atoms;
R^{2a} is H;
R^{2b} is selected from H or methyl;
R^{4a} is selected from H, C₁₋₄ alkyl or C₃₋₆ cycloalkyl;
R^{4b} is selected from H, C₁₋₄ alkyl, C₃₋₆ cycloalkyl, or a 4- to 12-membered heterocyclyl comprising 1, 2 or 3 heteroatoms independently selected from N, O or S;
or, R^{2b} and R^{4b}, together with the atoms to which they are attached, form a 5- to 12-membered heteroaryl or a 4- to 12-membered heterocyclyl, wherein the heteroaryl or the heterocyclyl each comprises 1 N atom and optionally 1 or 2 additional heteroatoms independently selected from N, O or S; wherein on one or more C atoms, the heteroaryl or the heterocyclyl is optionally substituted with 1, 2 or 3 substituents independently selected from OH, CN, halogen, R⁷, -OR⁷, -S(=O)₂R⁷, -C(=O)R⁷, -NR^{6c}R^{6d}, -C(=O)NR^{6a}R^{6b} or Het^{c}; wherein on an optional additional N atom, the heteroaryl or the heterocyclyl is optionally substituted with 1 or 2 substituents independently selected from R⁷, -S(=O)₂R⁷, -C(=O)R⁷, -C(=O)NR^{6a}R^{6b} or Het^{d};
the dotted bond towards R^{2b} is a bond optionally present when R^{2b} and R^{4b} form a ring together;
when R^{2b} and R^{4b} form the ring together, R^{4a} is H; when the dotted bond towards R^{2b} is a bond, R^{2a} is absent; or,
R^{4a} and R^{4b}, together with the N atom to which they are attached, form a 5- to 12-membered heteroaryl or a 4- to 12-membered heterocyclyl, wherein the heteroaryl or the heterocyclyl each comprises 1 N atom and optionally 1 or 2 additional heteroatoms independently selected from N, O or S; wherein on one or more C atoms, the heteroaryl or the heterocyclyl is optionally substituted with 1, 2 or 3 substituents independently selected from OH, CN, halogen, R⁷, -OR⁷, -S(=O)₂R⁷, -C(=O)R⁷, -NR^{6c}R^{6d}, -C(=O)NR^{6a}R^{6b} or Het^{c}; wherein on the N atom, the heteroaryl or the heterocyclyl is optionally substituted with 1 or 2 substituents independently selected from R⁷, -S(=O)₂R⁷, -C(=O)R⁷, -C(=O)NR^{6a}R^{6b} or Het^{d};
when R^{4a} and R^{4b} form the ring together, R^{2a} is H and R^{2b} is H;
each Het^{c} is independently a 4- to 12-membered heterocyclyl comprising 1, 2 or 3 heteroatoms independently selected from N, O or S;
each Het^{d} is independently a 4- to 12-membered heterocyclyl comprising 1, 2 or 3 heteroatoms independently selected from N, O or S;
R³ is selected from CN, halogen, -C(=O)NR^{8a}R^{8b}, -CH₂NR^{8c}R^{8d}, Het^{a}, Het^{b}, -CH₂-Het^{a}, - CH₂-Het^{b}, -CH(R⁷⁻¹)-Het^{a}, -CH(R⁷⁻¹)-Het^{b}, -P(=O)-(C₁₋₄ alkyl)₂, -S(=O)₂-C₁₋₄ alkyl, - S(=O)(=NR^{x})-C₁₋₄ alkyl, C₁₋₆ alkyl or C₃₋₆ cycloalkyl, wherein the C₁₋₆ alkyl or the C₃₋₆ cycloalkyl is optionally substituted with 1 or 2 substituents independently selected from halogen, OH, CN or -O-C₁₋₄ alkyl;
R^{8a}, R^{8c} and R^{8d} are each independently selected from H, C₁₋₄ alkyl or C₃₋₆ cycloalkyl, wherein the C₁₋₄ alkyl or the C₃₋₆ cycloalkyl is optionally substituted with OH or -O-C₁₋₄ alkyl;
R^{8b} is selected from C₁₋₄ alkyl or C₃₋₆ cycloalkyl, wherein the C₁₋₄ alkyl or the C₃₋₆ cycloalkyl is optionally substituted with OH or -O-C₁₋₄ alkyl;
or, pair R^{8a}/R^{8b} or pair R^{8c}/R^{8d}, together with the N atom to which they are attached, form a 4- to 12-membered heterocyclyl, wherein the heterocyclyl comprises 1 N atom and optionally 1 or 2 additional heteroatoms independently selected from N, O or S; wherein on one or more C atoms, the heterocyclyl is optionally substituted with 1, 2 or 3 substituents independently selected from OH, CN, halogen, R⁷, -OR⁷, -S(=O)₂R⁷, -C(=O)R⁷, -NR^{6c}R^{6d} or -C(=O)NR^{6a}R^{6b}; wherein on the N atom, the heterocyclyl is optionally substituted with 1 or 2 substituents independently selected from R⁷, -S(=O)₂R⁷, -C(=O)R⁷ or -C(=O)NR^{6a}R^{6b};
Het^{b} is a 4- to 12-membered heterocyclyl, wherein the heterocyclyl comprises 1, 2 or 3 heteroatoms independently selected from N, O or S; wherein on one or more C atoms, the heterocyclyl is optionally substituted with 1, 2 or 3 substituents independently selected from OH, CN, halogen, R⁷, -OR⁷, -S(=O)₂R⁷, -C(=O)R⁷, -NR^{6c}R^{6d} or -C(=O)NR^{6a}R^{6b}; wherein on the N atom, the heterocyclyl is optionally substituted with 1 or 2 substituents independently selected from R⁷, -S(=O)₂R⁷, -C(=O)R⁷ or -C(=O)NR^{6a}R^{6b};
each R^{x} is independently selected from H or C₁₋₄ alkyl;
each R⁷ is independently selected from C₁₋₄ alkyl or C₃₋₆ cycloalkyl, wherein the C₁₋₄ alkyl or the C₃₋₆ cycloalkyl is optionally substituted with one or more substituents independently selected from halogen, OH, -O-C₁₋₄ alkyl or CN;
R^{6a}, R^{6b}, R^{6c} and R^{6d} are each independently selected from H, C₃₋₆ cycloalkyl or C₁₋₄ alkyl, wherein the C₃₋₆ cycloalkyl or the C₁₋₄ alkyl is optionally substituted with -OR⁵, and two substituents on the same carbon atom of the C₁₋₄ alkyl optionally together form a C₃₋₆ cycloalkyl;
each R⁵ is independently selected from H or C₁₋₄ alkyl;
each R⁷⁻¹ is independently selected from C₁₋₄ alkyl or C₃₋₆ cycloalkyl, wherein the C₁₋₄ alkyl or the C₃₋₆ cycloalkyl is optionally substituted with one or more halogens;
Het^{a} is 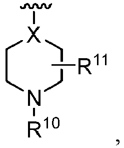 X is selected from N or CH, and R¹⁰ and R¹¹ are each independently selected from H, C₁₋₈ alkyl, C₂₋₈ alkenyl, C₂₋₈ alkynyl or C₃₋₈ cycloalkyl; and
the pharmaceutically acceptable form is selected from pharmaceutically acceptable salts, esters, stereoisomers, tautomers, solvates, oxynitrides, isotopically labeled compounds, metabolites and prodrugs.

In some embodiments, in the compound of formula I or the pharmaceutically acceptable form thereof described above, when R¹ is selected from H, halogen, CN, -NR^{6e}R^{6f}, - C(=O)NR^{6g}R^{6h}, C₁₋₄ alkyl or C₃₋₈ cycloalkyl and when A⁴ is CH, A², A³, A⁵ and A⁶ are not all CH and A² is not N.

In some embodiments, in the compound of formula I or the pharmaceutically acceptable form thereof described above, A¹ and A³ are each independently selected from N or CR¹⁻¹, R¹⁻¹ is selected from H, halogen, CN, methyl, ethyl or isopropyl, wherein the methyl, the ethyl or the isopropyl is optionally substituted with one or more halogens.

In some preferred embodiments, in the compound of formula I or the pharmaceutically acceptable form thereof described above, A¹ and A³ are each independently selected from N or CR¹⁻¹, and R¹⁻¹ is selected from H, halogen, CN or methyl; and preferably, R¹⁻¹ is selected from H, F, Cl, CN or methyl.

In some embodiments, in the compound of formula I or the pharmaceutically acceptable form thereof described above, A² is selected from N or CR²⁻¹, and R²⁻¹ is selected from H, OH, halogen, CN, C₁₋₃ alkyl, C₁₋₃ alkoxy or C₃₋₆ cycloalkyl, wherein the C₁₋₃ alkyl, the C₁₋₃ alkoxy or the C₃₋₆ cycloalkyl is optionally substituted with one or more substituents independently selected from oxo, halogen, OH, CN, NH₂ or methyl.

In some preferred embodiments, in the compound of formula I or the pharmaceutically acceptable form thereof described above, A² is selected from N or CR²⁻¹, and R²⁻¹ is selected from H, OH, halogen, CN, CF₃, methoxy, ethoxy, methyl, ethyl or cyclopropyl; and preferably, R²⁻¹ is selected from H, OH, F, Cl, CN, CF₃, methoxy, ethoxy, methyl, ethyl or cyclopropyl.

In some embodiments, in the compound of formula I or the pharmaceutically acceptable form thereof described above, A⁴ is selected from N or CR⁴⁻¹, R⁴⁻¹ is selected from H, halogen, CN, OH, NH₂, -NH-CH₃, -N(CH₃)₂, C₁₋₃ alkyl, C₁₋₃ alkoxy or C₃₋₆ cycloalkyl, wherein the C₁₋₃ alkyl, the C₁₋₃ alkoxy or the C₃₋₆ cycloalkyl is optionally substituted with one or more halogens.

In some preferred embodiments, in the compound of formula I or the pharmaceutically acceptable form thereof described above, A⁴ is selected from N or CR⁴⁻¹, and R⁴⁻¹ is selected from H, halogen, CN, OH, NH₂, methyl or methoxy; and preferably, R⁴⁻¹ is selected from H, F, Cl, CN, OH, NH₂, methyl or methoxy.

In some embodiments, in the compound of formula I or the pharmaceutically acceptable form thereof described above, A⁵ is selected from N or CR⁵⁻¹, R⁵⁻¹ is selected from H, halogen, CN, OH, NH₂, -NH-CH₃, -N(CH₃)₂, C₁₋₄ alkyl, C₁₋₄ alkoxy or C₃₋₆ cycloalkyl, wherein the C₁₋₄ alkyl, the C₁₋₄ alkoxy or the C₃₋₆ cycloalkyl is optionally substituted with one or more halogens.

In some preferred embodiments, in the compound of formula I or the pharmaceutically acceptable form thereof described above, A⁵ is selected from N or CR⁵⁻¹, and R⁵⁻¹ is selected from H, halogen, CN, OH, NH₂, -NH-CH₃, -N(CH₃)₂, methyl, CF₃, methoxy, trifluoromethoxy or cyclopropyl; and preferably, R⁵⁻¹ is selected from H, F, Cl, CN, OH, NH₂, -NH-CH₃, - N(CH₃)₂, methyl, CF₃, methoxy, trifluoromethoxy or cyclopropyl.

In some embodiments, in the compound of formula I or the pharmaceutically acceptable form thereof described above, A⁶ is selected from N or CR⁶⁻¹, and R⁶⁻¹ is selected from H, halogen, OH, CN, NO₂, oxo, C₁₋₈ alkyl, C₃₋₈ cycloalkyl, -OR⁶⁻², -SR⁶⁻², -S(=O)R⁶⁻², -S(=O)₂R⁶⁻², NH₂, -NHR⁶⁻², -NR⁶⁻²R⁶⁻³, -C(=O)R⁶⁻², -C(=O)OR⁶⁻², -C(=O)NHR⁶⁻², -C(=O)NR⁶⁻²R⁶⁻³, -NR⁶⁻²C(=O)R⁶⁻² or -NR⁶⁻²C(=O)NR⁶⁻²R ⁶⁻³, wherein R⁶⁻² and R⁶⁻³ are each independently selected from H, C₁₋₈ alkyl or C₃₋₈ cycloalkyl, wherein the C₁₋₈ alkyl or the C₃₋₈ cycloalkyl is optionally substituted with one or more substituents independently selected from H, halogen, CN, oxo, - NR⁶⁻¹⁻¹R⁶⁻¹⁻², -OR⁶⁻¹⁻¹, -SR⁶⁻¹⁻¹, -S(=O)R⁶⁻¹⁻¹ or -S(=O)₂R⁶⁻¹⁻¹, and R⁶⁻¹⁻¹ and R⁶⁻¹⁻² are each independently selected from H, methyl or ethyl;
and preferably, R⁶⁻¹ is selected from H, halogen, OH, CN, NO₂, oxo, C₁₋₄ alkyl, C₃₋₆ cycloalkyl, -OR⁶⁻², -SR⁶⁻², -S(=O)R⁶⁻², -S(=O)₂R⁶⁻², NH₂, -NHR⁶⁻², -NR⁶⁻²R⁶⁻³, -C(=O)R⁶⁻², - C(=O)OR⁶⁻², -C(=O)NHR⁶⁻², -C(=O)NR⁶⁻²R⁶⁻³, -NR⁶⁻²C(=O)R⁶⁻² or -NR⁶⁻²C(=O)NR⁶⁻²R⁶⁻³, wherein R⁶⁻² and R⁶⁻³ are each independently selected from H, C₁₋₄ alkyl or C₃₋₆ cycloalkyl, wherein the C₁₋₄ alkyl or the C₃₋₆ cycloalkyl is optionally substituted with one or more substituents independently selected from H, halogen, CN, oxo, -NR⁶⁻¹⁻¹R⁶⁻¹⁻², -OR⁶⁻¹⁻¹, -SR⁶⁻¹⁻¹, -S(=O)R⁶⁻¹⁻¹ or -S(=O)₂R⁶⁻¹⁻¹, and R⁶⁻¹⁻¹ and R⁶⁻¹⁻² are each independently selected from H, methyl or ethyl.

In some preferred embodiments, in the compound of formula I or the pharmaceutically acceptable form thereof described above, A⁶ is selected from N or CR⁶⁻¹, R⁶⁻¹ is selected from H, halogen, OH, CN, NO₂, oxo, C₁₋₄ alkyl, C₃₋₆ cycloalkyl or C₁₋₄ alkoxy, wherein the C₁₋₄ alkyl, the C₃₋₆ cycloalkyl or the C₁₋₄ alkoxy is optionally substituted with one or more substituents independently selected from H, halogen, CN or oxo.

In some more preferred embodiments, in the compound of formula I or the pharmaceutically acceptable form thereof described above, A⁶ is selected from N or CR⁶⁻¹, and R⁶⁻¹ is selected from H, halogen, OH, CN, NO₂, oxo, methyl, ethyl, CF₃, methoxy, trifluoromethoxy or cyclopropyl; and further preferably, R⁶⁻¹ is selected from H, F, Cl, OH, CN, NO₂, methyl, ethyl, CF₃, methoxy, trifluoromethoxy or cyclopropyl.

In some embodiments, in the compound of formula I or the pharmaceutically acceptable form thereof described above, R¹ is selected from H, halogen, CN, -C(=O)OR⁶⁻², C₁₋₄ alkyl, C₃₋₈ cycloalkyl or 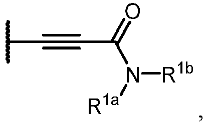 wherein the C₁₋₄ alkyl or the C₃₋₈ cycloalkyl is optionally substituted with one or more substituents independently selected from CN, OH, halogen or CF₃,
R^{1a} and R^{1b} are each independently selected from H, C₁₋₈ alkyl, C₂₋₈ alkenyl, C₂₋₈ alkynyl, C₃₋₈ cycloalkyl, 3- to 8-membered heterocyclyl, 5- to 10-membered heteroaryl or C₆₋₁₀ aryl, and R⁶⁻² is selected from H, C₁₋₈ alkyl or C₃₋₈ cycloalkyl.

In some preferred embodiments, in the compound of formula I or the pharmaceutically acceptable form thereof described above, R¹ is selected from H, halogen, CN, -C(=O)OR⁶⁻², C₁₋₄ alkyl, C₃₋₆ cycloalkyl or 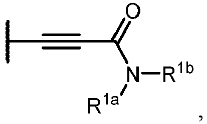 wherein the C₁₋₄ alkyl or the C₃₋₆ cycloalkyl is optionally substituted with one or more substituents independently selected from CN, OH, halogen or CF₃,
R^{1a} and R^{1b} are each independently selected from H, C₁₋₄ alkyl, C₃₋₆ cycloalkyl or 3- to 6-membered heterocyclyl, and R⁶⁻² is selected from H, C₁₋₄ alkyl or C₃₋₆ cycloalkyl.

In some more preferred embodiments, in the compound of formula I or the pharmaceutically acceptable form thereof described above, R¹ is selected from H, halogen, CN, methyl, ethyl, cyclopropyl, -CH₂OH, -C(=O)OCH₃, CF₃, 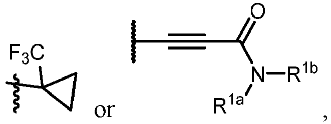 and R^{1a} and R^{1b} are each independently selected from H, methyl or ethyl;
and further preferably, R¹ is selected from H, F, Cl, CN, methyl, ethyl, cyclopropyl, - CH₂OH, -C(=O)OCH₃, CF₃,

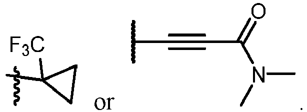

In some embodiments, in the compound of formula I or the pharmaceutically acceptable form thereof described above,
R^{2a} is H,
R^{4a} is H,
R^{2b} and R^{4b}, together with the atoms to which they are attached, form a monocyclic 5-membered heteroaryl, a monocyclic 4-, 5-, 6- or 7-membered heterocyclyl, a bicyclic 6- to 12-membered heteroaryl or a bicyclic 6- to 12-membered heterocyclyl, wherein the heteroaryl or the heterocyclyl each comprises 1 N atom and optionally 1 or 2 additional heteroatoms independently selected from N, O or S, wherein on one or more C atoms, the heteroaryl or the heterocyclyl is optionally substituted with 1, 2 or 3 substituents independently selected from OH, CN, halogen, R⁷, -OR⁷, -S(=O)₂R⁷ or -C(=O)R⁷; wherein on an optional additional N atom, the heteroaryl or the heterocyclyl is optionally substituted with 1 or 2 substituents independently selected from R⁷, -S(=O)₂R⁷ or -C(=O)R⁷,
R⁷ is independently selected from C₁₋₄ alkyl or C₃₋₆ cycloalkyl, wherein the C₁₋₄ alkyl or the C₃₋₆ cycloalkyl is optionally substituted with one or more substituents independently selected from halogen, OH, -O-C₁₋₄ alkyl or CN.

In some preferred embodiments, in the compound of formula I or the pharmaceutically acceptable form thereof described above, R^{2b} and R^{4b}, together with the atoms to which they are attached, form a monocyclic 4-, 5-, 6- or 7-membered heterocyclyl, wherein the heterocyclyl comprises 1 N atom and optionally 1 or 2 additional heteroatoms independently selected from N, O or S, wherein on one or more C atoms, the heterocyclyl is optionally substituted with 1, 2 or 3 substituents independently selected from OH, CN, halogen, R⁷, -OR⁷, -S(=O)₂R⁷ or - C(=O)R⁷; wherein on an optional additional N atom, the heterocyclyl is optionally substituted with 1 or 2 substituents independently selected from R⁷, -S(=O)₂R⁷ or -C(=O)R⁷,
and R⁷ is independently selected from methyl, ethyl or cyclopropyl.

In some more preferred embodiments, in the compound of formula I or the pharmaceutically acceptable form thereof described above, R^{2b} and R^{4b}, together with the atoms to which they are attached, form a monocyclic 6-membered heterocyclyl, wherein the heterocyclyl comprises 1 N atom and optionally 1 or 2 additional heteroatoms independently selected from N, O or S, wherein on one or more C atoms, the heterocyclyl is optionally substituted with 1, 2 or 3 substituents independently selected from OH, CN, halogen, methyl, ethyl or cyclopropyl; wherein on an optional additional N atom, the heterocyclyl is optionally substituted with 1 or 2 substituents independently selected from methyl, ethyl or cyclopropyl.

In some particularly preferred embodiments, in the compound of formula I or the pharmaceutically acceptable form thereof described above, R^{2b} and R^{4b}, together with the atoms to which they are attached, form morpholinyl, piperidinyl or piperazinyl.

In some embodiments, in the compound of formula I or the pharmaceutically acceptable form thereof described above, R^{2a} is H, R^{2b} is H, R^{4a} is selected from H, C₁₋₄ alkyl or C₃₋₆ cycloalkyl, and R^{4b} is selected from H, C₁₋₄ alkyl or C₃₋₆ cycloalkyl; and preferably, R^{4a} is methyl, and R^{4b} is methyl.

In some embodiments, in the compound of formula I or the pharmaceutically acceptable form thereof described above, R^{2a} is H, R^{2b} is H, and R^{4a} and R^{4b}, together with the N atom to which they are attached, form a 4- to 12-membered heterocyclyl, wherein the heterocyclyl comprises 1 N atom and optionally 1 or 2 additional heteroatoms independently selected from N, O or S, wherein on one or more C atoms, the heterocyclyl is optionally substituted with 1, 2 or 3 substituents independently selected from OH, CN, halogen, R⁷, -OR⁷, -S(=O)₂R⁷ or - C(=O)R⁷; preferably, R^{4a} and R^{4b}, together with the N atom to which they are attached, form a pyrrolidinyl, wherein on one or more C atoms, the pyrrolidinyl is optionally substituted with 1, 2 or 3 substituents independently selected from OH, CN, halogen, R⁷, -OR⁷, -S(=O)₂R⁷ or - C(=O)R⁷; and more preferably, R^{4a} and R^{4b}, together with the N atom to which they are attached, form

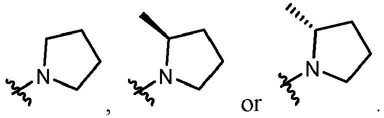

In some embodiments, in the compound of formula I or the pharmaceutically acceptable form thereof described above, R³ is selected from CN, halogen, Het^{a}, Het^{b}, -CH₂-Het^{a}, -CH₂-Het^{b}, -CH(R⁷⁻¹)-Het^{a}, -CH(R⁷⁻¹)-Het^{b}, -P(=O)-(C₁₋₄ alkyl)₂, -S(=O)₂-C₁₋₄ alkyl, C₁₋₆ alkyl or C₃₋₆ cycloalkyl, wherein the C₁₋₆ alkyl or the C₃₋₆ cycloalkyl is optionally substituted with 1 or 2 substituents independently selected from halogen, OH, CN or -O-C₁₋₄ alkyl,
Het^{b} is selected from a monocyclic 4-, 5-, 6- or 7-membered heterocyclyl or a bicyclic 6- to 12-membered heterocyclyl, wherein the heterocyclyl comprises 1, 2 or 3 heteroatoms independently selected from N, O or S, wherein on one or more C atoms, the heterocyclyl is optionally substituted with 1, 2 or 3 substituents independently selected from OH, CN, halogen, methyl or ethyl,
Het^{a} is 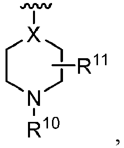 X is selected from N or CH, R¹⁰ and R¹¹ are each independently selected from H, C₁₋₈ alkyl, C₂₋₈ alkenyl, C₂₋₈ alkynyl or C₃₋₈ cycloalkyl,
R⁷⁻¹ is independently selected from C₁₋₄ alkyl or C₃₋₆ cycloalkyl, wherein the C₁₋₄ alkyl or the C₃₋₆ cycloalkyl is optionally substituted with one or more halogens.

In some preferred embodiments, in the compound of formula I or the pharmaceutically acceptable form thereof described above, R³ is selected from CN, halogen, Het^{b}, -CH₂-Het^{b}, - CH(R⁷⁻¹)-Het^{b}, -P(=O)-(C₁₋₄ alkyl)₂, -S(=O)₂-C₁₋₄ alkyl, C₁₋₄ alkyl or C₃₋₆ cycloalkyl, wherein the C₁₋₄ alkyl or the C₃₋₆ cycloalkyl is optionally substituted with 1 or 2 substituents independently selected from halogen, OH, CN or -O-C₁₋₄ alkyl,
Het^{b} is selected from a monocyclic 6-membered heterocyclyl, the heterocyclyl comprising 1, 2 or 3 heteroatoms independently selected from N, O or S, wherein on one or more C atoms, the heterocyclyl is optionally substituted with 1, 2 or 3 substituents independently selected from OH, CN, halogen, methyl or ethyl,
R⁷⁻¹ is independently selected from C₁₋₄ alkyl or C₃₋₆ cycloalkyl, wherein the C₁₋₄ alkyl or the C₃₋₆ cycloalkyl is optionally substituted with one or more halogens.

In some more preferred embodiments, in the compound of formula I or the pharmaceutically acceptable form thereof described above, R³ is selected from CN, halogen, Het^{b}, -CH₂-Het^{b}, - P(=O)-(C₁₋₄ alkyl)₂, -S(=O)₂-C₁₋₄ alkyl, C₁₋₄ alkyl or C₃₋₆ cycloalkyl, and Het^{b} is selected from a monocyclic 6-membered heterocyclyl, wherein the heterocyclyl comprises 1, 2 or 3 heteroatoms independently selected from N, O or S.

In some particularly preferred embodiments, in the compound of formula I or the pharmaceutically acceptable form thereof described above, R³ is selected from CN, halogen, 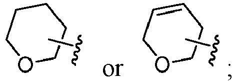 and further preferably, R³ is selected from CN, F, Cl, 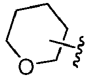 or

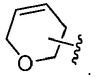

In some embodiments, the compound of formula I or the pharmaceutically acceptable form thereof described above is a compound having a structure of formula I-1 or formula I-2 or a pharmaceutically acceptable form thereof: 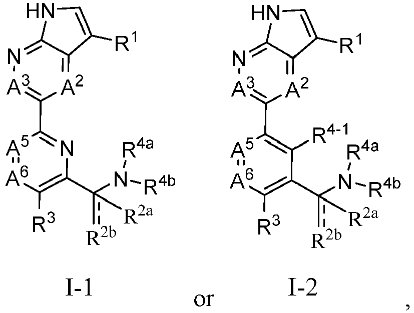
wherein A², A³, A⁵, A⁶, R¹, R⁴⁻¹, R^{2a}, R^{2b}, R^{4a}, R^{4b} and R³ are as defined in formula I,
provided that when R¹ is selected from H, halogen, CN, -NR^{6e}R^{6f}, -C(=O)NR^{6g}R^{6h}, C₁₋₄ alkyl or C₃₋₈ cycloalkyl and when R⁴⁻¹ is H, A², A³, A⁵ and A⁶ are not all CH and A² is not N.

In some embodiments, the compound of formula I or the pharmaceutically acceptable form thereof described above is a compound having a structure of formula I-3 or formula I-4 or a pharmaceutically acceptable form thereof: 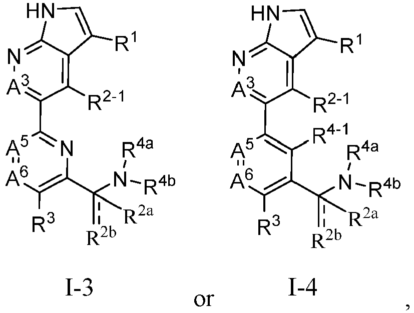
wherein A³, A⁵, A⁶, R¹, R²⁻¹, R⁴⁻¹, R^{2a}, R^{2b}, R^{4a}, R^{4b} and R³ are as defined in formula I,
provided that when R¹ is selected from H, halogen, CN, -NR^{6e}R^{6f}, -C(=O)NR^{6g}R^{6h}, C₁₋₄ alkyl or C₃₋₈ cycloalkyl and when R⁴⁻¹ is H, A³, A⁵ and A⁶ are not all CH, or R²⁻¹ is not H.

In some embodiments, the compound of formula I or the pharmaceutically acceptable form thereof described above is a compound having a structure selected from formula I-5 to formula I-7 or a pharmaceutically acceptable form thereof: 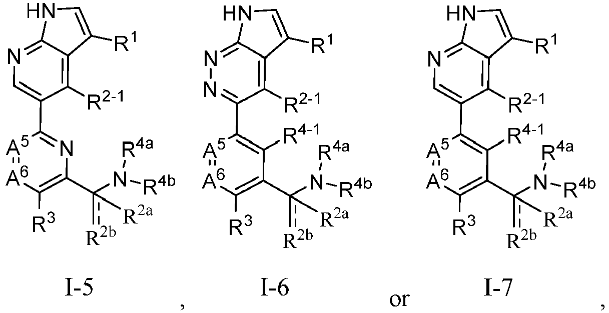
wherein A⁵, A⁶, R¹, R²⁻¹, R⁴⁻¹, R^{2a}, R^{2b}, R^{4a}, R^{4b} and R³ are as defined in formula I,
provided that in formula I-7, when R¹ is selected from H, halogen, CN, -NR^{6e}R^{6f}, - C(=O)NR^{6g}R^{6h}, C₁₋₄ alkyl or C₃₋₈ cycloalkyl and when R⁴⁻¹ is H, A⁵ and A⁶ are not all CH, or R²⁻¹ is not H.

In some embodiments, the compound of formula I or the pharmaceutically acceptable form thereof described above is a compound having a structure selected from formula I-8 to formula I-10 or a pharmaceutically acceptable form thereof: 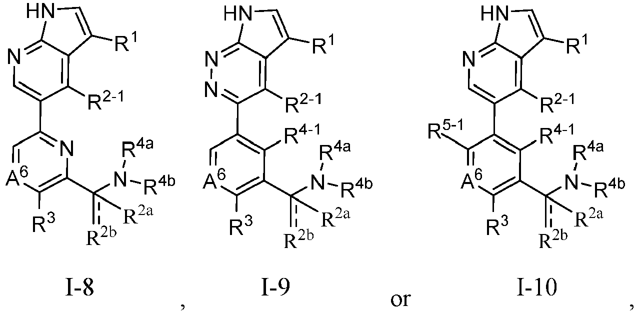
wherein A⁶, R¹, R²⁻¹, R⁴⁻¹, R⁵⁻¹, R^{2a}, R^{2b}, R^{4a}, R^{4b} and R³ are as defined in formula I,
provided that in formula I-10, when R¹ is selected from H, halogen, CN, -NR^{6e}R^{6f}, - C(=O)NR^{6g}R^{6h}, C₁₋₄ alkyl or C₃₋₈ cycloalkyl and when R⁴⁻¹ is H, A⁶ is not CH, or R²⁻¹ is not H, or R⁵⁻¹ is not H.

In some embodiments, the compound of formula I or the pharmaceutically acceptable form thereof described above is a compound having a structure selected from formula I-11 to formula I-13 or a pharmaceutically acceptable form thereof: 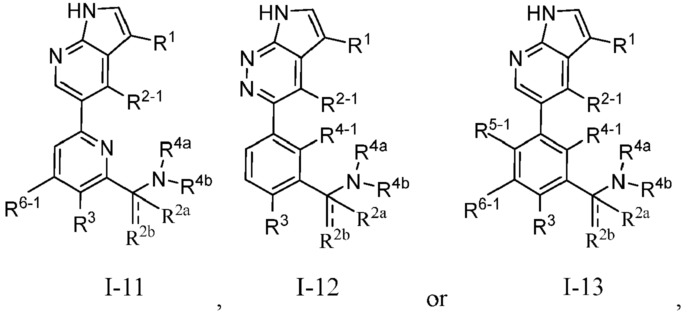
wherein R¹, R²⁻¹, R⁴⁻¹, R⁵⁻¹, R⁶⁻¹, R^{2a}, R^{2b}, R^{4a}, R^{4b} and R³ are as defined in formula I,
provided that in formula I-13, when R¹ is selected from H, halogen, CN, -NR^{6e}R^{6f}, - C(=O)NR^{6g}R^{6h}, C₁₋₄ alkyl, or C₃₋₈ cycloalkyl and when R⁴⁻¹ is H, R²⁻¹ is not H, or R⁵⁻¹ is not H, or R⁶⁻¹ is not H.

In some embodiments, the compound of formula I or the pharmaceutically acceptable form thereof described above is a compound having a structure of formula I-13-1 or a pharmaceutically acceptable form thereof: 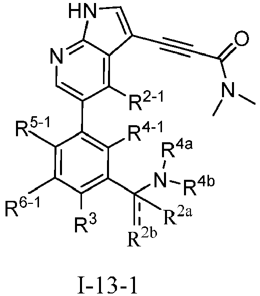 wherein R²⁻¹, R⁴⁻¹, R⁵⁻¹, R⁶⁻¹, R^{2a}, R^{2b}, R^{4a}, R^{4b} and R³ are as defined in formula I.

In some embodiments, the compound of formula I or the pharmaceutically acceptable form thereof described above is a compound having a structure selected from formula 1-14 to formula I-16 or a pharmaceutically acceptable form thereof: 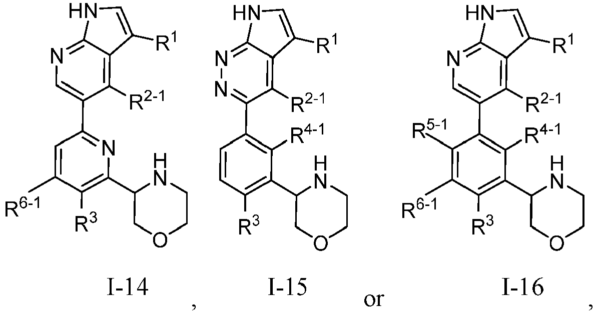
wherein R¹, R²⁻¹, R⁴⁻¹, R⁵⁻¹, R⁶⁻¹ and R³ are as defined in formula I,
provided that in formula I-16, when R¹ is selected from H, halogen, CN, -NR^{6e}R^{6f}, - C(=O)NR^{6g}R^{6h}, C₁₋₄ alkyl, or C₃₋₈ cycloalkyl and when R⁴⁻¹ is H, R²⁻¹ is not H, or R⁵⁻¹ is not H, or R⁶⁻¹ is not H.

In some embodiments, the compound of formula I or the pharmaceutically acceptable form thereof described above is a compound having a structure selected from formula I-17 to formula I-20 or a pharmaceutically acceptable form thereof: 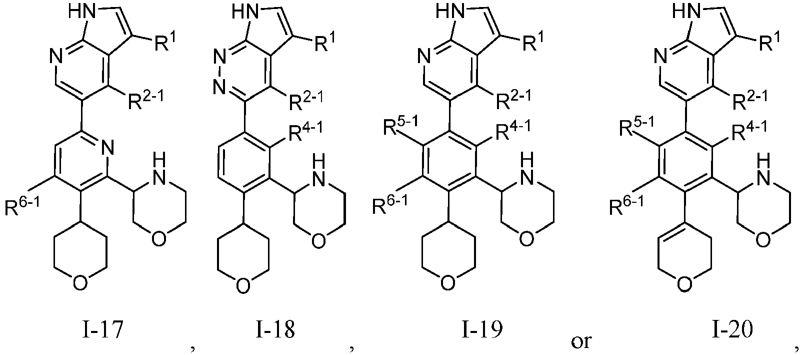
wherein R¹, R²⁻¹, R⁴⁻¹, R⁵⁻¹ and R⁶⁻¹ are as defined in formula I,
provided that in formula I-19 and formula I-20, when R¹ is selected from H, halogen, CN, -NR^{6e}R^{6f}, -C(=O)NR^{6g}R^{6h}, C₁₋₄ alkyl or C₃₋₈ cycloalkyl and when R⁴⁻¹ is H, R²⁻¹ is not H, or R⁵⁻¹ is not H, or R⁶⁻¹ is not H.

It will be understood by those skilled in the art that the present disclosure encompasses the compounds resulting from an arbitrary combination of various embodiments. An embodiment resulting from a combination of the technical features or preferred technical features in one embodiment with the technical features or preferred technical features in another embodiment is also within the scope of the present disclosure.

In a second aspect, the present disclosure further provides the following compounds, or pharmaceutically acceptable salts, esters, stereoisomers, tautomers, solvates, oxynitrides, isotopically labeled compounds, metabolites or prodrugs thereof: 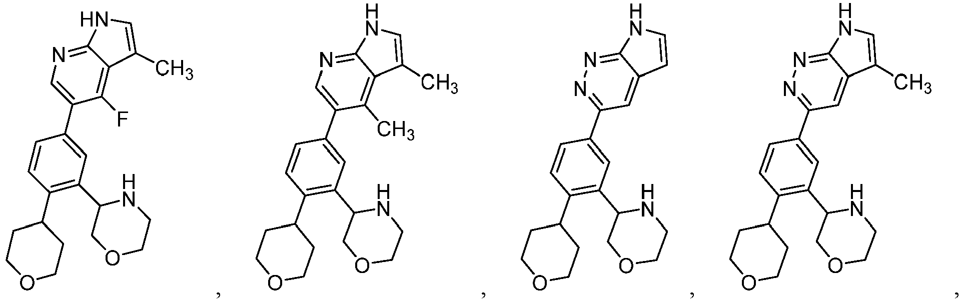 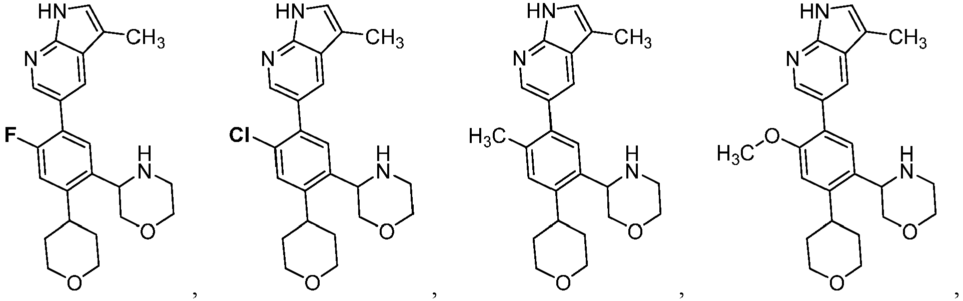 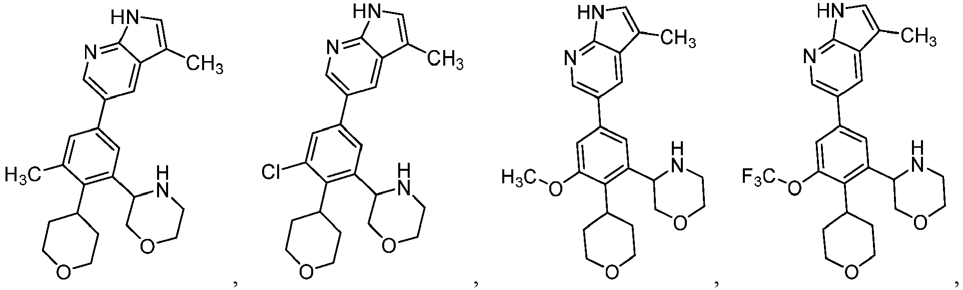 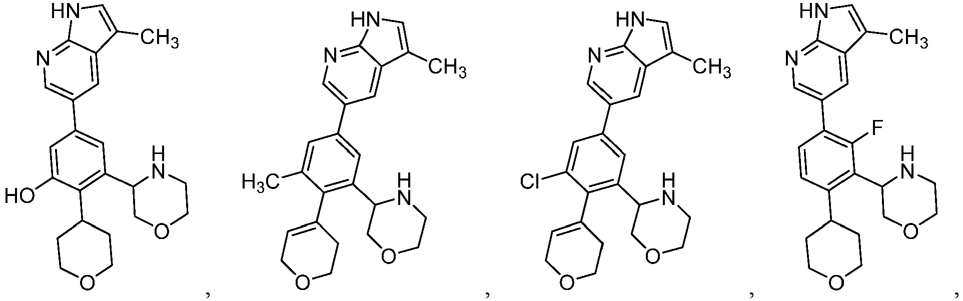 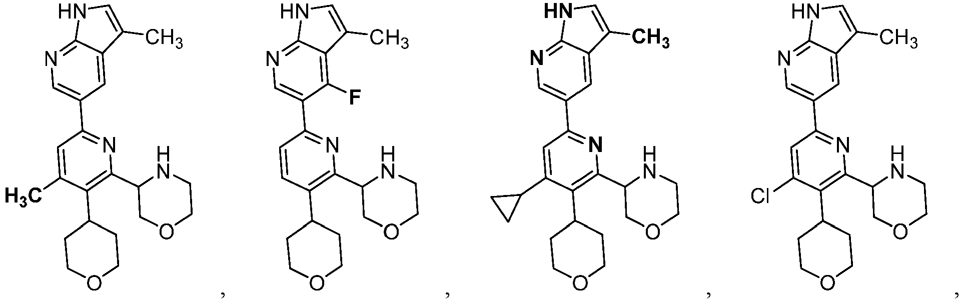 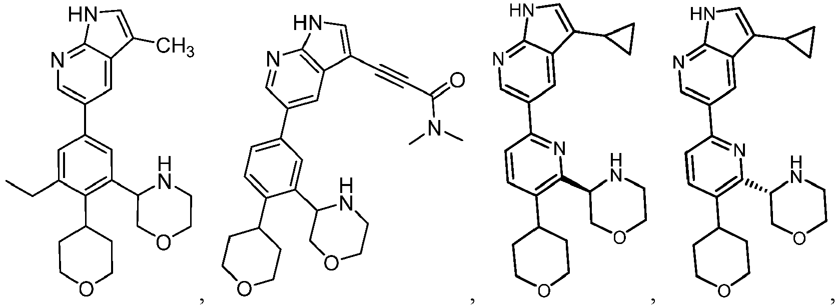 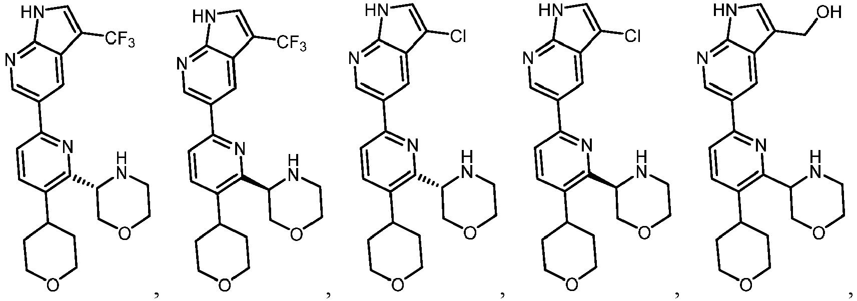 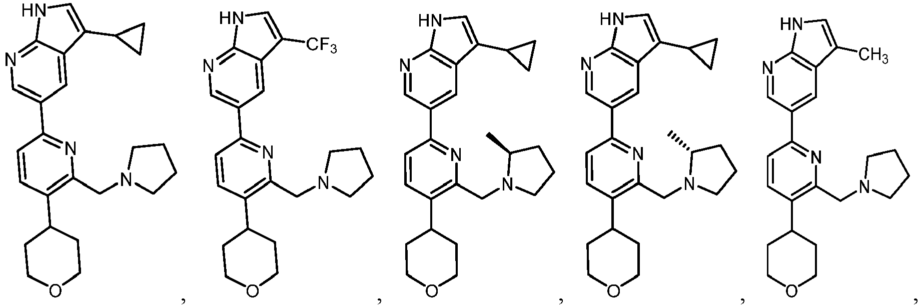 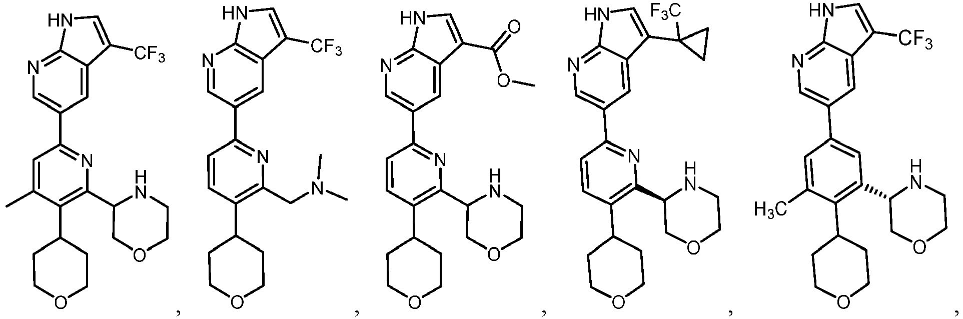 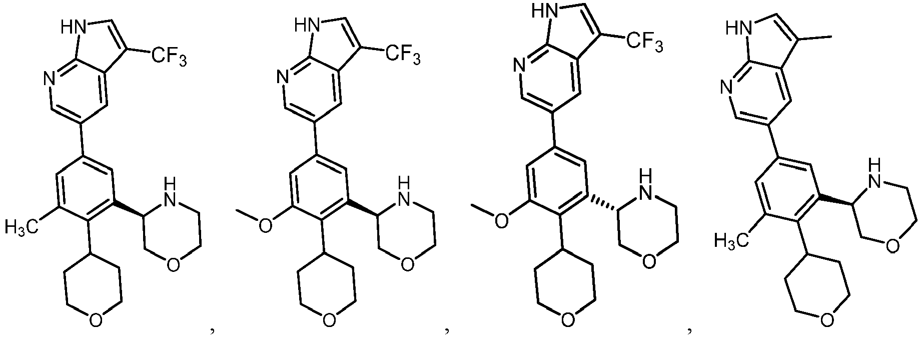 or

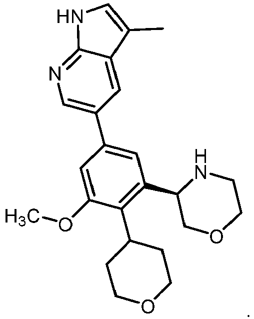

In a third aspect, the present disclosure provides a pharmaceutical composition comprising at least one of the compound of formula I, the compounds of formula I-1 to formula I-20 or the pharmaceutically acceptable forms thereof described above, and one or more pharmaceutically acceptable carriers.

In a fourth aspect, the present disclosure provides the compound of formula I, the compounds of formula I-1 to formula I-20 or the pharmaceutically acceptable forms thereof described above, or the pharmaceutical composition described above, for use in the prevention and/or treatment of a disease or disorder mediated at least in part by HPK1.

In a fifth aspect, the present disclosure provides use of the compound of formula I, the compounds of formula I-1 to formula I-20 or the pharmaceutically acceptable forms thereof described above, or the pharmaceutical composition described above in preparing a medicament for preventing and/or treating a disease or disorder mediated at least in part by HPK1.

In a sixth aspect, the present disclosure provides a method for preventing and/or treating a disease or disorder mediated at least in part by HPK1, comprising the following steps: administering to an individual in need thereof a prophylactically and/or therapeutically effective amount of the compound of formula I, the compounds of formula I-1 to formula I-20 or the pharmaceutically acceptable forms thereof described above, or the pharmaceutical composition described above.

The present disclosure is not limited to the particular embodiments described herein; and it is also to be understood that the terms used herein are intended only to describe but not to limit particular embodiments.

### Definition of terms

The following terms have the following meanings in the present disclosure, unless otherwise specified.

The term "comprise", "include", "have" or "contain" or any other variation thereof is intended to encompass non-exclusive or open-ended contents. For example, a composition, method or device comprising a series of elements is not necessarily limited to those explicitly listed, but may also comprise other elements not explicitly listed or elements inherent to the above-described composition, method or device.

When the lower limit and the upper limit of a numerical range are disclosed, any value or sub-range within the range is intended to be specifically disclosed. In particular, each of the numerical ranges (e.g., in the form of "about a to b", or equivalently "approximately a to b", or equivalently "about a-b") of the parameters disclosed herein is to be understood to encompass each of the values and sub-ranges therein. For example, "C₁₋₄" should be understood to encompass any sub-range and each point value therein, such as C₂₋₄, C₃₋₄, C₁₋₂, C₁₋₃, C₁₋₄, etc., as well as C₁, C₂, C₃, C₄, etc.

The term "pharmaceutical composition" refers to a composition that can be used as a medicament, comprising a pharmaceutically active ingredient (or therapeutic agent) and optionally one or more pharmaceutically acceptable carriers. The term "pharmaceutically acceptable carrier" refers to an auxiliary material administered together with a therapeutic agent, which is suitable, within the scope of sound medical judgment, for contact with the tissues of humans and/or other animals without undue toxicity, irritation, allergic response, or other problems or complications commensurate with a reasonable benefit/risk ratio. The pharmaceutically acceptable carrier that can be used in the present disclosure includes, but is not limited to, a) diluents; b) lubricants; c) binders; d) disintegrants; e) absorbents, colorants, flavoring agents, and/or sweeteners; f) emulsifiers or dispersants; and/or g) substances for enhancing absorption of the compounds, etc.

The pharmaceutical composition described above may act systemically and/or topically. For this purpose, it can be administered by a suitable route, e.g., by parenteral, topical, intravenous, oral, subcutaneous, intra-arterial, intradermal, transdermal, rectal, intracranial, intraperitoneal, intranasal, or intramuscular route, or administrated as an inhalant.

The administration routes described above can be achieved by suitable dosage forms. The dosage forms that can be used in the present disclosure include, but are not limited to: tablets, capsules, pastilles, hard candies, pulvis, sprays, creams, ointments, suppositories, gels, pastes, lotions, aqueous suspensions, injectable solutions, elixirs, syrups, etc.

When being administered orally, the pharmaceutical composition described above may be formulated into any orally acceptable dosage form, including, but not limited to, tablets, capsules, aqueous solutions, aqueous suspensions, etc.

The pharmaceutical composition described above may also be administered in the form of sterile injections, including sterile injectable aqueous or oil suspensions, or sterile injectable aqueous or oil solutions. The carriers that can be used include, but are not limited to: water, Ringer's solution and isotonic sodium chloride solution. In addition, sterilized non-volatile oil can also be used as a solvent or suspending medium, such as monoglyceride or diglyceride.

The pharmaceutical composition may comprise 0.01 mg to 1000 mg of at least one of the compound of formula I, the compounds of formula I-1 to formula I-20 or the pharmaceutically acceptable forms thereof described above.

The term "disease or disorder mediated at least in part by HPK1" refers to a disease in which the pathogenesis includes at least part of factors associated with HPK1, such as non-small cell lung cancer, small cell lung cancer, squamous cell carcinoma, head and neck cancer, oral cancer, pharyngeal cancer, thyroid cancer, esophageal cancer, stomach cancer, gastrointestinal stromal tumor, liver cancer, colon cancer, rectal cancer, villous adenoma of large intestine, breast cancer, breast ductal carcinoma, ovarian cancer, peritoneal cancer, endometrial cancer, corpus uteri cancer, cervical cancer, kidney cancer, renal pelvis cancer, prostate cancer, bladder cancer, neurofibromatosis, osteocarcinoma, brain cancer, testicular cancer, glioma, skin cancer, melanoma, cytoma and sarcoma, multiple myeloma, leukemia, non-Hodgkin's lymphoma or myelodysplastic syndrome.

The term "effective amount" refers to a dose capable of inducing a biological or medical response in a cell, tissue, organ or organism (e.g., an individual) and sufficient to achieve the desired prophylactic and/or therapeutic effect.

The optimal desired response can be achieved by adjusting the dosing regimen. For example, the drug can be administered in a single dose, administered in divided doses over time, or administered after a proportional reduction or increase in dose as appropriate. It is understood that, for any particular individual, the specific dosing regimen should be adjusted as needed and according to the professional judgment by the person administering the composition or supervising the administration of the composition.

The term "in need thereof' refers to a judgment by a physician or other paramedics that an individual needs or will benefit from a prophylactic and/or therapeutic procedure, and the judgment is made based on various factors of the physician or other paramedics in their area of expertise.

The term "individual" (or referred to as subject) refers to a human or non-human animal. The individual of the present disclosure includes an individual (patient) with a disease and/or disorder and a normal individual. The non-human animal of the present disclosure includes all vertebrates, such as non-mammals (such as birds, amphibians, reptiles, etc.), and mammals (such as non-human primates, livestock and/or domesticated animals (e.g., sheep, dogs, cats, cows, pigs, etc.)).

The term "treat/treating/treatment" refers to alleviating or eliminating a targeted disease or disorder. If a subject receives a therapeutic amount of the compound or the pharmaceutically acceptable form thereof of the present disclosure or the pharmaceutical composition of the present disclosure and then at least one index and symptom of the subject shows observable and/or detectable alleviation and/or amelioration, it indicates that the subject has been successfully "treated". It is understood that the treatment includes not only complete treatment, but also incomplete treatment, but achieves some biologically or medically relevant results. Specifically, "treat/treating/treatment" means that the compound or the pharmaceutically acceptable form thereof of the present disclosure or the pharmaceutical composition of the present disclosure can achieve at least one of the following effects, for example: (1) preventing the occurrence of a disease in an animal that may have a tendency towards the disease but has not yet experienced or exhibited the pathology or symptomatology of the disease; (2) inhibiting a disease (i.e., preventing further progression of pathology and/or symptomatology) in an animal that is experiencing or exhibiting the pathology or symptomatology of the disease; and (3) ameliorating a disease (i.e., reversing pathology and/or symptomatology) in an animal that is experiencing or exhibiting the pathology or symptomatology of the disease.

The term "pharmaceutically acceptable salt" refers to a salt of the compound of the present disclosure that is substantially non-toxic to an organism. The pharmaceutically acceptable salt generally includes, but is not limited to, a salt formed by a reaction of the compound of the present disclosure with a pharmaceutically acceptable inorganic/organic acid or inorganic/organic base, such salt also being known as an acid addition salt or base addition salt. For a review of suitable salts, please see, for example, Jusiak, Soczewinski, et al., Remington's Pharmaceutical Sciences [M], Mack Publishing Company, 2005 and Stahl, Wermuth, Handbook of Pharmaceutical Salts: Properties, Selection, and Use [M], Wiley-VCH, 2002**.** Methods for preparing the pharmaceutically acceptable salts of the compounds of the present disclosure are known to those skilled in the art.

The term "pharmaceutically acceptable ester" refers to an ester that is substantially non-toxic to an organism and that can be hydrolyzed in an organism to form the compound of the present disclosure or a salt thereof. The pharmaceutically acceptable ester generally includes, but is not limited to, an ester formed by the compound of the present disclosure with a pharmaceutically acceptable carboxylic acid or sulfonic acid, such ester also being known as a carboxylate or sulfonate.

The term "isomer" refers to the compounds that have the same molecular weight because of the same number and type of atoms, but differ in the spatial arrangement or configuration of the atoms.

The term "stereoisomer" (or "optical isomer") refers to a stable isomer having a perpendicular asymmetric plane due to the presence of at least one chiral element (including a chiral center, a chiral axis a chiral plane, etc.), thereby enabling rotation of plane-polarized light. Since the compounds of the present disclosure have asymmetric centers and other chemical structures that may lead to stereoisomerism, the present disclosure also includes these stereoisomers and mixtures thereof. Unless otherwise indicated, all stereoisomeric forms of the compounds of the present disclosure are within the scope of the present disclosure.

The term "tautomer" (or "tautomeric form") refers to structural isomers with different energies that can be interconverted through a low energy barrier. A chemical equilibrium of tautomers can be achieved if tautomerism is possible (e.g., in a solution). For example, proton tautomers (or proton transfer tautomers) interconvert in a manner including, but not limited to, proton transfer, such as keto-enol isomerization, imine-enamine isomerization, and amide-imino alcohol isomerization. Unless otherwise indicated, all tautomeric forms of the compounds of the present disclosure are within the scope of the present disclosure.

The term "solvate" refers to a substance formed by the association of the compound of the present disclosure (or a pharmaceutically acceptable salt thereof) with at least one kind of solvent molecule through non-covalent intermolecular forces. For example, solvates include, but are not limited to, hydrates (including hemihydrates, monohydrates, dihydrates, trihydrates, etc.), ethanolates, acetonates, etc.

The term "oxynitride" refers to a compound formed by oxidation of a nitrogen atom in the structure of tertiary amine or a nitrogen-containing (aromatic) heterocyclic compound. For example, a nitrogen atom in the parent nucleus of the compound of formula I can form a corresponding oxynitride.

The term "isotopically labeled compound" refers to a derivative compound formed by replacing a particular atom in the compound of the present disclosure with its isotopic atom. Unless otherwise indicated, the compound of the present disclosure includes various isotopes of H, C, N, O, F, P, S and Cl, such as, but not limited to, ²H(D), ³H(T), ¹³C, ¹⁴C, ¹⁵N, ¹⁷O, ¹⁸O, ¹⁸F, ³¹P, ³²P, ³⁵S, ³⁶S and ³⁷Cl.

The term "metabolite" refers to a derivative compound formed by metabolism of the compound of the present disclosure. Further information on metabolism can be found in Goodman and Gilman's: The Pharmacological Basis of Therapeutics (9th ed.) [M], McGraw-Hill international Editions, 1996. The present disclosure encompasses all possible metabolite forms of the compounds of the present disclosure, i.e., substances formed in the individual to whom the compound of the present disclosure is administered. Metabolites of the compounds can be identified by techniques well known in the art, and their activities can be experimentally characterized.

The term "prodrug" refers to a derivative compound capable of providing, directly or indirectly, the compound of the present disclosure upon administration to an individual. Particularly preferred derivative compounds or prodrugs are the compounds that, when administered to an individual, can increase the bioavailability of the compounds of the present disclosure (e.g., more readily absorbed into the blood), or the compounds that facilitate delivery of the parent compound to the site of action (e.g., the lymphatic system). Unless otherwise indicated, all prodrug forms of the compounds of the present disclosure are within the scope of the present disclosure, and various prodrug forms are known in the art, see, e.g., T. Higuchi, V. Stella, Pro-drugs as Novel Drug Delivery Systems [J], American Chemical Society, Vol. 14, 1975**.** In addition, the present disclosure further encompasses the compounds of the present disclosure containing a protective group. In any process of preparing the compounds of the present disclosure, it may be necessary and/or desirable to protect sensitive or reactive groups on any of the molecules concerned, thereby forming a chemically protected form of the compounds of the present disclosure. This may be realized by conventional protective groups, such as those described in T. W. Greene, P. G. M. Wuts, Protective Groups in Organic Synthesis [M], John Wiley & Sons, 2006**.** These protective groups may be removed at an appropriate subsequent stage by methods known in the art.

The term "each independently" means that at least two groups (or ring systems) with the same or similar value ranges in the structure may have the same or different meanings under a certain circumstance. For example, if a substituent X and a substituent Y are each independently hydrogen, halogen, hydroxyl, cyano, alkyl or aryl, then when the substituent X is hydrogen, the substituent Y may be either hydrogen or halogen, hydroxyl, cyano, alkyl or aryl; similarly, when the substituent Y is hydrogen, the substituent X may be either hydrogen or halogen, hydroxyl, cyano, alkyl or aryl.

The term "substituted" refers to that one or more (e.g., 1, 2, 3 or 4) atoms (e.g., hydrogen atoms) or atomic groups (e.g., trifluoromethanesulfonate groups) on a specified group are substituted with other atoms or atomic groups, provided that the specified group satisfies the valence requirements in the present case and forms a stable compound after substitution. A combination of substituents and/or variables is permissible only if such combination is capable of forming a stable compound. If a substituent is described as "optionally substituted", the substituent may be unsubstituted or substituted. If the first substituent is described as being optionally substituted with one or more substituents in the list of second substituents, one or more hydrogen atoms in the first substituent may be individually or each independently substituted with one or more substituents in the list of second substituents, or may be unsubstituted.

When used herein alone or in combination with an additional group, the term "halogen" refers to fluorine (F), chlorine (Cl), bromine (Br) or iodine (I).

When used herein alone or in combination with an additional group, the term "alkyl" refers to a linear or branched aliphatic hydrocarbyl. For example, the term "C₁₋₄ alkyl" as used in the present disclosure refers to an alkyl having 1 to 4 carbon atoms. For example, the alkyl includes, but is not limited to, methyl, ethyl, *n*-propyl, isopropyl, *n*-butyl, *sec*-butyl, isobutyl, *tert*-butyl or the like. The alkyl may be optionally substituted or unsubstituted.

When used herein alone or in combination with an additional group, the term "alkylene" refers to a linear or branched divalent aliphatic hydrocarbyl, and the two groups (or fragments) attached to it may be attached either to the same carbon atom or to different carbon atoms. For example, the term "C₁₋₄ alkylene" as used herein refers to an alkylene (such as methylene, 1,1-ethylidene, 1,2-ethylidene, 1,2-propylidene, 1,3-butylidene, etc.) having 1 to 4 carbon atoms. The alkylene may be optionally substituted or unsubstituted.

When used herein alone or in combination with an additional group, the term "alkoxy" refers to an alkyl that is attached to the rest of a molecule through an oxygen atom. For example, the term "C₁₋₆ alkoxy" as used herein refers to an alkoxy having 1 to 6 carbon atoms. For example, the alkoxy includes, but is not limited to, methoxy, ethoxy, *n*-propoxy, isopropoxy, *n-*butoxy, isobutoxy, *tert*-butoxy, etc. The alkoxy may be optionally substituted or unsubstituted.

When used herein alone or in combination with an additional group, the term "cycloalkyl" refers to a saturated or partially saturated, monocyclic or polycyclic (e.g., bicyclic) non-aromatic hydrocarbyl. For example, the term "C₃₋₈ cycloalkyl" as used in the present disclosure refers to a cycloalkyl having 3 to 8 carbon atoms. Common cycloalkyl includes, but is not limited to, monocyclic cycloalkyl such as cyclopropyl, cyclobutyl, cyclopentyl, cyclohexyl, cycloheptyl, cyclooctyl, cyclononyl, etc.; or bicyclic cycloalkyl, including fused rings, bridged rings or spiro rings, such as bicyclo[1.1.1]pentyl, bicyclo[2.2.1]heptyl, bicyclo[3.2.1]otyl, bicyclo[5.2.0]nonyl, decahydronaphthyl, etc. The cycloalkyl in the present disclosure is optionally substituted with one or more of the substituents described herein.

When used herein alone or in combination with an additional group, the term "heterocyclyl" refers to a saturated or partially saturated, monocyclic or polycyclic (e.g., bicyclic, such as fused cyclic, bridged cyclic or spiro cyclic) non-aromatic group, ring atoms of which consist of carbon atoms and at least one heteroatom selected from N, O and S, wherein the S atom is optionally substituted to form S(=O), S(=O)₂ or S(=O)(=NR^{x}), with R^{x} independently selected from H or C₁₋₄ alkyl. If the valence requirements are met, the heterocyclyl can be attached to the rest moiety of the molecule through any one of the ring atoms. For example, the term "3- to 8-membered heterocyclyl" as used in the present disclosure refers to a heterocyclyl having 3 to 8 ring atoms. Common heterocyclyl includes, but is not limited to, oxiranyl, aziridinyl, azetidinyl, oxetanyl, tetrahydrofuryl, dioxolyl, pyrrolidinyl, pyrrolidinonyl, imidazolidinyl, pyrazolidinyl, tetrahydropyranyl, piperidinyl, piperazinyl, morpholinyl, thiomorpholinyl, dithianyl or trithianyl. The heterocyclyl in the present disclosure is optionally substituted with one or more of the substituents described herein.

When used herein alone or in combination with an additional group, the term "aryl" refers to a monocyclic or fused polycyclic, aromatic hydrocarbyl having a conjugated π-electron system. For example, the term "C₆₋₁₀ aryl" as used in the present disclosure refers to an aryl having 6 to 10 carbon atoms. Common aryl includes, but is not limited to, phenyl, naphthyl, anthracyl, phenanthryl, acenaphthenyl, azulenyl, fluorenyl, indenyl, pyrenyl, etc. The aryl in the present disclosure is optionally substituted with one or more of the substituents described herein.

When used herein alone or in combination with an additional group, the term "heteroaryl" refers to a monocyclic or fused polycyclic aromatic group having a conjugated π-electron system, ring atoms of which consist of carbon atoms and at least one heteroatom selected from N, O and S. If the valence requirements are met, the heteroaryl can be attached to the rest moiety of the molecule through any one of the ring atoms. For example, the term "5- to 10-membered heteroaryl" as used in the present disclosure refers to a heteroaryl having 5 to 10 ring atoms. Common heteroaryl includes, but is not limited to, thienyl, furyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidyl, pyrazinyl, triazinyl and benzo derivatives thereof, pyrrolopyridyl, pyrrolopyrizinyl, pyrazolopyridyl, imidazopyridyl, pyrrolopyrimidinyl, pyrazolopyrimidinyl, purinyl, etc. The heteroaryl in the present disclosure is optionally substituted with one or more of the substituents (e.g., halogen, C₁₋₆ alkyl, etc.) described herein.

When used herein alone or in combination with an additional group, the term "alkenyl" refers to a linear or branched, aliphatic hydrocarbyl having at least one C=C double bond. For example, the term "C₂₋₆ alkenyl" as used in the present disclosure refers to an alkenyl having 2 to 6 carbon atoms. Common alkenyl includes, but is not limited to, vinyl, propenyl, *n*-butenyl, 3-methylbut-2-enyl, *n*-pentenyl, *n*-octenyl, *n*-decenyl, etc. The alkenyl in the present disclosure is optionally substituted with one or more of the substituents described herein.

When used herein alone or in combination with an additional group, the term "alkynyl" refers to a linear or branched, aliphatic hydrocarbyl having at least one C≡C triple bond. For example, the term "C₂₋₆ alkynyl" as used in the present disclosure refers to an alkynyl having 2 to 6 carbon atoms. Common alkynyl includes, but is not limited to, ethynyl, 2-propynyl, 2-butynyl, 1,3-butadiynyl, etc. The alkynyl in the present disclosure is optionally substituted with one or more of the substituents described herein.

When used herein alone or in combination with an additional group, the term "oxo" refers to =O.

### DETAILED DESCRIPTION

In order to render the objective and technical solutions of the present disclosure clearer, the embodiments of the present disclosure will be described in detail below with reference to the examples. Those skilled in the art will understand, however, that the following examples are intended only to illustrate the present disclosure, and should not be considered to limit the scope of the present disclosure.

The reagents or instruments used in the examples are all conventional products that are commercially available. Procedures without specified conditions shall be conducted according to conventional conditions or conditions recommended by the manufacturers. The term "room temperature" as used in the present disclosure refers to 20 °C±5 °C. When used to modify a numerical value or numerical range, the term "about" as used in the present disclosure means to include the numerical value or numerical range as well as an error range acceptable to those skilled in the art for the numerical value or numerical range, for example, the error range is ±10%, ±5%, ±4%, ±3%, ±2%, ±1%, ±0.5%, etc.

The structures of the compounds described in the following examples were determined by nuclear magnetic resonance (NMR) and/or mass spectrometry (MS).

Nuclear magnetic resonance (NMR) was determined using a Bruker 400 MHz nuclear magnetic resonance instrument, the solvents for the determination were deuterated methanol (CD₃OD), deuterated chloroform (CDCl₃) and hexadeuterated dimethyl sulfoxide (DMSO-d₆), and the internal standard substance was tetramethylsilane (TMS).

The abbreviations in the nuclear magnetic resonance (NMR) data in the following examples have the following meanings:
s: singlet; d: doublet; t: triplet; q: quartet; dd: double doublet; qd: quartet doublet; ddd: double double doublet; ddt: double double triplet; dddd: double double double doublet; m: multiplet; br: broad; J: coupling constant; Hz: Hertz; δ: chemical shift.

All chemical shift (δ) values are given in parts per million (ppm).

Mass spectrometry (MS) was determined using an Agilent 6120B mass spectrometer, and an electrospray ion source (ESI) was used as an ion source.

### Synthesis of compounds

### Example 1 Synthesis of Compound A-1 and Compound A-2

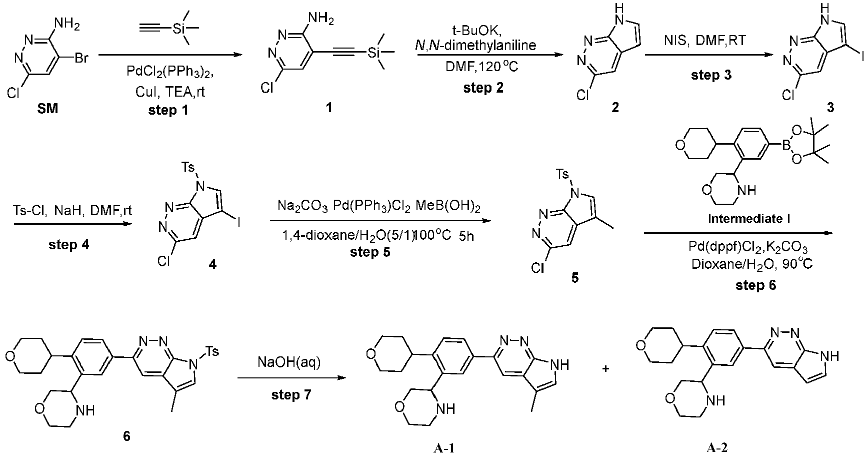

Compound SM (10 g, 47.975 mmol, 1.00 equiv), compound (trimethylsilyl)acetylene (5.18 g, 52.773 mmol, 1.1 equiv), copper(I) iodide (0.91 g, 4.798 mmol, 0.1 equiv), PdCl₂(PPh₃)₂ (3.37 g, 4.798 mmol, 0.1 equiv) and triethylamine (12.14 g, 119.938 mmol, 2.5 equiv) were dissolved in toluene (125 mL), and the solution was allowed to react at room temperature under nitrogen protection for 3 h. After the reaction was completed, water (75 mL) was added at room temperature for quenching. The reaction system was filtered, and the filtrate was extracted with ethyl acetate (3 × 50 mL). The organic phases were combined, washed with saturated brine (3 × 50 mL), then dried over anhydrous sodium sulfate, and filtered. The filtrate was concentrated to give **compound 1** as a brown solid crude product (14 g). m/z (ES+), [M+H]⁺ = 226.

**Compound 1** (14 g, 62.016 mmol, 1.00 equiv), potassium *tert*-butoxide (10.44 g, 93.024 mmol, 1.5 equiv) and dimethylaniline (1.50 g, 12.403 mmol, 0.2 equiv) were dissolved in DMF (150 mL), and the solution was allowed to react at 120 °C under nitrogen protection for 2 h. After the reaction was completed, the reaction solution was cooled to room temperature. A saturated aqueous ammonium chloride solution was added at room temperature for quenching, and the reaction system was filtered. The filter cake was washed with ethyl acetate (3 × 50 mL), and the filtrate was extracted with ethyl acetate (2 × 50 mL). The organic phases were combined, washed with saturated brine (3 × 50 mL), and then dried over anhydrous sodium sulfate. The anhydrous sodium sulfate was filtered off, the filtrate was concentrated, and the crude product was purified by column chromatography to give **compound 2** as an orange-yellow oil (1.5 g, yield: 15.75%). m/z (ES+), [M+H]⁺ = 154.

**Compound 2** (1.5 g, 9.768 mmol, 1.00 equiv) and compound N-iodosuccinimide (2.31 g, 10.256 mmol, 1.05 equiv) were dissolved in DMF (15 mL), and the solution was allowed to react at 30 °C under nitrogen protection for 2 h. After the reaction was completed, the system was cooled to room temperature, and water was added to quench the reaction. The mixture was extracted with ethyl acetate (2 × 20 mL), and the organic phases were combined, washed with saturated brine (3 × 20 mL), and then dried over anhydrous sodium sulfate. The anhydrous sodium sulfate was filtered off, the filtrate was concentrated, and the crude product was purified by column chromatography to give **compound 3** as a yellow solid (2 g, yield: 73.27%). m/z (ES+), [M+H]⁺ = 280.

**Compound 3** (2 g, 7.156 mmol, 1.00 equiv) was dissolved in DMF (20 mL), and sodium hydride (60%, 0.86g) was added at 0 °C. The reaction system was stirred at room temperature under nitrogen protection for 30 min. Subsequently, 4-methylbenzenesulfonyl chloride (2.05 g, 10.734 mmol, 1.5 equiv) was added, and the mixture was stirred at room temperature for 1 h. After the reaction was completed, a saturated aqueous ammonium chloride solution was added at 0 °C for quenching, and the mixture was extracted with ethyl acetate (3 × 20 mL). The organic phases were combined, washed with saturated brine (3 × 20 mL), and then dried over anhydrous sodium sulfate. The anhydrous sodium sulfate was filtered off, the filtrate was concentrated, and the crude product was purified by column chromatography to give **compound 4** as a yellow solid (2.9 g, yield: 93.45%). m/z (ES+), [M+H]⁺ = 434.

**Compound 4** (500 mg, 1.153 mmol, 1.00 equiv) and methylboronic acid (103.53 mg, 1.730 mmol, 1.5 equiv) were dissolved in 1,4-dioxane/water (5 mL:1 mL), then Pd(PPh₃)Cl₂ (80.93 mg, 0.115 mmol, 0.1 equiv) and sodium carbonate (366.62 mg, 3.459 mmol, 3 equiv) were added, and the reaction system was allowed to react at 100 °C under nitrogen protection for 5 h. After the reaction was completed, the mixture was cooled to room temperature and concentrated, and the crude product was purified by column chromatography to give **compound 5** as a yellow oil (60 mg, yield: 16.17%). m/z (ES+), [M+H]⁺ = 322.

**Compound 5** (60 mg, 0.186 mmol, 1.00 equiv) and **intermediate I** (69.61 mg, 0.186 mmol, 1 equiv) were dissolved in 1,4-dioxane/H₂O (1 mL:0.2 mL), then Pd(dppf)Cl₂·CH₂Cl₂ (15.19 mg, 0.019 mmol, 0.1 equiv) and potassium carbonate (77.31 mg, 0.558 mmol, 3 equiv) were added to the reaction system, and the reaction mixture was allowed to react at 90 °C under nitrogen protection overnight. After the reaction was completed, the mixture was cooled to room temperature, concentrated, and subjected to column chromatography to give **compound 6** as a yellow oil (36 mg, yield: 36.25%). m/z (ES+), [M+H]⁺ = 533.

**Compound 6** (30 mg, 0.028 mmol, 1.00 equiv) and a sodium hydroxide solution (2 M, 0.5 mL) were dissolved in methanol (1 mL), and the reaction system was allowed to react at 65 °C under nitrogen protection for 2 h. After the reaction was completed, the system was cooled to room temperature. The reaction system was extracted with ethyl acetate (3 × 20 mL), and the organic phases were combined, washed with saturated brine (3 × 20 mL), and then dried over anhydrous sodium sulfate. The anhydrous sodium sulfate was filtered off, the filtrate was concentrated, and the resulting crude product was purified by high performance liquid chromatography (column: Xselect CSH C18 OBD column, 30 × 150 mm, 5 µm; mobile phase A: water (0.1% FA), mobile phase B: ACN; flow rate: 60 mL/min; gradient: 3% B to 14% B within 10 min, 14% B; wavelength: 254/220 nm; RT1 (min): 8.83, 9.15 (min)) to give **compound A-1** as an off-white solid and **compound A-2** as a white solid.
Compound A-1 (0.8 mg, yield: 3.55%). m/z (ES+), [M+H]⁺ =379. ¹H NMR (400 MHz, CD₃OD*d*₄): δ (ppm) 8.33 (s, 1H), 8.25 (s, 1H), 8.10 (d, *J* = 8.6 Hz, 1H), 7.68 (d, *J* = 8.6 Hz, 1H), 7.67 (s, 1H), 4.23 - 4.05 (m, 5H), 4.02 - 3.92 (m, 3H), 3.74 (t, *J=* 11.6 Hz, 3H), 3.62 - 3.44 (m, 3H), 2.43 (s, 3H), 2.08 - 1.84 (m, 4H).
Compound A-2 (4.2 mg, yield: 19.6%). m/z (ES+), [M+H]⁺ = 365. ¹H NMR (400 MHz, CD₃OD*d*₄): δ (ppm) 8.51 (s, 1H), 8.36 (s, 1H), 8.19 (s, 1H), 8.04 (d, *J=* 8.2 Hz, 1H), 7.93 (s, 1H), 7.68 (d, *J* = 8.3 Hz, 1H), 6.76 (s, 1H), 4.44-3.82 (m, 6H), 3.74 (t, *J=* 12.0 Hz, 2H), 3.60-3.49 (m, 2H), 3.25 (t, *J=* 12.2 Hz, 1H), 1.97 - 2.00 (m, 3H), 1.73 (d, *J* = 13.5 Hz, 1H).

### Example 2 Synthesis of Compound A-3

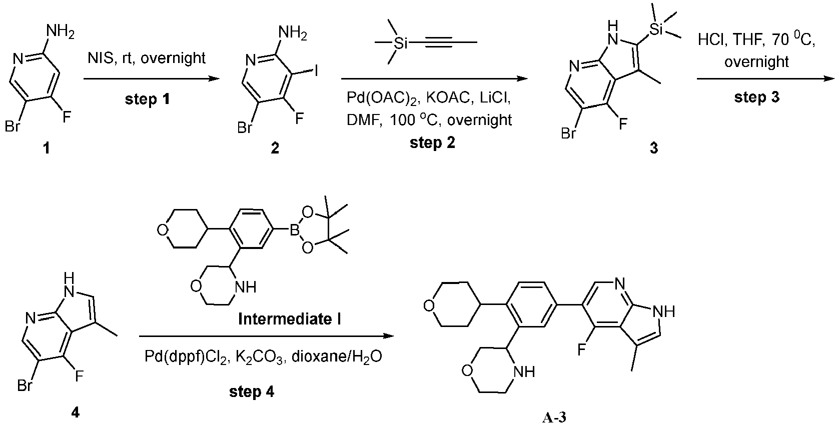

In a 250 mL reaction flask, **compound** 1 (5 g, 26.178 mmol, 1.00 equiv) and *N-*iodosuccinimide (6.18 g, 27.487 mmol, 1.05 equiv) were dissolved in acetic acid (60 mL), then trifluoroacetic acid (1 mL, 13.463 mmol, 0.51 equiv) was added at room temperature under nitrogen, and the mixture was stirred at room temperature overnight. After the reaction was completed, the reaction solution was poured into ice water for quenching. The mixture was neutralized with an ammonium hydroxide solution and the pH was adjusted to 8-9. The precipitated solid was filtered under vacuum, and the filter cake was washed with water and then purified by column chromatography to give **compound 2** as a pale pink solid (2.95 g, yield: 35.4%). m/z (ES+), [M+H]⁺ = 317.

In a 100 mL reaction flask, **compound 2** (2.75 g, 8.675 mmol, 1.00 equiv) and trimethylsilylpropyne (4.87 g, 43.375 mmol, 5 equiv) were dissolved in DMF (10 mL), then potassium acetate (2.55 g, 26.025 mmol, 3 equiv), palladium acetate (0.19 g, 0.868 mmol, 0.1 equiv) and lithium chloride (0.37 g, 8.675 mmol, 1 equiv) were added, and the reaction mixture was stirred at 100 °C under nitrogen overnight. After the reaction was completed, the reaction solution was purified by reversed-phase column chromatography to give **compound 3** as a brown oil (515 mg, yield: 19.7%). m/z (ES+), [M+H]⁺ = 302.

In a 40 mL sealed tube, **compound 3** (500 mg, 1 equiv), tetrahydrofuran (6 mL) and hydrochloric acid (2 mol/L, 6 mL) were added, and the reaction system was stirred at 70 °C overnight. The reaction solution was poured into ice water, the pH was adjusted to 7 with a saturated aqueous sodium bicarbonate solution, and the mixture was extracted with ethyl acetate. The organic phases were combined and concentrated under reduced pressure, and the resulting crude product was purified by column chromatography to give **compound 4** as a yellow solid (160 mg, yield: 42.2%). m/z (ES+), [M+H]⁺ = 230.

In an 8 mL sealed tube, a mixed solution of **compound 4** (68 mg, 0.297 mmol, 1.00 equiv), boronic ester **intermediate I** (132.99 mg, 0.356 mmol, 1.2 equiv), potassium carbonate (123.99 mg, 0.891 mmol, 3 equiv), Pd(dppf)Cl₂ (21.72 mg, 0.030 mmol, 0.1 equiv), 1,4-dioxane/water (3 mL, 10/1) was added, and the reaction mixture was stirred at 80 °C under nitrogen overnight. After the reaction was completed, the reaction solution was concentrated under reduced pressure, and the resulting mixture was purified by thin layer chromatography (dichloromethane:methanol = 12:1) to give a crude product, which was further purified by preparative high-pressure liquid chromatography (column: YMC-Actus Triart C18 ExRS, 30 × 150 mm, 5 µm; mobile phase A: water (10 mmol/L NH₄HCO₃), mobile phase B: ACN; flow rate: 60 mL/min; gradient: 25% B to 55% B within 8 min, 55% B; wavelength: 254/220 nm; RT1 (min): 7.82) to give compound **A-3** as a white solid (8 mg, yield: 7.7%). m/z (ES+), [M+H]⁺ = 396.

¹H NMR (DMSO-*d*₆, 400 MHz): δ (ppm) 1.57 (d, *J*=13.0 Hz, 1H), 1.62 - 1.85 (m, 3H), 2.37 (s, 3H), 2.97 (d, *J*=12.0 Hz, 1H), 3.02 - 3.20 (m, 2H), 3.35 (t, *J*=10.8 Hz, 1H), 3.54-3.60 (m, 3H), 3.77-3.78 (m, 1H), 3.82 (d, *J*=11.5 Hz, 1H), 3.95 (d, *J*=10.7 Hz, 2H), 4.29 (dd, *J*=10.4, 3.1 Hz, 1H), 7.04 - 7.33 (m, 1H), 7.41 (d, *J*=8.1 Hz, 1H), 7.44 - 7.53 (m, 1H), 7.76 (s, 1H), 8.22 - 8.24 (m, 2H).

¹⁹F NMR (376 MHz, DMSO-*d*₆): δ (ppm) -122.51.

### Example 3 Synthesis of Compound A-4

**The synthesis method of compound 1 was similar to that of intermediate compound 4 in Example 2.**

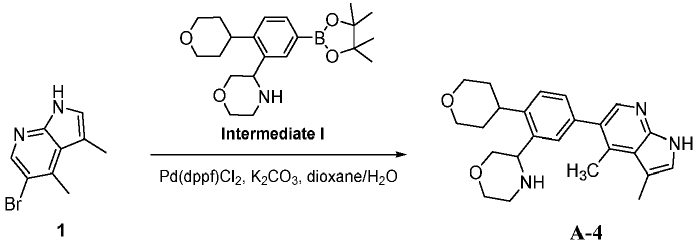

In an 8 mL sealed tube, **compound 1** (100 mg, 0.444 mmol, 1.00 equiv), **intermediate I** (497.54 mg, 1.332 mmol, 3 equiv), potassium carbonate (184.20 mg, 1.332 mmol, 3 equiv) and Pd(dppf)Cl₂CH₂Cl₂ (36.19 mg, 0.044 mmol, 0.1 equiv) were dissolved in a 1,4-dioxane/water solution (5 mL/0.5 mL), and the reaction system was stirred at 80 °C under nitrogen protection overnight. After the reaction was completed, the system was concentrated and purified by thin layer chromatography (dichloromethane:methanol = 12:1) to give a crude product, which was further purified by preparative high-pressure liquid chromatography (column: YMC-Actus Triart C18 ExRS, 30 × 150 mm, 5 µm; mobile phase A: water (10 mmol/L NH₄HCO₃), mobile phase B: ACN; flow rate: 60 mL/min; gradient: 33% B to 45% B within 8 min, 45% B; wavelength: 254/220 nm; RT1 (min): 7.62) to give **compound A-4** as a white solid (7 mg, yield: 4%). m/z (ES+), [M+H]⁺ = 392. ¹H NMR (400 MHz, DMSO-*d*₆): δ (ppm) 11.22 (s, 1H), 7.96 (s, 1H), 7.60 (s, 1H), 7.37 (d, *J* = 8.0 Hz, 1H), 7.25 (d, *J* = 8.0 Hz, 1H), 7.18 (s, 1H), 4.16 (d, *J=* 9.8 Hz, 1H), 3.86 - 4.06 (m, 2H), 3.72-3.76 (m, 2H), 3.50-3.55 (m, 3H), 3.22-3.24 (m, 2H), 2.68 -3.02 (m, 3H), 2.57 (s, 3H), 2.46 (s, 3H), 1.53 - 1.91 (m, 4H).

### Example 4 Synthesis of Compound A-5

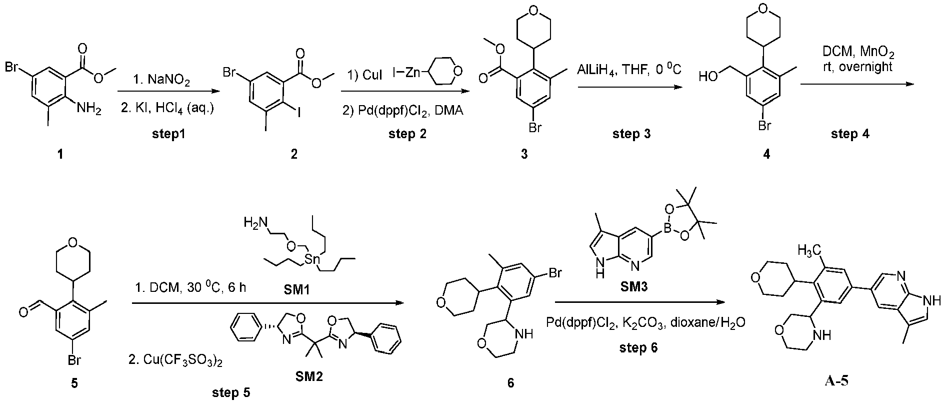

In a 100 mL reaction flask, **compound 1** (4.5 g, 18.436 mmol, 1.00 equiv) was dissolved in a hydrochloric acid solution (3 mol/L, 50 mL), and sodium nitrite (1.65 g, 23.967 mmol, 1.3 equiv) was slowly added to the reaction flask at 0 °C. After nitrogen purging, the mixture was stirred at 0 °C for 2 h. The above reaction solution was added dropwise to an aqueous solution (10 mL) of potassium iodide (3.67 g, 22.123 mmol, 1.2 equiv) at 60 °C, and the mixture was allowed to react at 60 °C for 1.5 h. After the reaction was completed, the reaction solution was extracted with ethyl acetate, and the organic phases were combined and concentrated under reduced pressure. The crude product was purified by column chromatography to give **compound 2** as a saffron yellow oil (5.84 g, yield: 89.2%). m/z (ES+), [M+H]⁺ = 355. ¹H NMR (DMSO-*d*₆, 400 MHz): δ (ppm) 2.44 (s, 3H), 3.86 (s, 3H), 7.56 (s, 1H), 7.73 (s, 1H).

In a 40 mL sealed tube, compound 2 (2.3 g, 6.479 mmol, 1.00 equiv), Pd(dppf)Cl₂ (0.24 g, 0.324 mmol, 0.05 equiv) and copper(I) iodide (0.12 g, 0.648 mmol, 0.1 equiv) were dissolved in N,N-dimethylacetamide (15 mL), then (tetrahydro-2*H*-pyran-4-yl)zinc iodide (19.41 mL, 19.41 mmol, 3.00 equiv in DMA) was added under nitrogen, and the mixture was stirred at 80 °C for 2 h. After the reaction was completed, water was added to the reaction system for quenching, and the mixture was extracted with ethyl acetate. The organic phases were combined and concentrated under reduced pressure, and the crude product was purified by column chromatography to give **compound 3** as a yellow solid (408 mg, yield: 20.1%). m/z (ES+), [M+H]⁺ = 314.

In a 40 mL sealed tube, **compound 3** (408 mg, 1.303 mmol, 1.00 equiv) was dissolved in tetrahydrofuran (6 mL), then lithium aluminum hydride (49.44 mg, 1.303 mmol, 1 equiv) was added slowly at 0 °C, and the mixture was stirred at 0 °C under nitrogen for 1.5 h. After the reaction was completed, the reaction system was quenched with ice water, and the mixture was extracted with ethyl acetate (2 × 80 mL). The organic phases were combined and concentrated under reduced pressure to give **compound 4** as a brown-yellow oil (370 mg, yield: 99.6%).

In a 40 mL sealed tube, **compound 4** (370 mg, 1.297 mmol, 1.00 equiv) and manganese dioxide (902.35 mg, 10.376 mmol, 8 equiv) were dissolved in dichloromethane (5 mL), and the solution was stirred at 30 °C under nitrogen overnight. After the reaction was completed, the reaction system was concentrated under reduced pressure, and the resulting crude product was purified by thin layer chromatography (petroleum ether:ethyl acetate = 5:1) to give **compound** 5 as a yellow solid (309 mg, yield: 84.11%). m/z (ES+), [M]⁺ = 283.

In a 40 mL sealed tube, **compound 5** (309 mg, 1.091 mmol, 1.00 equiv), a 4A molecular sieve (100 mg) and **compound SM1** (397.39 mg, 1.091 mmol, 1 equiv) were dissolved in dichloromethane (8 mL), and the solution was stirred at 30 °C under nitrogen for 6 h. After the reaction was completed, the mixture was filtered, and the filtrate was concentrated by rotary evaporation. The resulting product was dissolved in hexafluoroisopropanol (1 mL), then the solution was added dropwise to a mixture of **compound SM2** (78.93 mg, 0.218 mmol, 0.2 equiv) and copper trifluoromethanesulfonate (72.99 mg, 0.218 mmol, 0.2 equiv) in hexafluoroisopropanol (2 mL), and the resulting mixture was stirred at room temperature under nitrogen overnight. After the reaction was completed, the reaction system was concentrated under reduced pressure, and the crude product was purified by thin layer chromatography (dichloromethane:methanol = 20:1) to give **compound 6** as a yellow solid (100 mg, yield:26.9%). m/z (ES+), [M]⁺ = 340.

In an 8 mL sealed tube, **compound 6** (100 mg, 0.294 mmol, 1.00 equiv), **compound SM3** (75.86 mg, 0.294 mmol, 1 equiv), Pd(dppf)Cl₂ (23.94 mg, 0.029 mmol, 0.1 equiv), and potassium carbonate (62.30 mg, 0.588 mmol, 2 equiv) were added to water (1 mL) and 1,4-dioxane (4 mL), and the mixture was stirred at 80 °C under nitrogen for 2 h. After the reaction was completed, the reaction solution was concentrated, and the mixture was purified by column chromatography (dichloromethane:methanol = 10:1) to give a crude product, which was further purified by preparative high-pressure liquid chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 25% B to 50% B within 8 min, 50% B; wavelength: 220 nm; RT1 (min): 7.30) to give **compound A-5** as a white solid (14.6 mg, yield: 12.7%). m/z (ES+), [M+H]⁺ = 392. ¹H NMR (DMSO-*d*₆, 400 MHz): δ (ppm) 1.55-1.65 (m, 2H), 2.09-2.13 (m, 2H), 2.30 (d, *J*=1.0 Hz, 3H), 2.43 (s, 2H), 2.54 (s, 1H), 2.77 (s, 1H), 2.92 (s, 2H), 3.45-3.48 (m, 3H), 3.55 (s, 1H), 3.66 (s, 1H), 3.77 (dd, *J*=10.4, 2.8 Hz, 1H), 4.01 (dd, *J*=10.0, 4.9 Hz, 2H), 4.14-4.35 (m, 1H), 7.27 (s, 1H), 7.44 (s, 1H), 7.78 (s, 1H), 8.08 (s, 1H), 8.45 (s, 1H), 11.36 (s, 1H).

### Example 5 Synthesis of Compound A-6

**The synthesis method of compound 1 was similar to that of intermediate compound 6 in Example 4.**

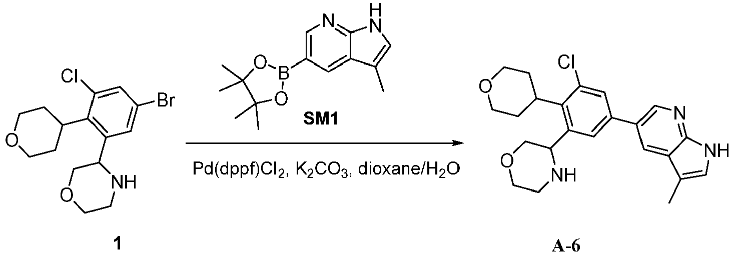

In an 8 mL sealed tube, **compound 1** (60 mg, 0.166 mmol, 1.00 equiv), **compound SM1** (42.94 mg, 0.166 mmol, 1 equiv), Pd(dppf)Cl₂ (13.55 mg, 0.017 mmol, 0.1 equiv) and potassium carbonate (68.97 mg, 0.498 mmol, 3 equiv) were added to water (0.4 mL) and 1,4-dioxane (4 mL), and the mixture was stirred at 80 °C under nitrogen for 2 h. After the reaction was completed, the reaction mixture was concentrated under reduced pressure, and the residue was purified by column chromatography (dichloromethane:methanol = 10:1) to give a crude product, which was further purified by preparative high-pressure liquid chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 30% B to 50% B within 8 min) to give **compound A-6** as a white solid (6.2 mg, yield: 9.0%). m/z (ES+), [M+H]⁺ = 412. ¹H NMR (DMSO-*d*₆, 400 MHz): δ (ppm) 1.28 - 1.77 (m, 2H), 2.09-2.10 (m, 1H), 2.30 (s, 3H), 2.65 (s, 1H), 2.80 - 3.06 (m, 3H), 3.23 (s, 1H), 3.40 - 3.63 (m, 4H), 3.73-3.76 (m, 2H), 4.00 (s, 2H), 4.11 - 4.48 (m, 1H), 7.27 (s, 1H), 7.66 (s, 1H), 7.95 (s, 1H), 8.15 (s, 1H), 8.46 (s, 1H), 11.40 (s, 1H).

### Example 6 Synthesis of Compound A-7

**The synthesis method of compound 1 was similar to that of intermediate compound 6 in Example 4.**

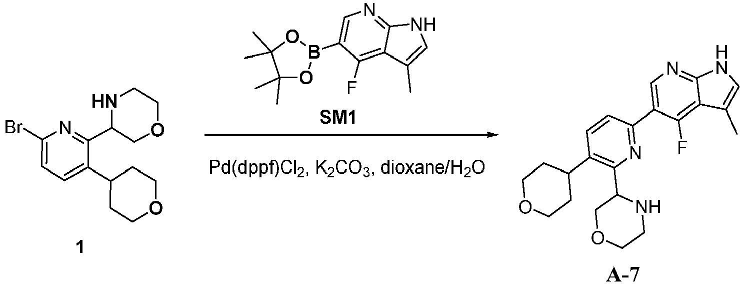

In an 8 mL sealed tube, **compound 1** (30 mg, 0.092 mmol, 1.00 equiv), **compound SM1** (50.63 mg, 0.184 mmol, 2 equiv), Pd(dppf)Cl₂ (7.47 mg, 0.009 mmol, 0.1 equiv) and sodium carbonate (29.15 mg, 0.276 mmol, 3 equiv) were added to a 1,4-dioxane/water (4 mL/1 mL) solution, and the mixture was stirred at 80 °C under nitrogen protection for 2 h. After the reaction was completed, the reaction system was concentrated, and the mixture was purified by column chromatography (dichloromethane/methanol = 10:1) to give a crude product, which was further purified by preparative high-pressure liquid chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 25% B to 45% B within 8 min, 45% B; wavelength: 254 nm; RT1 (min): 6.93) to give **compound A-7** as a white solid (1.1 mg, yield: 2.89%). [M+H]⁺ = 397. ¹H NMR (400 MHz, CD₃OD-*d*₄): δ (ppm) 8.81 (d, *J=* 9.6 Hz, 1H), 7.85 (d, J=8.3 Hz, 1H), 7.78 (d, *J* = 8.3 Hz, 1H), 7.14 (s, 1H), 4.48 (dd, *J* = 10.3 Hz, 3.3 Hz, 1H), 4.11-4.19 (m, 2H), 3.97 (dd, *J* = 11.7 Hz, 3.3 Hz, 1H), 3.85 - 3.94 (m, 1H), 3.58 - 3.72 (m, 3H), 3.55 (d, *J* = 11.0 Hz, 1H), 3.09 -3.22 (m, 3H), 2.49 (s, 3H), 1.95-1.99 (m, 1H), 1.84-1.88 (m, 2H), 1.71-1.74 (m, 1H).
¹⁹F NMR (376 MHz, CD₃OD-*d*₄): δ (ppm) -120.61.

### Example 7 Synthesis of Compound A-8

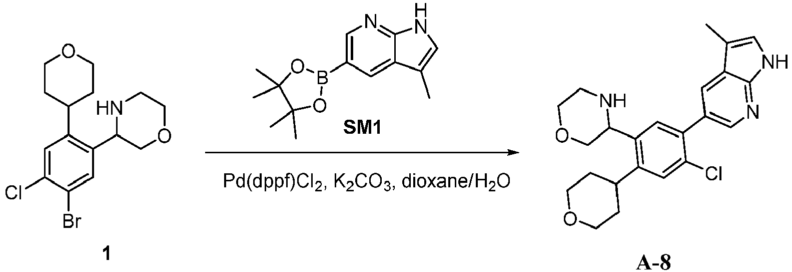

In an 8 mL sealed tube, **compound 1** (60 mg, 0.166 mmol, 1.00 equiv), **SM1** (42.94 mg, 0.166 mmol, 1 equiv), Pd(dppf)Cl₂ (13.55 mg, 0.017 mmol, 0.1 equiv) and sodium carbonate (52.89 mg, 0.498 mmol, 3 equiv) were added to a 1,4-dioxane/water (4 mL/1 mL) solution, and the mixture was stirred at 80 °C under nitrogen for 2 h. After the reaction was completed, the reaction system was concentrated and purified by column chromatography (dichloromethane/ methanol = 10:1) to give a crude product, which was further purified by preparative high-pressure liquid chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 30% B to 50% B within 8 min, 50% B; wavelength: 254 nm; RT1 (min): 7.07) to give **compound A-8** as a white solid (3.1 mg, yield: 4.5%). [M+H]⁺ = 412. ¹H NMR (CD₃OD-*d*₄, 400 MHz): δ (ppm) 1.69 (d, *J*=13.1 Hz, 1H), 1.76 - 1.99 (m, 3H), 2.02 (s, 1H), 2.36 (s, 3H), 2.96-3.01 (m, 1H), 3.05 - 3.16 (m, 1H), 3.22-3.24 (m, 1H), 3.36 - 3.50 (m, 1H), 3.62-3.64 (m, 3H), 3.75 - 3.88 (m, 2H), 4.08 (d, *J* = 11.2 Hz, 2H), 4.24 (dd, *J* = 10.0, 3.0 Hz, 1H), 7.19 (s, 1H), 7.45 (s, 1H), 7.63 (s, 1H), 7.99 (s, 1H), 8.20 (s, 1H).

### Example 8 Synthesis of Compounds A-9 and A-39

**The synthesis method of compound 1 was similar to that of intermediate compound 6 in Example 4.**

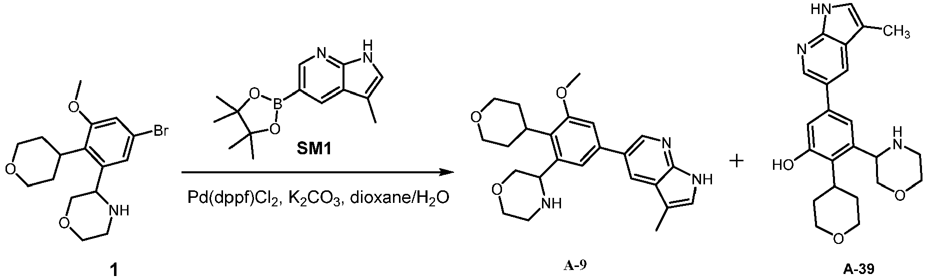

In an 8 mL sealed tube, **compound 1** (50 mg, 0.140 mmol, 1.00 equiv), **compound SM1** (36.23 mg, 0.140 mmol, 1 equiv), Pd(dppf)Cl₂·CH₂Cl₂ (20.54 mg, 0.028 mmol, 0.2 equiv) and potassium carbonate (58.19 mg, 0.420 mmol, 3 equiv) were added to a 1,4-dioxane/water (4 mL/1 mL) solution, and the reaction system was stirred at 80 °C under nitrogen for 2 h. After the reaction was completed, the reaction system was concentrated, and the mixture was purified by column chromatography (dichloromethane:methanol = 10:1) to give a crude product, which was further purified by preparative high-pressure liquid chromatography (column: XBridge Shield RP18 OBD column, 30 × 150 mm, 5 µm; mobile phase A: water (10 mmol/L NH₄HCO₃ + 0.1% NH₃·H₂O); mobile phase B: ACN; flow rate: 60 mL/min; gradient: 25% B to 50% B within 8 min, 50% B; wavelength: 220 nm; RT1 (min): 7.43) to give **compound A-9** as a white solid (1.9 mg, yield: 3.3%) and **compound A-39** as a white solid (0.5 mg, yield: 0.86%).

Compound A-9: m/z (ES+), [M+H]⁺ = 408. ¹H NMR(DMSO-*d*₆, 400 MHz): δ (ppm) 11.35 (s, 1H), 8.49 (s, 1H), 8.12 (s, 1H), 7.56 (s, 1H), 7.27 (s, 1H), 7.21 (s, 1H), 4.18 (d, *J* = 9.7 Hz, 1H), 3.95-3.97 (m, 2H), 3.90 (s, 3H), 3.77 (d, *J* = 10.7 Hz, 1H), 3.67 (d, *J* = 10.8 Hz, 1H), 3.45-3.49 (m, 3H), 3.22-3.24 (m, 2H), 2.92-2.97 (m, 3H), 2.36-2.40 (m, 1H), 2.32 (s, 3H), 1.24-1.44 (m, 3H).

Compound A-39: m/z (ES+), [M+H]⁺ = 394.15.

### Example 9 Synthesis of Compound A-10

**The synthesis method of compound 1 was similar to that of intermediate compound 6 in Example 4.**

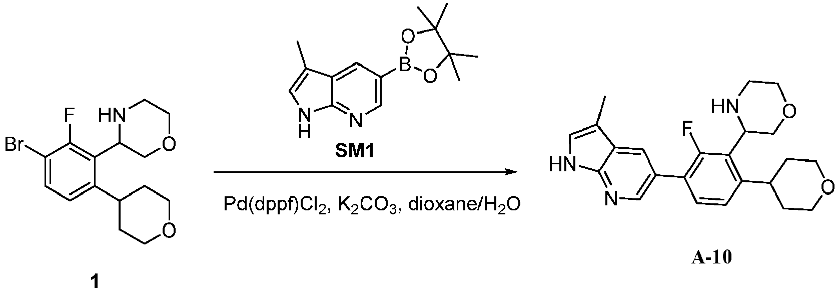

In a 40mL sealed tube, **compound 1** (100 mg, 0.291 mmol, 1.00 equiv), **compound SM1** (74.99 mg, 0.291 mmol, 1 equiv), Pd(dppf)Cl₂ (23.67 mg, 0.029 mmol, 0.1 equiv) and potassium carbonate (120.45 mg, 0.873 mmol, 3 equiv) were added to a 1,4-dioxane/water (5 mL/0.5 mL) solution, and the mixture was allowed to react at 80 °C under nitrogen for 2 h. After the reaction was completed, the reaction solution was concentrated by rotary evaporation, and the mixture was purified by column chromatography (dichloromethane:methanol = 15:1) to give a crude product, which was further purified by preparative high-pressure liquid chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 20% B to 45% B within 8 min) to give **compound A-10** as a white solid (11.7 mg). m/z (ES+), [M+H]⁺ = 396. ¹H NMR (DMSO-*d*₆, 400 MHz): δ (ppm) 11.41 (s, 1H), 8.30 (s, 1H), 8.01 (s, 1H), 7.45 (t, *J* = 8.1 Hz, 1H), 7.24 - 7.29 (m, 2H), 4.38 (d, *J* = 9.9 Hz, 1H), 3.97-4.00 (m, 2H), 3.71-3.78 (m, 3H), 3.67 (dd, *J* = 10.8, 3.4 Hz, 1H), 3.44-3.58 (m, 3H), 2.90 (d, *J* = 6.1 Hz, 2H), 2.75 (s, 1H), 2.29 (s, 3H), 1.62-1.86 (m, 4H).
¹⁹F NMR (DMSO-*d*₆, 376 MHz): δ (ppm) -118.68.

### Example 10 Synthesis of Compound A-11

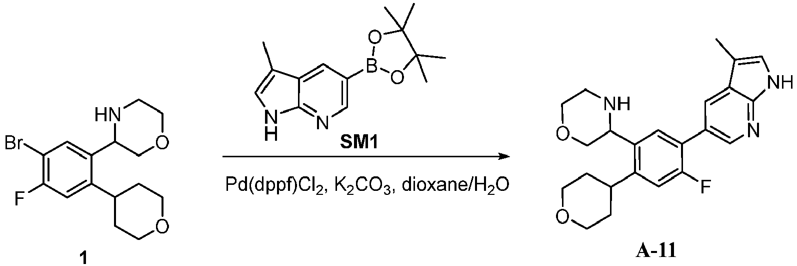

In an 8 mL sealed tube, **compound 1** (70.01 mg, 0.204 mmol, 1.05 equiv) was dissolved in a 1,4-dioxane/water (5 mL:1 mL) solution, then **SM1** (50 mg, 0.194 mmol, 1.00 equiv), Pd(dppf)Cl₂ (14.17 mg, 0.019 mmol, 0.1 equiv) and potassium carbonate (80.90 mg, 0.582 mmol, 3 equiv) were added, and the reaction system was stirred at 80 °C under nitrogen protection for 2 h. After the reaction was completed, the reaction solution was concentrated by rotary evaporation, and the mixture was purified by column chromatography (dichloromethane/ methanol = 15:1) to give a crude product, which was further purified by preparative high-pressure liquid chromatography (column: XBridge Prep OBD C18 column, 30 × 150 mm, 5µm; mobile phase A: water (10 mmol/L NH₄HCO₃), mobile phase B: ACN; flow rate: 60 mL/min; gradient: 35% B to 40% B within 7 min, 40% B; wavelength: 220 nm; RT1 (min): 6.32) to give **compound A-11** as a white solid (24.8 mg, yield: 30.8%). m/z (ES+), [M+H]⁺ = 396. ¹H NMR (DMSO-*d*₆, 400 MHz): δ (ppm) 11.44 (s, 1H), 8.33 (s, 1H), 8.01 (s, 1H), 7.77 (d, *J* = 8.6 Hz, 1H), 7.30 (s, 1H), 7.24 (d, *J* = 8.6 Hz, 1H), 4.12 (dd, *J* = 10.0, 3.0 Hz, 1H), 3.98 (dt, *J* = 13.7, 4.4 Hz, 2H), 3.84 - 3.60 (m, 2H), 3.49-3.55 (m, 3H), 3.27-3.35 (m, 2H), 2.82-2.97 (m, 3H), 2.30 (s, 3H), 1.49-1.95 (m, 4H).
¹⁹F NMR (DMSO-*d*₆, 376 MHz): δ -120.44.

### Example 11 Synthesis of Compound A-12

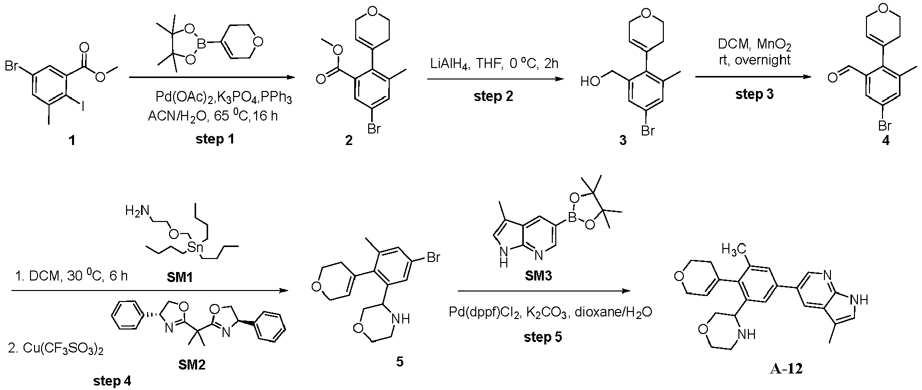

In a 50 mL reaction flask, **compound 1** (3 g, 8.451 mmol, 1.00 equiv), boronic ester (1.95 g, 9.296 mmol, 1.1 equiv), palladium acetate (0.19 g, 0.845 mmol, 0.1 equiv), potassium phosphate (3.59 g, 16.902 mmol, 2 equiv) and triphenylphosphane (0.22 g, 0.845 mmol, 0.1 equiv) were dissolved in an acetonitrile/water (40 mL/10 mL) solution, and the mixture was stirred at 65 °C under nitrogen overnight. After the reaction was completed, the reaction system was concentrated, and the crude product was purified by column chromatography to give **compound 2** as a yellow solid (1.953 g, yield: 73.1%). m/z (ES+), [M+H]⁺ = 313.

In a 50 mL reaction flask, **compound 2** (1.11 g, 3.567 mmol, 1.00 equiv) was dissolved in tetrahydrofuran (15 mL), then lithium aluminum hydride (0.14 g, 3.567 mmol, 1 equiv) was added slowly at 0 °C, and the reaction system was stirred at 0 °C under nitrogen for 2 h. After the reaction was completed, the reaction was quenched with ice water, and the mixture was extracted with ethyl acetate (80 mL × 2). The organic phases were combined and concentrated under reduced pressure to give **compound 3** as a pale yellow oil (944 mg, yield: 93.5%). m/z (ES+), [M+H]⁺ = 284.

In a 40 mL sealed tube, **compound 3** (940 mg, 3.320 mmol, 1.00 equiv) and manganese dioxide (2308.76 mg, 26.560 mmol, 8 equiv) were dissolved in dichloromethane (15 mL), and the reaction system was stirred at 30 °C under nitrogen overnight. After the reaction was completed, the reaction system was concentrated, and the mixture was subjected to thin layer chromatography (petroleum ether:ethyl acetate = 5:1) to give **compound 4** as a yellow solid (858 mg, yield: 91.9%). m/z (ES+), [M]⁺ = 281.

In a 40 mL sealed tube, **compound 4** (450 mg, 1.601 mmol, 1.00 equiv) and a 4A molecular sieve (800 mg) were dissolved in dichloromethane (15 mL). Under nitrogen, **compound SM1** (582.87 mg, 1.601 mmol, 1 equiv) was added, and the reaction system was allowed to react at 30 °C for 6 h. After the reaction was completed, the mixture was filtered, and the filtrate was concentrated. The resulting crude product was dissolved in hexafluoroisopropanol (10 mL), and **copper trifluoromethanesulfonate** (107.05 mg, 0.320 mmol, 0.2 equiv) and **compound SM2** (115.78 mg, 0.320 mmol, 0.2 equiv) were added. The reaction mixture was stirred at room temperature under nitrogen overnight. After the reaction was completed, the reaction system was concentrated, and the crude product was subjected to thin layer chromatography (dichloromethane:methanol = 12:1) to give **compound 5** as a yellow solid (300 mg, yield: 55.4%). m/z (ES+), [M+2H]⁺ = 340.

In an 8 mL sealed tube, **compound 5** (340 mg, 1.005 mmol, 1.00 equiv) was dissolved in a 1,4-dioxane/water (10 mL/1 mL) solution, then **compound SM3** (259.47 mg, 1.005 mmol, 1 equiv), Pd(dppf)Cl₂ (73.55 mg, 0.100 mmol, 0.1 equiv) and potassium carbonate (419.81 mg, 3.015 mmol, 3 equiv) were added, and the reaction system was allowed to react at 80 °C under nitrogen for 2 h. After the reaction was completed, the reaction system was concentrated, and the mixture was purified by column chromatography (dichloromethane:methanol = 12:1) to give a crude product, which was further purified by preparative high-pressure liquid chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 25% B to 50% B within 8 min, 50% B; wavelength: 220 nm; RT1 (min): 7.40) to give **compound A-12** as a white solid (7.8 mg, yield: 1.9%). m/z (ES+), [M+H]⁺ = 390. ¹H NMR (DMSO-*d*₆, 400 MHz): δ (ppm) 11.36 (s, 1H), 8.47 (s, 1H), 8.11 (s, 1H), 7.76 (s, 1H), 7.48 (s, 1H), 7.27 (s, 1H), 5.62 (d, *J* = 14.8 Hz, 1H), 4.12 - 4.38 (m, 2H), 3.66 - 4.10 (m, 4H), 3.38-3.69 (m, 3H), 3.23 (t, *J*=10.3 Hz, 1H), 2.87 (d, J=8.5 Hz, 3H), 2.38 (s, 3H), 2.24 (s, 3H), 2.21-2.25 (m, 2H).

### Example 12 Synthesis of Compound A-13

**The synthesis method of compound 1 was similar to that of intermediate compound 5 in Example 11.**

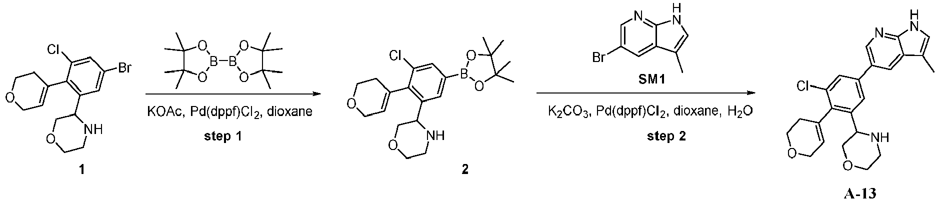

In a 40 mL sealed tube, **compound 1** (250 mg, 0.697 mmol, 1.00 equiv), bis(pinacolato)diboron (194.71 mg, 0.767 mmol, 1.1 equiv), Pd(dppf)Cl₂ (113.56 mg, 0.139 mmol, 0.2 equiv) and potassium acetate (136.82 mg, 1.394 mmol, 2 equiv) were added to 1,4-dioxane (5 mL), and the reaction system was stirred at 80 °C under nitrogen overnight. After the reaction was completed, the reaction system was concentrated by rotary evaporation, and the mixture was purified by column chromatography (dichloromethane:methanol = 20:1) to give **compound 2** as a yellow solid (200 mg, yield: 70.72%). m/z (ES+), [M+H]⁺ = 406.

In a 40 mL sealed tube, **compound 2** (50 mg, 0.237 mmol, 1.00 equiv), compound **SM1** (96.12 mg, 0.237 mmol, 1 equiv), Pd(dppf)Cl₂ (19.30 mg, 0.024 mmol, 0.1 equiv) and potassium carbonate (98.22 mg, 0.711 mmol, 3 equiv) were added to a 1,4-dioxane/water (5 mL/0.5 mL) solution, and the reaction system was stirred at 80 °C under nitrogen for 6 h. After the reaction was completed, the reaction solution was concentrated by rotary evaporation, and the mixture was purified by column chromatography (dichloromethane:methanol = 10:1) to give a crude product, which was further purified by preparative high-pressure liquid chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 30% B to 50% B within 8 min) to give **compound A-13** as a white solid (3.9 mg, yield: 3.99%). m/z (ES+), [M+H]⁺ = 410. ¹H NMR (DMSO-*d*₆, 400 MHz): δ (ppm) 11.43 (s, 1H), 8.49 (s, 1H), 8.18 (s, 1H), 7.92 (d, *J* = 8.0 Hz, 1H), 7.75 (s, 1H), 7.30 (s, 1H), 5.72 (d, *J* = 17.0 Hz, 1H), 4.14-4.37 (m, 2H), 3.83-3.89 (m, 3H), 3.65-3.79 (m, 2H), 3.46-3.64 (m, 2H), 3.42 (d, *J*=10.5 Hz, 1H), 3.26 (d, *J* = 10.2 Hz, 1H), 2.79-2.88 (m, 2H), 2.33-2.49 (m, 4H), 2.26 (s, 1H).

### Example 13 Synthesis of Compound A-14

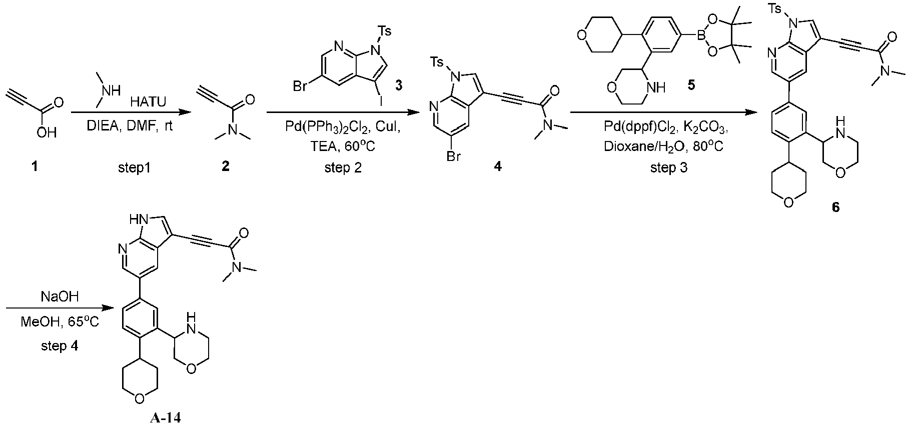

In a 40 mL sealed tube, **compound 1** (2 g, 28.552 mmol, 1.00 equiv), dimethylamine (2.33 g, 28.552 mmol, 1 equiv), HATU (16.28 g, 42.828 mmol, 1.5 equiv) and DIEA (14.76 g, 114.208 mmol, 4 equiv) were dissolved in DMF (20 mL, 273.617 mmol, 9.58 equiv), and the solution was allowed to react at room temperature under nitrogen for 3 h. After the reaction was completed, water and ethyl acetate were added for extraction, and the organic phase was concentrated by rotary evaporation and then subjected to preparative thin layer chromatography (petroleum ether/ethyl acetate = 10:1) to give compound **2** as a yellow solid (2.2 g, yield: 79.34%). MS (ESI): [M+H]⁺ = 98.

In a 40 mL sealed tube, compound **2** (2.2 g, 22.653mmol, 5.40 equiv), compound **3** (2 g, 4.192 mmol, 1.00 equiv), copper(I) iodide (0.08 g, 0.419 mmol, 0.1 equiv) and Pd(PPh₃)₂Cl₂ (0.48 g, 0.419 mmol, 0.1 equiv) were dissolved in triethylamine (20 mL), and the solution was allowed to react at 60 °C under nitrogen overnight. After the reaction was completed, the reaction solution was filtered, concentrated by rotary evaporation to remove the solvent, and subjected to preparative thin layer chromatography (petroleum ether/ethyl acetate = 1:1) to give compound **4** as a yellow solid (740 mg, yield: 40%). MS (ESI): [M+H]⁺ = 446.0.

In a 40 mL sealed tube, compound **4** (740 mg, 1.658 mmol, 1.00 equiv), compound **5** (618.93 mg, 1.658 mmol, 1 equiv), Pd(dppf)Cl₂ (135.06 mg, 0.166 mmol, 0.1 equiv) and potassium carbonate (687.44 mg, 4.974 mmol, 3 equiv) were dissolved in 1,4-dioxane (8 mL) and water (0.8 mL), and the solution was allowed to react at 80 °C under nitrogen for 3 h. After the reaction was completed, the reaction solution was filtered, concentrated by rotary evaporation to remove the solvent, and subjected to preparative thin layer chromatography (dichloromethane/methanol = 10:1) to give compound **6** as a yellow solid (640 mg, yield: 63%). MS (ESI): [M+H]⁺ = 613.4.

In a 40 mL sealed tube, compound 6 (640 mg, 1.044 mmol, 1.00 equiv) and NaOH (2 mL, 50.004 mmol, 47.87 equiv, 2 M) were added to methanol (7 mL), and the mixture was allowed to react at 65 °C under nitrogen for 1 h. After the reaction was completed, the reaction solution was concentrated by rotary evaporation and subjected to column chromatography (dichloromethane/methanol = 7:1) to give a crude product, which was further subjected to preparative high-pressure chromatography (column: CHIRALPAK IG, 2 × 25 cm, 5 µm; mobile phase A: Hex (0.5% 2 M NH3-MeOH) -- HPLC, mobile phase B: EtOH:DCM = 1:1 -- HPLC; flow rate: 20 mL/min; gradient: 40% B to 40% B within 15.5 min; wavelength: 220/254 nm; RT1 (min): 9.20; RT2 (min): 12.70; sample solvent: EtOH:DCM = 1:1 -- HPLC; injection volume: 0.85 mL) to give compound **A-14** as a white solid (100 mg, yield: 20.3%). MS (ESI): [M+H]⁺ = 459.2. ¹H NMR(DMSO-*d*₆, 400 MHz): δ (ppm) 12.56 (s, 1H), 8.62 (d, *J* = 2.0 Hz, 1H), 8.20 (s, 1H), 8.13 (d, *J* = 2.0 Hz, 1H), 7.95 (d, *J* = 2.0 Hz, 1H), 7.63 (dd, *J* = 8.0, 2.0 Hz, 1H), 7.42 (d, *J* = 8.0 Hz, 1H), 4.18-4.16 (m, 1H), 4.02-3.98 (m, 2H), 3.79-3.76 (m, 1H), 3.72-3.68 (m, 1H), 3.57-3.52 (m, 3H), 3.35-3.15 (m, 5H), 3.02-2.88 (m, 6H), 1.86-1.56 (m, 4H).

### Example 14 Synthesis of Compound A-15

**The synthesis method of compound 1 was similar to that of intermediate compound 6 in Example 4.**

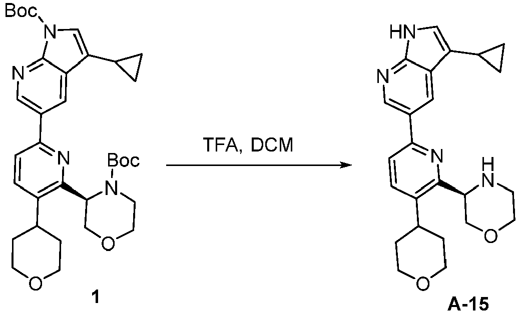

**Compound 1** (80 mg, 0.132 mmol, 1.0 equiv) was dissolved in dichloromethane (1 mL), and trifluoroacetic acid (1 mL) was added. After nitrogen purging in the reaction flask, the reaction system was stirred at room temperature under nitrogen for 1 h. After the reaction was completed, the reaction solution was concentrated by rotary evaporation. The resulting crude product was purified by preparative high-pressure liquid chromatography (column: XBridge Shield RP18 OBD column, 30 × 150 mm, 5 µm; mobile phase A: water (10 mmol/L NH₄HCO₃), mobile phase B: ACN; flow rate: 60 mL/min; gradient: 24% B to 49% B within 8 min, 49% B; wavelength: 254 nm; RT1 (min): 7.28) to give compound A-15 as a white solid (32.6 mg, yield: 60.31%). MS (ESI): m/z [M+H]⁺ = 405. ¹H NMR (400 MHz, DMSO-*d₆*): δ (ppm) 11.45 (s, 1H), 8.98 (s, 1H), 8.60 (s, 1H), 7.92 (d, *J* = 8.2 Hz, 1H), 7.80 (d, *J* = 8.3 Hz, 1H), 7.20 (s, 1H), 4.29 (d, *J* = 9.6 Hz, 1H), 3.98 (d, *J* = 10.9 Hz, 2H), 3.88 (d, *J* = 11.0 Hz, 1H), 3.77 (d, *J* = 10.2 Hz, 1H), 3.42-3.59 (m, 4H), 3.12-3.19 (m, 1H), 2.97 (s, 2H), 2.65 (s, 1H), 2.00-2.11 (m, 1H), 1.63-1.85 (m, 4H), 0.87-0.93 (m, 2H), 0.67-0.71 (m, 2H).

### Example 15 Synthesis of Compound A-16

**The synthesis method of compound 1 was similar to that of intermediate compound 6 in Example 4.**

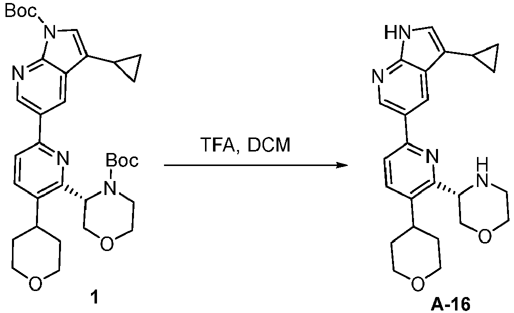

**Compound 1** (80 mg, 0.132 mmol, 1.0 equiv) was dissolved in dichloromethane (1 mL), and trifluoroacetic acid (1 mL) was added. After nitrogen purging in the reaction flask, the reaction system was stirred at room temperature under nitrogen for 1 h. After the reaction was completed, the reaction solution was concentrated by rotary evaporation. The resulting crude product was purified by preparative high-pressure liquid chromatography (column: XBridge Shield RP18 OBD column, 30 × 150 mm, 5 µm; mobile phase A: water (10 mmol/L NH₄HCO₃), mobile phase B: ACN; flow rate: 60 mL/min; gradient: 24% B to 49% B within 8 min, 49% B; wavelength: 254 nm; RT1 (min): 7.30) to give compound A-16 as a white solid (30.7 mg, yield: 56.80%). MS (ESI): m/z [M+H]⁺ = 405. ¹H NMR (400 MHz, DMSO-*d₆*): δ (ppm) 11.45 (s, 1H), 8.98 (s, 1H), 8.60 (s, 1H), 7.92 (d, *J* = 8.2 Hz, 1H), 7.80 (d, *J* = 8.3 Hz, 1H), 7.20 (s, 1H), 4.29 (d, *J* = 8.4 Hz, 1H), 3.98 (d, *J* = 11.1 Hz, 2H), 3.88 (dd, *J* = 11.2, 3.1 Hz, 1H), 3.77 (d, *J* = 11.1 Hz, 1H), 3.45-3.59 (m, 4H), 3.13-3.20 (m, 1H), 2.98 (s, 2H), 2.68 (s, 1H), 2.02-2.10 (m, 1H), 1.61-1.82 (m, 4H), 0.84-0.92 (m, 2H), 0.65-0.73 (m, 2H).

### Example 16 Synthesis of Compound A-17

**The synthesis method of compound 1 was similar to that of intermediate compound 6 in Example 4.**

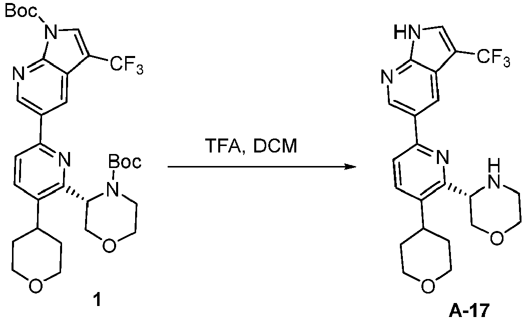

**Compound 1** (50 mg, 0.079 mmol, 1.0 equiv) was dissolved in dichloromethane (1 mL), and trifluoroacetic acid (1 mL) was added. After nitrogen purging in the reaction flask, the reaction system was stirred at room temperature under nitrogen for 1 h. After the reaction was completed, the reaction solution was concentrated by rotary evaporation. The resulting crude product was purified by preparative high-pressure liquid chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 20% B to 50% B within 8 min, 50% B; wavelength: 220 nm; RT1 (min): 7.58) to give compound A-17 as a white solid (4.5 mg, yield: 12.3%). MS (ESI): m/z [M+H]⁺ = 433. ¹H NMR (400 MHz, DMSO-*d₆*): δ (ppm) 9.17 (s, 1H), 8.60 (s, 1H), 8.23 (s, 1H), 7.99 (d, *J* = 8.2 Hz, 1H), 7.84 (d, *J* = 8.3 Hz, 1H), 4.30 (d, *J* = 9.9 Hz, 1H), 3.99 (dd, *J* = 10.9, 3.4 Hz, 2H), 3.89 (d, *J* = 11.1 Hz, 1H), 3.78 (d, *J* = 10.4 Hz, 1H), 3.45-3.63 (m, 4H), 3.17-3.22 (m, 1H), 2.98 (s, 2H), 1.63-1.86 (m, 4H).
¹⁹F NMR (376 MHz, DMSO-*d₆*): -55.10.

### Example 17 Synthesis of Compound A-18

**The synthesis method of compound 1 was similar to that of intermediate compound 6 in Example 4.**

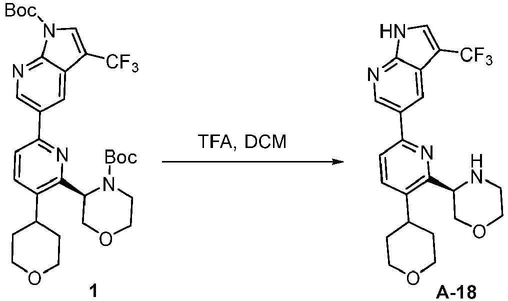

**Compound 1** (100 mg, 0.158 mmol, 1.0 equiv) was dissolved in dichloromethane (1 mL), and trifluoroacetic acid (1 mL) was added. After nitrogen purging in the reaction flask, the reaction system was stirred at room temperature under nitrogen for 1 h. After the reaction was completed, the reaction solution was concentrated by rotary evaporation. The resulting crude product was purified by preparative high-pressure liquid chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 20% B to 50% B within 8 min, 50% B; wavelength: 220 nm; RT1 (min): 7.58) to give compound A-18 as a white solid (22.1 mg, yield: 31.98%). MS (ESI): m/z [M+H]⁺ = 433. ¹H NMR (400 MHz, DMSO-*d₆*): δ (ppm) 12.69 (s,1H), 9.18 (s, 1H), 8.61 (s, 1H), 8.23 (s, 1H), 7.99 (d, *J* = 8.2 Hz, 1H), 7.85 (d, *J* = 8.3 Hz, 1H), 4.30 (d, *J* = 9.9 Hz, 1H), 3.99 (dd, *J* = 10.9, 3.4 Hz, 2H), 3.89 (dd, *J* = 11.1, 3.1 Hz, 1H), 3.78 (d, *J* = 10.4 Hz, 1H), 3.45-3.63 (m, 4H), 3.16-3.22 (m, 1H), 2.98-3.00 (m, 2H), 1.75-1.81 (m, 3H), 1.63 (d, *J* = 12.8 Hz, 1H).
¹⁹F NMR (376 MHz, DMSO-*d*₆): -55.12.

### Example 18 Synthesis of Compounds A-19 and A-20

**The synthesis method of compound 1 was similar to that of intermediate compound 6 in Example 4.**

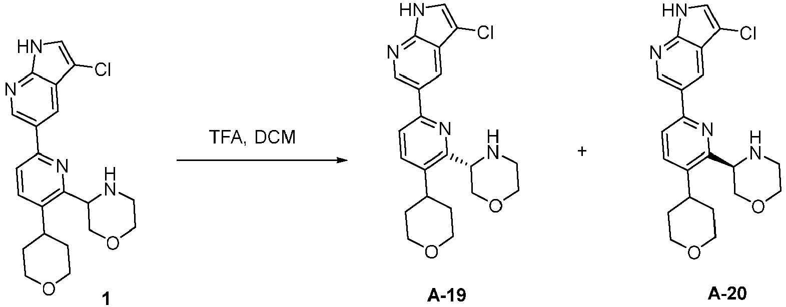

**Compound 1** was purified by chiral preparative high-pressure liquid chromatography (column: CHIRALPAK IG, 2 × 25 cm, 5 µm; mobile phase A: Hex (0.2% DEA) -- HPLC, mobile phase B: EtOH:DCM = 1:1 -- HPLC; flow rate: 20 mL/min; gradient: 90% B to 90% B within 20 min; wavelength: 220/254 nm; RT1 (min): 5.45; RT2 (min): 15.32; mobile phase solvent: EtOH:DCM = 1:1 -- HPLC; sample injection volume: 2 mL; number of runs: 1) to give compound A-19 as a white solid (8.5mg) and compound A-20 as a white solid (8.3mg).
**A-19:**
   MS (ESI): m/z [M+H]⁺ = 399. ¹H NMR (400 MHz, DMSO-*d₆*): δ (ppm) 11.96 (s, 1H), 9.11 (s, 1H), 8.54 (s, 1H), 7.98 (d, *J* = 8.1 Hz, 1H), 7.81 (d, *J* = 8.0 Hz, 1H), 7.75 (s, 1H), 4.30 (d, *J* = 9.9 Hz, 1H), 3.98 (d, *J* = 11.0 Hz, 2H), 3.60-3.89 (m, 2H), 3.46-3.57 (m, 5H), 3.16-3.21 (m, 1H), 2.97 (s, 2H), 1.61-1.79 (m, 4H).
**A-20:**
   MS (ESI): m/z [M+H]⁺ = 399. ¹H NMR (400 MHz, DMSO-*d₆*): δ (ppm) 12.17 (s, 1H), 9.11 (s, 1H), 8.54 (s, 1H), 7.98 (d, *J* = 8.3 Hz, 1H), 7.82 (d, *J* = 8.3 Hz, 1H), 7.75 (s, 1H), 4.30 (dd, *J* = 10.2 Hz, 3.2 Hz, 1H), 3.98 (dd, *J* = 10.1, 2.9 Hz, 2H), 3.88 (dd, *J* = 11.1, 3.1 Hz, 1H), 3.77 (dd, *J* = 10.6, 2.5 Hz, 1H), 3.47-3.61 (m, 5H), 3.15-3.19 (m, 1H), 2.99 (s, 2H), 1.56-1.80 (m, 4H).

### Example 19 Synthesis of Compound A-21

**The synthesis method of compound 1 was similar to that of intermediate compound 6 in Example 4.**

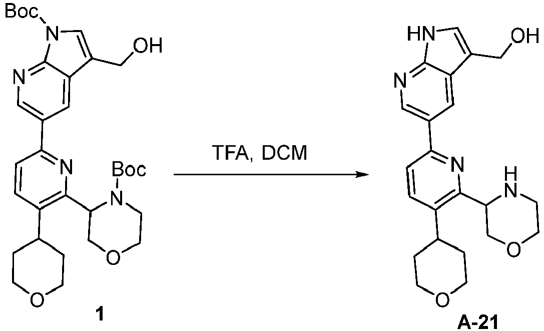

**Compound 1** (20 mg, 0.034 mmol, 1.0 equiv) was dissolved in dichloromethane (1 mL), and trifluoroacetic acid (1 mL) was added. After nitrogen purging in the reaction flask, the reaction system was stirred at room temperature under nitrogen for 1 h. After the reaction was completed, the reaction solution was concentrated by rotary evaporation. The resulting crude product was purified by preparative high-pressure liquid chromatography (column: XBridge Shield RP18 OBD column, 30 × 150 mm, 5 µm; mobile phase A: water (10 mmol/L NH₄HCO₃), mobile phase B: ACN; flow rate: 60 mL/min; gradient: 10% B to 31% B within 8 min, 31% B; wavelength: 254 nm; RT1 (min): 7.45/9.37) to give compound A-21 as a white solid (1.5 mg, yield: 9.74%). MS (ESI): m/z [M+H]⁺ = 395. ¹H NMR (400 MHz, DMSO-*d₆*): δ (ppm) 11.58 (s, 1H), 9.00 (s, 1H), 8.64 (s, 1H), 7.89 (d, *J* = 8.2 Hz, 1H), 7.81 (d, *J* = 8.3 Hz, 1H), 7.41 (s, 1H), 4.94 (t, *J* = 5.5 Hz, 1H), 4.70 (d, J = 5.5 Hz, 2H), 4.30 (d, *J* = 9.9 Hz, 1H), 3.98 (d, *J* = 11.0 Hz, 2H), 3.88 (d, *J=* 11.3 Hz, 1H), 3.78 (d, *J* = 11.3 Hz, 1H), 3.45-3.61 (m, 4H), 3.17-3.21 (m, 1H), 2.98 (s, 2H), 1.63-1.78 (m, 4H).

### Example 20 Synthesis of Compound A-22

**The synthesis method of compound 1 was similar to that of intermediate compound 6 in Example 4.**

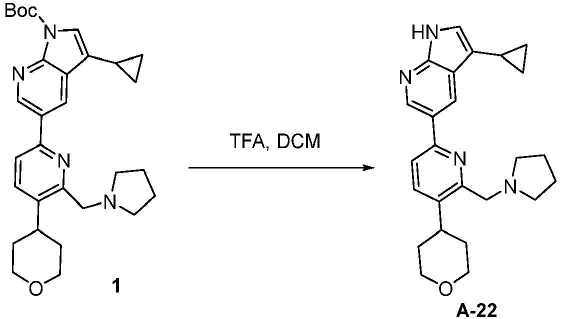

**Compound 1** (80 mg, 0.159 mmol, 1.0 equiv) was dissolved in dichloromethane (1 mL), and trifluoroacetic acid (1 mL) was added. After nitrogen purging in the reaction flask, the reaction system was stirred at room temperature under nitrogen for 1 h. After the reaction was completed, the reaction solution was concentrated by rotary evaporation. The resulting crude product was purified by preparative high-pressure liquid chromatography (column: XBridge Shield RP18 OBD column, 30 × 150 mm, 5 µm; mobile phase A: water (10 mmol/L NH₄HCO₃), mobile phase B: ACN; flow rate: 60 mL/min; gradient: 23% B to 48% B within 8 min, 48% B; wavelength: 254 nm; RT1 (min): 7.50) to give compound A-22 as a white solid (28.8 mg, yield: 44.77%). MS (ESI): m/z [M+H]⁺ = 403. ¹H NMR (400 MHz, DMSO-*d₆*): δ (ppm) 11.42 (s, 1H), 8.90 (s, 1H), 8.58 (s, 1H), 7.90 (d, *J* = 8.2 Hz, 1H), 7.78 (d, *J* = 8.2 Hz, 1H), 7.20 (s, 1H), 3.99 (d, *J* = 10.8 Hz, 2H), 3.90 (s, 2H), 3.41-3.52 (m, 2H), 3.20-3.23 (m, 1H), 2.51-2.55 (m, 4H), 1.98-2.05 (m, 1H), 1.65-1.82 (m, 8H), 0.89 (t, *J* = 9.9 Hz, 2H), 0.65 (t, *J* = 9.9 Hz, 2H).

### Example 21 Synthesis of Compound A-23

**The synthesis method of compound 1 was similar to that of intermediate compound 6 in Example 4.**

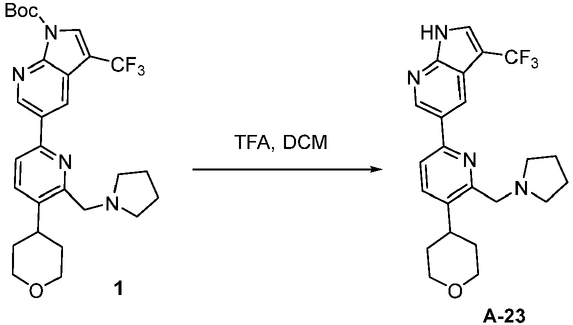

**Compound 1** (50 mg, 0.094 mmol, 1.0 equiv) was dissolved in dichloromethane (4 mL), and trifluoroacetic acid (1.5 mL) was added. After nitrogen purging in the reaction flask, the reaction system was stirred at room temperature under nitrogen for 1 h. After the reaction was completed, the reaction solution was concentrated by rotary evaporation. The resulting crude product was purified by preparative high-pressure liquid chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 23% B to 50% B within 7 min, 50% B; wavelength: 220 nm; RT1 (min): 6.23) to give compound A-23 as a white solid (10.1 mg, yield: 24.47%). MS (ESI): m/z [M+H]⁺ = 431. ¹H NMR (400 MHz, DMSO-*d₆*): δ (ppm) 12.64 (s, 1H), 9.09 (s, 1H), 8.63 (s, 1H), 8.22 (s, 1H), 7.98 (d, *J* = 8.1 Hz, 1H), 7.82 (d, *J* = 8.1 Hz, 1H), 3.81-4.05- (m, 5H), 3.36-3.62 (m, 4H), 1.64-1.92 (m, 9H), 1.24 (s, 1H). ¹⁹F NMR (376 MHz, DMSO-*d*₆): -55.18.

### Example 22 Synthesis of Compound A-24

**The synthesis method of compound 1 was similar to that of intermediate compound 6 in Example 4.**

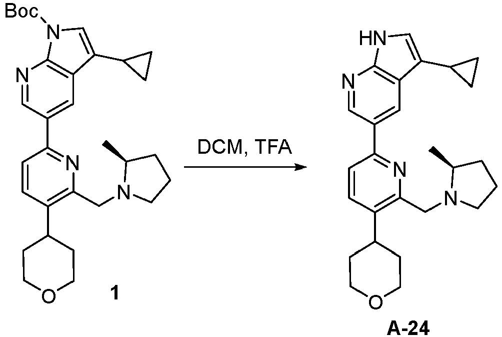

**Compound 1** (60 mg, 0.116 mmol, 1.0 equiv) was dissolved in dichloromethane (6 mL), and trifluoroacetic acid (2 mL) was added. After nitrogen purging in the reaction flask, the reaction system was stirred at room temperature under nitrogen for 1 h. After the reaction was completed, the reaction solution was concentrated by rotary evaporation. The resulting crude product was purified by preparative high-pressure liquid chromatography (column: XBridge Shield RP18 OBD column, 30 × 150 mm, 5 µm; mobile phase A: water (10 mmol/L NH₄HCO₃ + 0.1% NH₃·H₂O), mobile phase B: ACN; flow rate: 60 mL/min; gradient: 35% B to 60% B within 8 min, 60% B to 70% B within 9 min, 70% B; wavelength: 254 nm; RT1 (min): 8.43) to give compound A-24 as a white solid (25 mg, yield: 51.89%). MS (ESI): m/z [M+H]⁺ = 417. ¹H NMR (400 MHz, DMSO-*d₆*): δ (ppm) 11.42 (s, 1H), 8.90 (s, 1H), 8.58 (s, 1H), 7.90 (d, *J=* 8.2 Hz, 1H), 7.78 (d, *J* = 8.2 Hz, 1H), 7.20 (s, 1H), 4.38 (d, *J* = 11.9 Hz, 1H), 4.00 (dd, J = 11.1, 3.6 Hz, 2H), 3.34-3.53 (m, 4H), 2.67-2.69 (m, 1H), 2.26-2.31 (m, 1H), 1.87-2.03 (m, 2H), 1.60-1.77 (m, 4H), 1.48-1.64 (m, 2H), 1.26-1.41 (m, 1H), 1.19 (d, *J=* 5.9 Hz, 3H), 0.80-0.95 (m, 2H), 0.60-0.73 (m, 2H).

### Example 23 Synthesis of Compound A-25

**The synthesis method of compound 1 was similar to that of intermediate compound 6 in Example 4.**

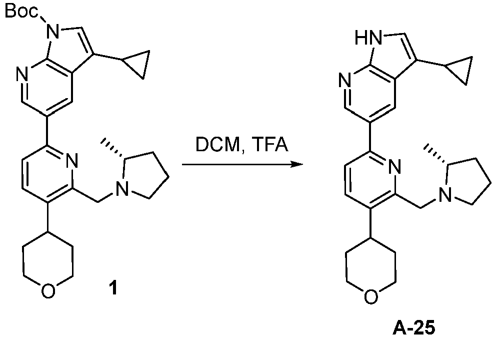

**Compound** 1 (60 mg, 0.077 mmol, 1.0 equiv) was dissolved in dichloromethane (6 mL), and trifluoroacetic acid (2 mL) was added. After nitrogen purging in the reaction flask, the reaction system was stirred at room temperature under nitrogen for 1 h. After the reaction was completed, the reaction solution was concentrated by rotary evaporation. The resulting crude product was purified by preparative high-pressure liquid chromatography (column: XBridge Shield RP18 OBD column, 30 × 150 mm, 5 µm; mobile phase A: water (10 mmol/L NH₄HCO₃ + 0.1% NH₃·H₂O), mobile phase B: ACN; flow rate: 60 mL/min; gradient: 35% B to 60% B within 8 min, 60% B to 70% B within 9 min, 70% B; wavelength: 254 nm; RT1 (min): 8.32) to give compound A-25 as a white solid (23.3 mg, yield: 48.17%). MS (ESI): m/z [M+H]⁺ = 417. ¹H NMR (400 MHz, DMSO-*d₆*): δ (ppm) 11.42 (s, 1H), 8.90 (s, 1H), 8.58 (s, 1H), 7.90 (d, *J* = 8.1 Hz, 1H), 7.78 (d, *J* = 8.2 Hz, 1H), 7.20 (s, 1H), 4.38 (d, *J* = 12.0 Hz, 1H), 4.00 (dd, *J* = 11.1, 3.6 Hz, 2H), 3.34-3.57 (m, 4H), 2.69 (t, J = 7.7 Hz, 1H), 2.26-2.41 (m, 1H), 1.90-2.07 (m, 2H), 1.43-1.83 (m, 6H), 1.20-1.34 (m, 1H), 1.19 (d, *J* = 5.9 Hz, 3H), 0.87-0.92 (m, 2H), 0.65-0.69 (m, 2H).

### Example 24 Synthesis of Compound A-26

**The synthesis method of compound 1 was similar to that of intermediate compound 6 in Example 4.**

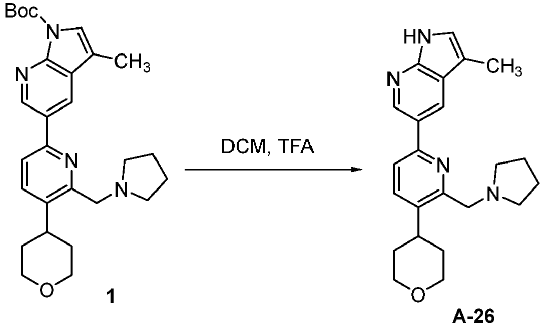

**Compound 1** (100 mg, 0.210 mmol, 1 equiv) was dissolved in dichloromethane (4 mL), and trifluoroacetic acid (1.5 mL) was added. After nitrogen purging in the reaction flask, the reaction system was stirred at room temperature under nitrogen for 1 h. After the reaction was completed, the reaction solution was concentrated by rotary evaporation. The resulting crude product was purified by preparative high-pressure liquid chromatography (column: XBridge Shield RP18 OBD column, 30 × 150 mm, 5 µm; mobile phase A: water (10 mmol/L NH₄HCO₃), mobile phase B: ACN; flow rate: 60 mL/min; gradient: 14% B to 38% B within 11 min, 38% B; wavelength: 254 nm; RT1 (min): 10.30) to give compound A-26 as a white solid (44 mg, yield: 55.59%). MS (ESI): m/z [M+H]⁺ = 377. ¹H NMR (400 MHz, DMSO-*d₆*): δ (ppm) 11.40 (s, 1H), 8.91 (s, 1H), 8.51 (s, 1H), 7.90 (d, *J* = 8.2 Hz, 1H), 7.78 (d, *J* = 8.2 Hz, 1H), 7.27 (s, 1H), 3.85-4.03 (m, 5H), 3.43-3.56 (m, 4H), 3.21-3.29 (m, 2H), 2.32 (s, 3H), 1.59-1.77 (m, 8H).

### Example 25 Synthesis of Compound A-27

**The synthesis method of compound 1 was similar to that of intermediate compound 6 in Example 4.**

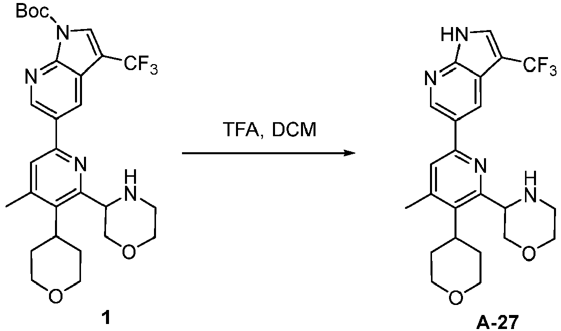

**Compound 1** (38 mg, 0.07 mmol, 1.0 equiv) was dissolved in dichloromethane (4 mL), and trifluoroacetic acid (2 mL) was added. After nitrogen purging in the reaction flask, the reaction system was stirred at room temperature under nitrogen for 1 h. After the reaction was completed, the reaction solution was concentrated by rotary evaporation. The resulting crude product was purified by preparative high-pressure liquid chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 22% B to 52% B within 8 min, 52% B; wavelength: 220 nm; RT1 (min): 7.37) to give compound A-27 as a white solid (1.9 mg, yield: 5.72%). MS (ESI): m/z [M+H]⁺ = 447. ¹H NMR (400 MHz, DMSO-*d₆*): δ (ppm) 9.14 (s, 1H), 8.61 (s, 1H), 7.94 (s, 1H), 7.72 (s, 1H), 4.63 (s, 1H), 4.54 (dd, *J* = 10.1, 3.2 Hz, 1H), 4.15 (dd, *J* = 11.6, 4.3 Hz, 2H), 3.87-4.05 (m, 2H), 3.55-3.73 (m, 4H), 3.37-3.48 (m, 1H), 3.12-3.18 (m, 2H), 2.61 (s, 3H), 2.28-2.41 (m, 2H), 1.75 (d, *J* = 13.2 Hz, 1H), 1.66 (d, *J*= 13.6 Hz, 1H).
¹⁹F NMR (376 MHz, CD₃OD-*d*₄): -58.47.

### Example 26 Synthesis of Compound A-28

**The synthesis method of compound 1 was similar to that of intermediate compound 6 in Example 4.**

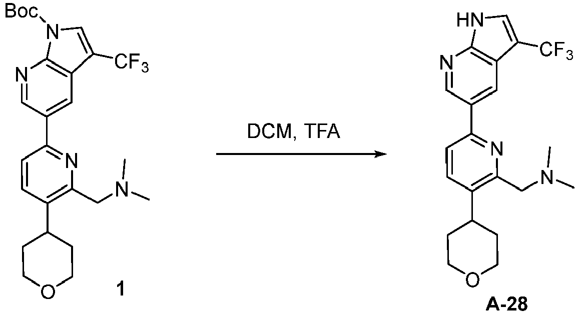

**Compound 1** (60 mg, 0.119 mmol, 1.0 equiv) was dissolved in dichloromethane (6 mL), and trifluoroacetic acid (2 mL) was added. After nitrogen purging in the reaction flask, the reaction system was stirred at room temperature under nitrogen for 1 h. After the reaction was completed, the reaction solution was concentrated by rotary evaporation. The resulting crude product was purified by preparative high-pressure liquid chromatography (column: XBridge Shield RP18 OBD column, 30 × 150 mm, 5 µm; mobile phase A: water (10 mmol/L NH₄HCO₃), mobile phase B: ACN; flow rate: 60 mL/min; gradient: 26% B to 51% B within 8 min, 51% B; wavelength: 254 nm; RT1 (min): 7.55) to give compound A-28 as a white solid (6 mg, yield: 9.9%). MS (ESI): m/z [M+H]⁺ = 405. ¹H NMR (400 MHz, DMSO-*d₆*): δ (ppm) 12.65 (s, 1H), 9.09 (s, 1H), 8.62 (s, 1H), 8.23 (s, 1H), 7.99 (d, *J* = 8.1 Hz, 1H), 7.84 (d, *J* = 8.2 Hz, 1H), 3.99 (dd, *J* = 10.6, 3.8 Hz, 2H), 3.69 (s, 2H), 3.47 (dd, J = 11.5, 2.6 Hz, 2H), 3.29-3.31 (m, 1H), 2.23 (s, 6H), 1.67-1.83 (m, 4H).
¹⁹F NMR (376 MHz, DMSO-d6): -55.55.

### Example 27 Synthesis of Compound A-29

**The synthesis method of compound 1 was similar to that of intermediate compound 6 in Example 4.**

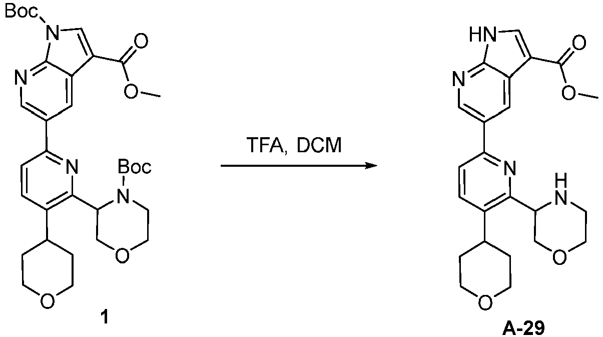

**Compound 1** (20 mg, 0.032 mmol, 1.0 equiv) was dissolved in dichloromethane (1 mL), and trifluoroacetic acid (1 mL) was added. After nitrogen purging in the reaction flask, the reaction system was stirred at room temperature under nitrogen for 1 h. After the reaction was completed, the reaction solution was concentrated by rotary evaporation. The resulting crude product was purified by preparative high-pressure liquid chromatography (column: XBridge Shield RP18 OBD column, 30 × 150 mm, 5 µm; mobile phase A: water (10 mmol/L NH₄HCO₃), mobile phase B: ACN; flow rate: 60 mL/min; gradient: 17% B to 42% B within 8 min, 42% B; wavelength: 254 nm; RT1 (min): 7.32) to give compound A-29 as a white solid (5.2 mg, yield: 38.25%). MS (ESI): m/z [M+H]⁺ = 423. ¹H NMR (400 MHz, DMSO-*d₆*): δ (ppm) 12.65 (s,1H), 9.10 (s, 1H), 8.91 (s, 1H), 8.28 (s, 1H), 7.93 (d, *J* = 8.2 Hz, 1H), 7.84 (d, *J* = 8.3 Hz, 1H), 4.31 (dd, *J* = 10.4, 3.1 Hz, 1H), 3.95-4.03 (m, 3H), 3.87 (s, 3H), 3.85-3.86 (m, 1H), 3.39-3.53 (m, 4H), 3.15-3.21 (m, 1H), 2.87-2.98 (m, 2H), 1.97-2.01 (m, 1H), 1.62-1.79 (m, 4H).

### Example 28 Synthesis of Compound A-30

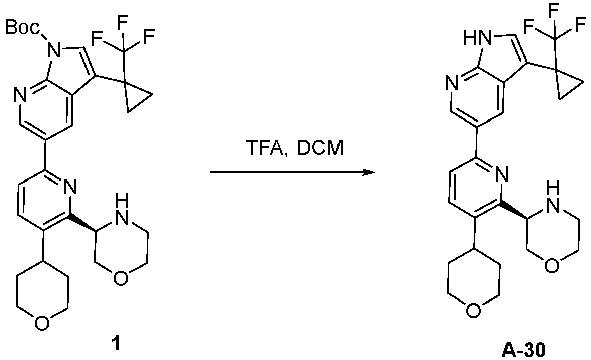

**Compound 1** (200 mg, 0.345 mmol, 1 equiv) was dissolved in dichloromethane (6 mL), and trifluoroacetic acid (2 mL) was added. After nitrogen purging in the reaction flask, the mixture was stirred at room temperature for 1 h. After the reaction was completed, the reaction solution was concentrated by rotary evaporation. The resulting crude product was purified by preparative high-pressure liquid chromatography (column: XBridge Shield RP18 OBD column, 30 × 150 mm, 5 µm; mobile phase A: water (10 mmol/L NH₄HCO₃), mobile phase B: ACN; flow rate: 60 mL/min; gradient: 28% B to 53% B within 8 min, 53% B; wavelength: 254 nm; RT1 (min): 7.32) to give compound A-30 as a white solid (29.1 mg, yield: 17.6%). MS (ESI): m/z [M+H]⁺ = 473. ¹H NMR (400 MHz, DMSO-*d*₆): δ (ppm) 11.93 (s, 1H), 9.03 (s, 1H), 8.55 (s, 1H), 7.90 (d, *J* = 8.2 Hz, 1H), 7.83 (d, *J* = 8.3 Hz, 1H), 7.65 (s, 1H), 4.29 (d, *J* = 9.6 Hz, 1H), 3.99 (d, *J* = 10.6 Hz, 2H), 3.89 (dd, *J* = 11.1, 3.2 Hz, 1H), 3.78 (d, *J* = 10.5 Hz, 1H), 3.52-3.59 (m, 3H), 3.33-3.47 (m, 1H), 3.22-3.33 (m, 1H), 2.89-3.00 (m, 2H), 1.62 -1.80 (m, 4H), 1.38-1.41 (m, 2H), 1.17 (s, 2H). ¹⁹F NMR (376 MHz, DMSO-d6): δ (ppm) -68.46.

### Example 29 Synthesis of Compounds A-32 and A-33

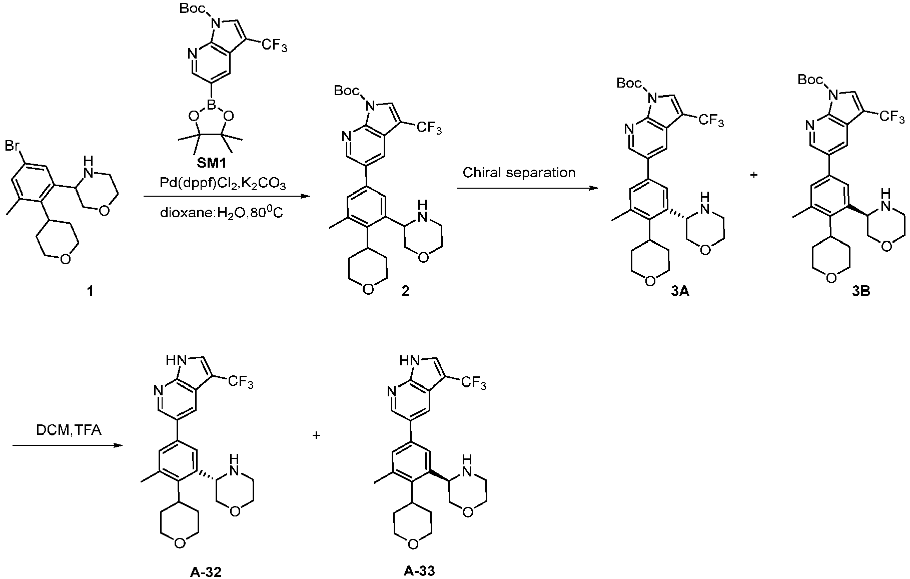

**The synthesis method of compound 1 was similar to that of intermediate compound 6 in Example 4.**

In a sealed tube, **compound 1** (80 mg, 0.235 mmol, 1.0 equiv), potassium carbonate (97.48 mg, 0.705 mmol, 3.0 equiv), Pd(dppf)Cl₂·CH₂Cl₂ (19.15 mg, 0.024 mmol, 0.1 equiv) and **compound SM1** (106.61 mg, 0.259 mmol, 1.1 equiv) were dissolved in 1,4-dioxane:water (5:1, 2.5 mL), and the solution was allowed to react at 80 °C under nitrogen for 2 h. After the reaction was completed, purification was performed by thin layer chromatography (dichloromethane:methanol = 5:1) to give **compound 2** as a yellow solid (60 mg, yield: 37.65%). m/z (ES+), [M+H]+ = 546.

**Compound 2** was subjected to chiral resolution (column: CHIRALPAK IG, 2 × 25 cm, 5 µm; mobile phase A: Hex (0.2% DEA) -- HPLC, mobile phase B: EtOH:DCM = 1:1 -- HPLC; flow rate: 20 mL/min; gradient: 30% B to 30% B within 10 min; wavelength: 220/254 nm; RT1 (min): 5.18; RT2 (min): 7.74; sample solvent: EtOH:DCM = 1:1 -- HPLC; injection volume: 0.7 mL; number of runs: 4) to give compounds **3A** (25 mg) and **3B** (30 mg).

**Compound 3A** (25 mg, 0.045 mmol, 1 equiv) was dissolved in dichloromethane (2.5 mL), and trifluoroacetic acid (1.5 mL) was added. After nitrogen purging in the reaction flask, the mixture was stirred at room temperature for 1 h. After the reaction was completed, the reaction solution was concentrated by rotary evaporation. The resulting crude product was purified by preparative high-pressure chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 30% B to 60% B within 7 min, 60% B; wavelength: 220 nm; RT1 (min): 7.07) to give compound A-32 as a white solid (4.1 mg, yield: 20%). m/z (ES+), [M+H]⁺ = 446. ¹H NMR (400 MHz, DMSO-*d*₆) δ (ppm): 12.60 (s, 1H), 8.65 (s, 1H), 8.21 (s, 1H), 8.14 (s, 1H), 7.78 (s, 1H), 7.46 (s, 1H), 4.07-4.2 (m, 1H), 4.01-4.04 (m, 2H), 3.77-3.80 (m, 1H), 3.68 (s, 1H), 3.51-3.57 (m, 4H), 2.94 (s, 3H), 2.08-2.33 (m, 4H), 1.54-1.77 (m, 2H).¹⁹F NMR (376 MHz, DMSO-*d*₆): δ (ppm): -55.07.

**Compound 3B** (30 mg, 0.073 mmol, 1 equiv) was dissolved in dichloromethane (2.5 mL), and trifluoroacetic acid (1.5 mL) was added. After nitrogen purging in the reaction flask, the mixture was stirred at room temperature for 1 h. After the reaction was completed, the reaction solution was concentrated by rotary evaporation. The resulting crude product was subjected to preparative high-pressure chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 35% B to 43% B within 7 min, 43% B; wavelength: 220 nm; RT1 (min): 7.58) to give compound A-33 as a white solid (3.3 mg, yield: 10.09%). m/z (ES+), [M+H]+ = 446. ¹H NMR (400 MHz, DMSO-*d*₆) δ (ppm): 12.60 (s, 1H), 8.65 (s, 1H), 8.21 (s, 1H), 8.14 (s, 1H), 7.78 (s, 1H), 7.46 (s, 1H), 4.07-4.2 (m, 1H), 4.01-4.04 (m, 2H), 3.77-3.80 (m, 1H), 3.68 (s, 1H), 3.51-3.57 (m, 4H), 2.94 (s, 3H), 2.08-2.33 (m, 4H), 1.54-1.77 (m, 2H).¹⁹F NMR (376 MHz, DMSO-*d*₆): δ (ppm): -55.07.

### Example 30 Synthesis of Compounds A-34 and A-35

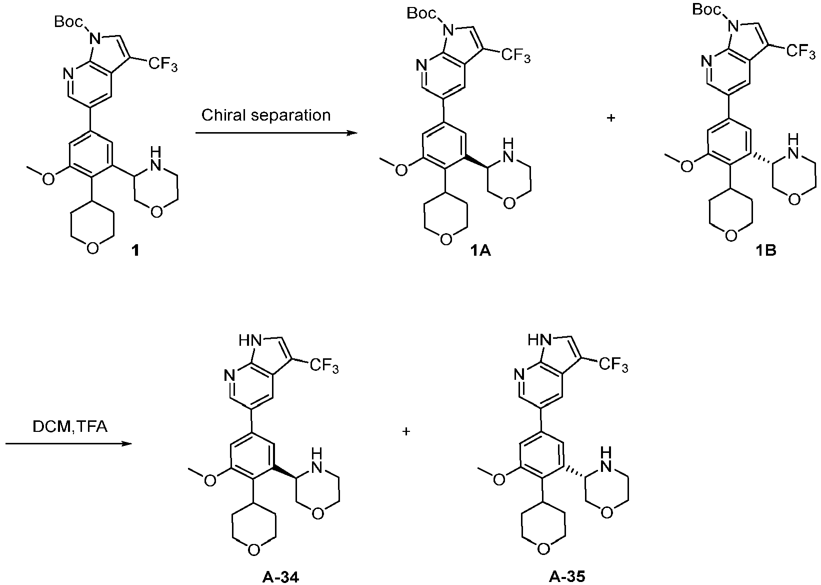

**Compound 1 was synthesized by referring to intermediate 2 in Example 30.**

**Compound 1** was subjected to chiral resolution (column: CHIRALPAK IG, 2 × 25 cm, 5 µm; mobile phase A: Hex (0.5% 2 M NH₃-MeOH) -- HPLC, mobile phase B: EtOH:DCM = 1:1 -- HPLC; flow rate: 20 mL/min; gradient: 30% B to 30% B within 9 min; wavelength: 220/254 nm; RT1 (min): 4.58; RT2 (min): 6.17; sample solvent: EtOH:DCM = 1:1 -- HPLC; injection volume: 0.45 mL) to give compounds **1A** (18mg) and **1B** (20 mg).

**Compound 1A** (18 mg, 0.032 mmol, 1.0 equiv) was dissolved in dichloromethane (2 mL), and trifluoroacetic acid (1 mL) was added. After nitrogen purging in the reaction flask, the mixture was stirred at room temperature for 0.5 h. After the reaction was completed, the reaction solution was concentrated by rotary evaporation. The resulting crude was purified by preparative high-pressure chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 25% B to 60% B within 7 min, 60% B; wavelength: 220 nm; RT1 (min): 7.40) to give compound A-34 as a white solid (6.7 mg, yield: 44.48%). MS (ESI): m/z = 462. ¹H NMR (400 MHz, DMSO-*d*₆): δ (ppm) 12.62(s, 1H), 8.69 (s, 1H), 8.22 (s, 1H), 8.18 (s, 1H), 7.55 (s, 1H), 4.22 (s, 1H), 3.93-4.00 (m, 2H), 3.91 (s, 3H), 3.79 (d, *J* = 10.8 Hz, 1H), 3.68 (d, *J* = 10.9 Hz, 1H), 3.33-3.50 (m, 4H), 3.10 (s, 2H), 2.99 (s, 2H), 2.43 (d, *J* = 12.7 Hz, 1H), 1.35 (d, *J* = 12.9 Hz, 2H). ¹⁹F NMR (376 MHz, DMSO-*d*₆): δ (ppm) -55.02.

Compound **1B** (20 mg, 0.034 mmol, 1 equiv) was dissolved in dichloromethane (2 mL), and trifluoroacetic acid (1 mL) was added. After nitrogen purging in the reaction flask, the mixture was stirred at room temperature for 0.5 h. After the reaction was completed, the reaction solution was concentrated by rotary evaporation. The resulting crude was purified by preparative high-pressure chromatography (column: XBridge Prep OBD C18 column, 30 × 150 mm, 5 µm; mobile phase A: water (10 mmol/L NH₄HCO₃), mobile phase B: ACN; flow rate: 60 mL/min; gradient: 30% B to 55% B within 7 min, 55% B; wavelength: 220 nm; RT1 (min): 7.37) to give compound A-35 as a white solid (7.9 mg, yield: 52.61%). MS (ESI): m/z =462. ¹H NMR (400 MHz, DMSO-*d*₆): δ (ppm) 12.62(s, 1H), 8.69 (s, 1H), 8.22 (s, 1H), 8.18 (s, 1H), 7.55 (s, 1H), 4.22 (s, 1H), 3.93-4.00 (m, 2H), 3.91 (s, 3H), 3.79 (d, *J* = 10.8 Hz, 1H), 3.68 (d, *J* = 10.9 Hz, 1H), 3.33-3.50 (m, 4H), 3.10 (s, 2H), 2.99 (s, 2H), 2.43 (d, *J* = 12.7 Hz, 1H), 1.35 (d, *J* = 12.9 Hz, 2H). ¹⁹F NMR (376 MHz, DMSO-*d*₆): δ (ppm) -55.03.

### Example 31 Synthesis of Compound A-36

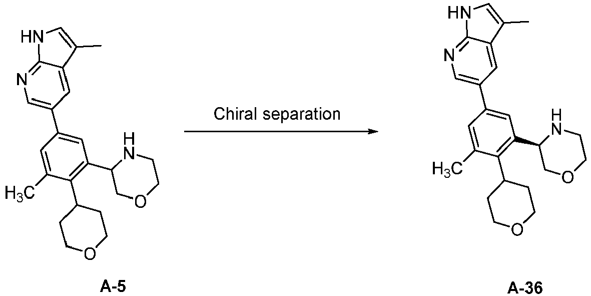

10 mg of compound A-5 was subjected to chiral resolution column CHIRALPAK IG, 2 × 25 cm, 5 µm, mobile phase A: Hex (0.5% 2 M NH₃-MeOH) -- HPLC, mobile phase B: EtOH:DCM = 1:1 -- HPLC, flow rate 20 mL/min; gradient 70% B to 70% B within 10 min; wavelength: 220/254 nm, RT1 (min): 6.31; RT2 (min): 8.58; sample solvent: EtOH:DCM = 1:1 - - HPLC; injection volume: 1.55 mL) to give compound A-36 (3 mg, yield: 30%). ¹H NMR(DMSO-*d*₆, 400 MHz): δ (ppm) 1.55-1.65 (m, 2H), 2.09-2.13 (m, 2H), 2.30 (d, *J*=1.0 Hz, 3H), 2.43 (s, 2H), 2.54 (s, 1H), 2.77 (s, 1H), 2.92 (s, 2H), 3.45-3.48 (m, 3H), 3.55 (s, 1H), 3.66 (s, 1H), 3.77 (dd, *J*=10.4, 2.8 Hz, 1H), 4.01 (dd, *J*=10.0, 4.9 Hz, 2H), 4.14-4.35 (m, 1H), 7.27 (s, 1H), 7.44 (s, 1H), 7.78 (s, 1H), 8.08 (s, 1H), 8.45 (s, 1H), 11.36 (s, 1H).

### Example 32 Synthesis of Compound A-38

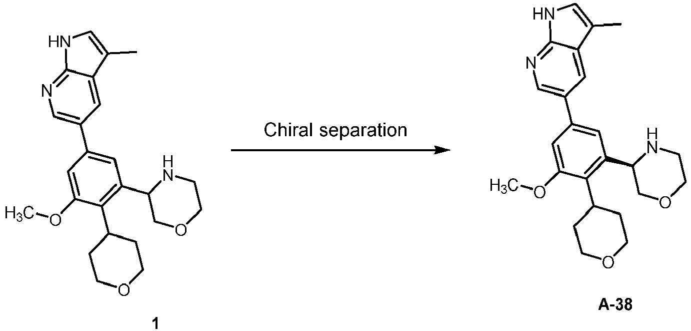

10 mg of compound 1 was subjected to chiral resolution (chromatography column: CHIRALPAK IG, 5 × 15 cm, 10 µm, mobile phase A: CO2, mobile phase B: methanol: dichloromethane = 1:1 (0.1% 2 M ammonium hydroxide solution-methanol); flow rate: 200 mL/min; gradient profile: constant solvent composition 50% B; temperature (°C): 35; pressure (bar): 100; wavelength: 220 nm; RT1 (min): 6.13; RT2 (min): 9.48; sample solvent: methanol: dichloromethane = 1:1 (0.1% 2 M ammonium hydroxide solution-methanol); sample injection volume: 19 mL) to give compound A-38 (4 mg, yield: 40%). MS (ESI) [M+H]+ = 408.2. ¹H NMR (DMSO-*d*₆, 400 MHz): δ (ppm) 1.46-1.73 (m, 2H), 2.09 (s, 2H), 2.32 (s, 3H), 2.35-2.41 (m, 1H), 2.81-3.02 (m, 2H), 3.23-3.25 (m, 2H), 3.41-3.50 (m, 3H), 3.65-3.88 (m, 2H), 3.90 (s, 3H), 3.91-3.99 (m, 2H), 4.17-4.19 (m, 1H), 7.21 (s, 1H), 7.26 (s, 1H), 7.55 (s, 1H), 8.11 (s, 1H), 8.49 (s, 1H), 11.35 (s, 1H).

### Effect Example: Biological Experimental Method

The compounds to be tested were each dissolved in 100% DMSO, with the mother liquor concentration being 10 mM. The liquor was 3-fold diluted at a test starting concentration of 10 µM to yield ten data points, with two replicates set for each point.

The inhibition of the activity of HPK1 kinase by the compound was assayed using an ADP-Glo^{™} platform. The reaction was performed in a 384-well plate containing 0.3 nM HPK1, 5 µM ATP, 0.05 mg/mL MBP, 0-10 µM compound, and 1% DMSO per well. The reaction buffer was as follows: 50 mM HEPES, 10 mM MgCl₂, 1 mM EGTA, 1 mM DTT, 0.01% Brij 35, pH = 7.5. The compound and the kinase were incubated at 25 °C for 15 min, and a substrate and ATP were added to initiate the reaction. After 1 h of reaction at 25 °C, the ADP-Glo^{™} reagent was added, and the reaction was terminated, followed by incubation at 25 °C for 1 h. The kinase detection reagent was added, and after 1 h of incubation at 25 °C, the chemiluminescence signal was detected. Based on this reading, inhibition percentage was calculated, and IC₅₀ for the compound was calculated using four-parameter fitting. The results are shown in Table 1:

| Compound | IC₅₀ |
|---|---|
| A-3 | B |
| A-6 | A |
| A-5 | A |
| A-7 | C |
| A-8 | C |
| A-9 | B |
| A-10 | C |
| A-11 | C |
| A-12 | A |
| A-13 | B |
| A-14 | A |
| A-15 | A |
| A-16 | C |
| A-18 | A |
| A-19 | C |
| A-20 | A |
| A-21 | B |
| A-22 | A |
| A-23 | B |
| A-24 | B |
| A-25 | A |
| A-26 | C |
| A-27 | A |
| A-28 | C |
| A-29 | A |
| A-30 | A |
| A-31 | C |
| A-32 | B |
| A-33 | A |
| A-34 | A |
| A-35 | C |
| A-36 | A |
| A-37 | A |
| A-38 | A |
| A-39 | A |

wherein A represents 0.01 nM ≤ IC₅₀ < 5 nM; B represents 5 nM ≤ IC₅₀ < 10 nM; and C represents 10 nM ≤ IC₅₀ < 100 nM.

### Mouse Pharmacokinetic (PK) Experiment

Male CD1 mice (5-8 weeks old) were subjected to intravenous (IV) administration by bolus injection via tail vein at a dosage level of 1 mg/kg, or subjected to oral (PO) administration via gavage at dosage levels of 5 mg/kg, 30 mg/kg and 100 mg/kg. For the IV and 5 mg/kg PO dose groups, the compounds were formulated in a vehicle of 2% DMSO + 98% (containing 10% HP-β-CD normal saline). For the 30 mg/kg and 100 mg/kg PO dose groups, the compounds were formulated in a vehicle of 0.5% MC + 0.1% Tween 80 aqueous solution. All animals had free access to food and water during the experiment. Blood samples were collected via the dorsal metatarsal vein at 0.033, 0.083, 0.25, 0.5, 1, 2, 4, 8, 12, and 24 h (IV), or 0.083, 0.25, 0.5, 1, 2, 4, 8, 12, and 24 h (PO) after administration, wherein approximately 0.03 mL (n =3 mice) of blood was taken per time point and placed in a tube containing EDTA-K2 as an anticoagulant. After 5 min of centrifugation at 4000× g, the resulting plasma samples were analyzed by an LC-MS/MS method, and PK parameters were calculated by a non-compartment model analysis method using WinNonlin software (Phoenix^{™}, version 8.3).

After intravenous administration at a dosage level of 1 mg/kg, some compounds of the present disclosure showed clearance (CL) below 30 mL/min/kg, and some compounds even showed clearance (CL) below 10 mL/min/kg.

After intravenous administration at a dosage level of 1 mg/kg, some compounds of the present disclosure showed an area under the drug-time curve (AUC₋ₗₐₛₜ) above 500 ng × h/mL, and some compounds even showed an area under the drug-time curve (AVC₋ₗₐₛₜ) above 1000 ng × h/mL.

After oral administration at a dosage level of 5 mg/kg, some compounds of the present disclosure showed a bioavailability (F) above 70%, and some compounds even showed a bioavailability (F) above 100%.

### Inhibitory Effects of Compounds on Human hERG Potassium Ion Channel

The potential inhibitory effects of the compounds on the human hERG potassium ion channel were assessed by an automated patch-clamp system. Cisapride was used as a positive control, and a CHO cell line stably expressing the hERG gene was used as an experimental material. Cells were cultured in a medium containing F12 (HAM), 10% FBS, 100 U/mL penicillin-streptomycin, 100 µg/mL hygromycin and 100 µg/mL G418. Inactivation was removed by depolarizing the membrane to +30 mV for 4.8 s and then restoring the voltage to - 50 mV for 5.2 s, so as to generate hERG currents and measure the inactivation tail currents. The sampling interval was 15 s, and the maximum amount of tail current magnitude would be used to determine the hERG current amplitude. IC₅₀ values were obtained by fitting the hERG inhibition percentage and compound concentration data to dose response curves using Graphpad Prism 8.0.

Some compounds of the present disclosure showed IC₅₀ values above 6 µM for inhibitory effects on the human hERG potassium ion channel, and some compounds showed IC₅₀ values even above 15 µM for inhibitory effects on the human hERG potassium ion channel.

## Claims

1. A compound having a structure of formula I or a pharmaceutically acceptable form thereof: 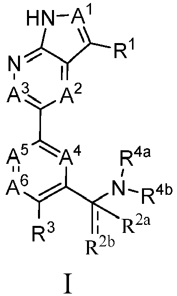 wherein,
A¹ and A³ are each independently selected from N or CR¹⁻¹, and R¹⁻¹ is selected from H, halogen, CN or C₁₋₄ alkyl, wherein the C₁₋₄ alkyl is optionally substituted with one or more halogens;
A² is selected from N or CR²⁻¹, and R²⁻¹ is selected from H, OH, halogen, CN, C₁₋₄ alkyl, C₁₋₆ alkoxy or C₃₋₆ cycloalkyl, wherein the C₁₋₄ alkyl, the C₁₋₆ alkoxy or the C₃₋₆ cycloalkyl is optionally substituted with one or more R³⁻¹, each R³⁻¹ is independently selected from oxo, halogen, OH, CN, C₁₋₆ alkyl, C₁₋₆ alkoxy, C₂₋₆ alkenyl or NR³⁻¹⁻¹R³⁻¹⁻², and R³⁻¹⁻¹ and R³⁻¹⁻² are each independently selected from H or C₁₋₆ alkyl;
A⁴ is selected from N or CR⁴⁻¹, and R⁴⁻¹ is selected from H, halogen, CN, OH, NH₂, -NH-CH₃, -N(CH₃)₂, C₁₋₄ alkyl, C₁₋₄ alkoxy or C₃₋₆ cycloalkyl, wherein the C₁₋₄ alkyl, the C₁₋₄ alkoxy or the C₃₋₆ cycloalkyl is optionally substituted with one or more halogens;
A⁵ is selected from N or CR⁵⁻¹, and R⁵⁻¹ is selected from H, halogen, CN, OH, NH₂, -NH-CH₃, -N(CH₃)₂, C₁₋₄ alkyl, C₁₋₄ alkoxy, C₃₋₆ cycloalkyl, C₂₋₆ alkenyl or C₂₋₆ alkynyl, wherein the C₁₋₄ alkyl, the C₁₋₄ alkoxy, the C₃₋₆ cycloalkyl, the C₂₋₆ alkenyl or the C₂₋₆ alkynyl is optionally substituted with one or more halogens;
A⁶ is selected from N or CR⁶⁻¹, and R⁶⁻¹ is selected from H, halogen, OH, CN, NO₂, oxo, C₁₋₈ alkyl, C₂₋₈ alkenyl, C₂₋₈ alkynyl, C₃₋₈ cycloalkyl, 3- to 8-membered heterocyclyl, 5- to 10-membered heteroaryl, C₆₋₁₀ aryl, C₁₋₆ alkoxy, -OR⁶⁻², -SR⁶⁻², -S(=O)R⁶⁻², -S(=O)₂R⁶⁻², NH₂, - NHR⁶⁻², -NR⁶⁻²R⁶⁻³, -C(=O)R⁶⁻², -C(=O)OR⁶⁻², -C(=O)NHR⁶⁻², -C(=O)NR⁶⁻²R⁶⁻³, -NR⁶⁻²C(=O)R⁶⁻² or -NR⁶⁻²C(=O)NR⁶⁻²R⁶⁻³, wherein R⁶⁻² and R⁶⁻³ are each independently selected from H, C₁₋₈ alkyl, C₂₋₈ alkynyl, C₃₋₈ cycloalkyl, 3- to 8-membered heterocyclyl, 5- to 10-membered heteroaryl or C_{6-1O} aryl, wherein the C₁₋₈ alkyl, the C₂₋₈ alkenyl, the C₂₋₈ alkynyl, the C₃₋₈ cycloalkyl, the 3- to 8-membered heterocyclyl, the 5- to 10-membered heteroaryl, the C₆₋₁₀ aryl or the C₁₋₆ alkoxy is optionally substituted with one or more substituents independently selected from H, halogen, CN, oxo, -NR⁶⁻¹⁻¹R⁶⁻¹⁻², -OR⁶⁻¹⁻¹, -SR⁶⁻¹⁻¹, -S(=O)R⁶⁻¹⁻¹ or -S(=O)₂R⁶⁻¹⁻¹, and R⁶⁻¹⁻¹ and R⁶⁻¹⁻² are each independently selected from H, C₁₋₄ alkyl, -C₁₋₄ alkylene-C₁₋₄ alkoxy, C₂₋₆ alkenyl or C₂₋₆ alkynyl;
R¹ is selected from H, halogen, CN, -NR^{6e}R^{6f}, -C(=O)OR⁶⁻², -C(=O)NR^{6g}R^{6h}, C₁₋₄ alkyl, C₃₋₈ cycloalkyl or 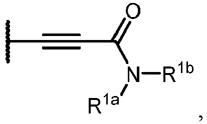 wherein the C₁₋₄ alkyl or the C₃₋₈ cycloalkyl is optionally substituted with one or more substituents independently selected from CN, OH, halogen, CF₃, - NR^{6e}R^{6f} or -C(=O)NR^{6g}R^{6h};
R^{1a} and R^{1b} are each independently selected from H, C₁₋₈ alkyl, C₂₋₈ alkenyl, C₂₋₈ alkynyl, C₃₋₈ cycloalkyl, 3- to 8-membered heterocyclyl, 5- to 10-membered heteroaryl, C₆₋₁₀ aryl, -OR^{1c}, -NR^{1c}R^{1d}, -S(=O)₂R^{1c}, -S(=O)₂NR^{1c}R^{1d}, -C(=O)R^{1c}, -C(=O)NR^{1c}R^{1d}, -NR^{1c}C(=O)R^{1d} or - NR^{1c}S(=O)₂R^{1d};
R^{1c} and R^{1d} are each independently selected from H, C₁₋₈ alkyl, C₂₋₈ alkynyl, C₃₋₈ cycloalkyl, 3- to 8-membered heterocyclyl, 5- to 10-membered heteroaryl or C₆₋₁₀ aryl, wherein the C₁₋₈ alkyl, the C₂₋₈ alkynyl, the C₃₋₈ cycloalkyl, the 3- to 8-membered heterocyclyl, the 5- to 10-membered heteroaryl or the C₆₋₁₀ aryl is optionally substituted with one or more substituents independently selected from H, halogen, CN, oxo, -NR⁶⁻¹⁻¹R⁶⁻¹⁻², -OR⁶⁻¹⁻¹, -SR⁶⁻¹⁻¹, -S(=O)R⁶⁻¹⁻¹ or -S(=O)₂R⁶⁻¹⁻¹, and R⁶⁻¹⁻¹ and R⁶⁻¹⁻² are each independently selected from H, C₁₋₄ alkyl, -C₁₋₄ alkylene-C₁₋₄ alkoxy, C₂₋₆ alkenyl or C₂₋₆ alkynyl;
or, R^{1a} and R^{1b}, together with the atoms to which they are attached, form a 5- to 8-membered ring comprising 1 or 2 heteroatoms independently selected from N, O or S;
R^{6e}, R^{6f}, R^{6g} and R^{6h} are each independently selected from H, -S(=O)₂R⁷, C₃₋₆ cycloalkyl or C₁₋₆ alkyl, wherein the C₃₋₆ cycloalkyl is optionally substituted with one or more substituents independently selected from -OR⁵, Het^{g} or Het^{e}, wherein the C₁₋₆ alkyl is optionally substituted with one or more substituents independently selected from -OR⁵, -NR^{9a}C(=O)R^{9b}, Het^{g} or Het^{e}, and two substituents on the same carbon atom of the C₁₋₆ alkyl optionally together form a C₃₋₆ cycloalkyl or a 3- to 6-membered heterocyclyl comprising 1 or 2 heteroatoms selected from N, O or S;
or, each of pair R^{6e}/R^{6f} and pair R^{6g}/R^{6h}, together with the N atom to which they are attached, form a 5- to 8-membered heterocyclyl;
each Het^{e} is independently a 5- to 12-membered heteroaryl comprising 1 N atom and optionally 1 or 2 additional heteroatoms independently selected from N, O or S, wherein the heteroaryl is optionally substituted with a substituent selected from Het^{f} or C₁₋₄ alkyl, wherein the C₁₋₄ alkyl is optionally substituted with Het^{f};
each Het^{g} is independently a 4- to 12-membered heterocyclyl comprising 1, 2 or 3 heteroatoms independently selected from N, O or S, wherein the heterocyclyl is optionally substituted with a substituent selected from oxo, Het^{f} or C₁₋₄ alkyl, wherein the C₁₋₄ alkyl is optionally substituted with Het^{f};
each Het^{f} is independently a 4- to 12-membered heterocyclyl comprising 1, 2 or 3 heteroatoms independently selected from N, O or S, wherein the heterocyclyl is optionally substituted with C₁₋₄ alkyl;
R^{9a} is selected from H or C₁₋₄ alkyl;
R^{9b} is selected from H or C₁₋₄ alkyl, wherein the C₁₋₄ alkyl is optionally substituted with 1, 2 or 3 halogen atoms;
R^{2a} is H;
R^{2b} is selected from H or methyl;
R^{4a} is selected from H, C₁₋₄ alkyl or C₃₋₆ cycloalkyl;
R^{4b} is selected from H, C₁₋₄ alkyl, C₃₋₆ cycloalkyl, or a 4- to 12-membered heterocyclyl comprising 1, 2 or 3 heteroatoms independently selected from N, O or S;
or, R^{2b} and R^{4b}, together with the atoms to which they are attached, form a 5- to 12-membered heteroaryl or a 4- to 12-membered heterocyclyl, wherein the heteroaryl or the heterocyclyl each comprises 1 N atom and optionally 1 or 2 additional heteroatoms independently selected from N, O or S; wherein on one or more C atoms, the heteroaryl or the heterocyclyl is optionally substituted with 1, 2 or 3 substituents independently selected from OH, CN, halogen, R⁷, -OR⁷, -S(=O)₂R⁷, -C(=O)R⁷, -NR^{6c}R^{6d}, -C(=O)NR^{6a}R^{6b} or Het^{c}; wherein on an optional additional N atom, the heteroaryl or the heterocyclyl is optionally substituted with 1 or 2 substituents independently selected from R⁷, -S(=O)₂R⁷, -C(=O)R⁷, -C(=O)NR^{6a}R^{6b} or Het^{d};
the dotted bond towards R^{2b} is a bond optionally present when R^{2b} and R^{4b} form a ring together;
when R^{2b} and R^{4b} form the ring together, R^{4a} is H; when the dotted bond towards R^{2b} is a bond, R^{2a} is absent; or,
R^{4a} and R^{4b}, together with the N atom to which they are attached, form a 5- to 12-membered heteroaryl or a 4- to 12-membered heterocyclyl, wherein the heteroaryl or the heterocyclyl each comprises 1 N atom and optionally 1 or 2 additional heteroatoms independently selected from N, O or S; wherein on one or more C atoms, the heteroaryl or the heterocyclyl is optionally substituted with 1, 2 or 3 substituents independently selected from OH, CN, halogen, R⁷, -OR⁷, -S(=O)₂R⁷, -C(=O)R⁷, -NR^{6c}R^{6d}, -C(=O)NR^{6a}R^{6b} or Het^{c}; wherein on the N atom, the heteroaryl or the heterocyclyl is optionally substituted with 1 or 2 substituents independently selected from R⁷, -S(=O)₂R⁷, -C(=O)R⁷, -C(=O)NR^{6a}R^{6b} or Het^{d};
when R^{4a} and R^{4b} form the ring together, R^{2a} is H and R^{2b} is H;
each Het^{c} is independently a 4- to 12-membered heterocyclyl comprising 1, 2 or 3 heteroatoms independently selected from N, O or S;
each Het^{d} is independently a 4- to 12-membered heterocyclyl comprising 1, 2 or 3 heteroatoms independently selected from N, O or S;
R³ is selected from CN, halogen, -C(=O)NR^{8a}R^{8b}, -CH₂NR^{8c}R^{8d}, Het^{a}, Het^{b}, -CH₂-Het^{a}, - CH₂-Het^{b}, -CH(R⁷⁻¹)-Het^{a}, -CH(R⁷⁻¹)-Het^{h}, -P(=O)-(C₁₋₄ alkyl)₂, -S(=O)₂-C₁₋₄ alkyl, - S(=O)(=NR^{x})-C₁₋₄ alkyl, C₁₋₆ alkyl or C₃₋₆ cycloalkyl, wherein the C₁₋₆ alkyl or the C₃₋₆ cycloalkyl is optionally substituted with 1 or 2 substituents independently selected from halogen, OH, CN or -O-C₁₋₄ alkyl;
R^{8a}, R^{8c} and R^{8d} are each independently selected from H, C₁₋₄ alkyl or C₃₋₆ cycloalkyl, wherein the C₁₋₄ alkyl or the C₃₋₆ cycloalkyl is optionally substituted with OH or -O-C₁₋₄ alkyl;
R^{8b} is selected from C₁₋₄ alkyl or C₃₋₆ cycloalkyl, wherein the C₁₋₄ alkyl or the C₃₋₆ cycloalkyl is optionally substituted with OH or -O-C₁₋₄ alkyl;
or, pair R^{8a}/R^{8b} or pair R^{8c}/R^{8d}, together with the N atom to which they are attached, form a 4- to 12-membered heterocyclyl, wherein the heterocyclyl comprises 1 N atom and optionally 1 or 2 additional heteroatoms independently selected from N, O or S; wherein on one or more C atoms, the heterocyclyl is optionally substituted with 1, 2 or 3 substituents independently selected from OH, CN, halogen, R⁷, -OR⁷, -S(=O)₂R⁷, -C(=O)R⁷, -NR^{6c}R^{6d} or -C(=O)NR^{6a}R^{6b}; wherein on the N atom, the heterocyclyl is optionally substituted with 1 or 2 substituents independently selected from R⁷, -S(=O)₂R⁷, -C(=O)R⁷ or -C(=O)NR^{6a}R^{6b};
Het^{b} is a 4- to 12-membered heterocyclyl, wherein the heterocyclyl comprises 1, 2 or 3 heteroatoms independently selected from N, O or S; wherein on one or more C atoms, the heterocyclyl is optionally substituted with 1, 2 or 3 substituents independently selected from OH, CN, halogen, R⁷, -OR⁷, -S(=O)₂R⁷, -C(=O)R⁷, -NR^{6c}R^{6d} or -C(=O)NR^{6a}R^{6b}; wherein on the N atom, the heterocyclyl is optionally substituted with 1 or 2 substituents independently selected from R⁷, -S(=O)₂R⁷, -C(=O)R⁷ or -C(=O)NR^{6a}R^{6b};
each R^{x} is independently selected from H or C₁₋₄ alkyl;
each R⁷ is independently selected from C₁₋₄ alkyl or C₃₋₆ cycloalkyl, wherein the C₁₋₄ alkyl or the C₃₋₆ cycloalkyl is optionally substituted with one or more substituents independently selected from halogen, OH, -O-C₁₋₄ alkyl or CN;
R^{6a}, R^{6b}, R^{6c} and R^{6d} are each independently selected from H, C₃₋₆ cycloalkyl or C₁₋₄ alkyl, wherein the C₃₋₆ cycloalkyl or the C₁₋₄ alkyl is optionally substituted with -OR⁵, and two substituents on the same carbon atom of the C₁₋₄ alkyl optionally together form a C₃₋₆ cycloalkyl;
each R⁵ is independently selected from H or C₁₋₄ alkyl;
each R⁷⁻¹ is independently selected from C₁₋₄ alkyl or C₃₋₆ cycloalkyl, wherein the C₁₋₄ alkyl or the C₃₋₆ cycloalkyl is optionally substituted with one or more halogens;
Het^{a} is 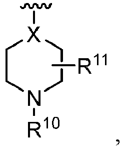 X is selected from N or CH, and R¹⁰ and R¹¹ are each independently selected from H, C₁₋₈ alkyl, C₂₋₈ alkenyl, C₂₋₈ alkynyl or C₃₋₈ cycloalkyl; and
the pharmaceutically acceptable form is selected from pharmaceutically acceptable salts, esters, stereoisomers, tautomers, solvates, oxynitrides, isotopically labeled compounds, metabolites and prodrugs.

2. The compound or the pharmaceutically acceptable form thereof according to claim 1, wherein,
when R¹ is selected from H, halogen, CN, -NR^{6e}R^{6f}, -C(=O)NR^{6g}R^{6h}, C₁₋₄ alkyl or C₃₋₈ cycloalkyl and when A⁴ is CH, A², A³, A⁵ and A⁶ are not all CH and A² is not N.

3. The compound or the pharmaceutically acceptable form thereof according to claim 2, wherein,
A¹ and A³ are each independently selected from N or CR¹⁻¹, R¹⁻¹ is selected from H, halogen, CN, methyl, ethyl or isopropyl, wherein the methyl, the ethyl or the isopropyl is optionally substituted with one or more halogens;
preferably, R¹⁻¹ is selected from H, halogen, CN or methyl; and more preferably, R¹⁻¹ is selected from H, F, Cl, CN or methyl.

4. The compound or the pharmaceutically acceptable form thereof according to claim 2 or 3, wherein,
A² is selected from N or CR²⁻¹, and R²⁻¹ is selected from H, OH, halogen, CN, C₁₋₃ alkyl, C₁₋₃ alkoxy or C₃₋₆ cycloalkyl, wherein the C₁₋₃ alkyl, the C₁₋₃ alkoxy or the C₃₋₆ cycloalkyl is optionally substituted with one or more substituents independently selected from oxo, halogen, OH, CN, NH₂ or methyl;
preferably, R²⁻¹ is selected from H, OH, halogen, CN, CF₃, methoxy, ethoxy, methyl, ethyl or cyclopropyl; and more preferably, R²⁻¹ is selected from H, OH, F, Cl, CN, CF₃, methoxy, ethoxy, methyl, ethyl or cyclopropyl.

5. The compound or the pharmaceutically acceptable form thereof according to any one of claims 2-4, wherein,
A⁴ is selected from N or CR⁴⁻¹, R⁴⁻¹ is selected from H, halogen, CN, OH, NH₂, -NH-CH₃, -N(CH₃)₂, C₁₋₃ alkyl, C₁₋₃ alkoxy or C₃₋₆ cycloalkyl, wherein the C₁₋₃ alkyl, the C₁₋₃ alkoxy or the C₃₋₆ cycloalkyl is optionally substituted with one or more halogens;
preferably, R⁴⁻¹ is selected from H, halogen, CN, OH, NH₂, methyl or methoxy; and more preferably, R⁴⁻¹ is selected from H, F, Cl, CN, OH, NH₂, methyl or methoxy.

6. The compound or the pharmaceutically acceptable form thereof according to any one of claims 2-5, wherein,
A⁵ is selected from N or CR⁵⁻¹, R⁵⁻¹ is selected from H, halogen, CN, OH, NH₂, -NH-CH₃, -N(CH₃)₂, C₁₋₄ alkyl, C₁₋₄ alkoxy or C₃₋₆ cycloalkyl, wherein the C₁₋₄ alkyl, the C₁₋₄ alkoxy or the C₃₋₆ cycloalkyl is optionally substituted with one or more halogens;
preferably, R⁵⁻¹ is selected from H, halogen, CN, OH, NH₂, -NH-CH₃, -N(CH₃)₂, methyl, CF₃, methoxy, trifluoromethoxy or cyclopropyl; and more preferably, R⁵⁻¹ is selected from H, F, Cl, CN, OH, NH₂, -NH-CH₃, -N(CH₃)₂, methyl, CF₃, methoxy, trifluoromethoxy or cyclopropyl.

7. The compound or the pharmaceutically acceptable form thereof according to any one of claims 2-6, wherein,
A⁶ is selected from N or CR⁶⁻¹, and R⁶⁻¹ is selected from H, halogen, OH, CN, NO₂, oxo, C₁₋₈ alkyl, C₃₋₈ cycloalkyl, -OR⁶⁻², -SR⁶⁻², -S(=O)R⁶⁻², -S(=O)₂R⁶⁻², NH₂, -NHR⁶⁻², -NR⁶⁻²R⁶⁻³, - C(=O)R⁶⁻², -C(=O)OR⁶⁻², -C(=O)NHR⁶⁻², -C(=O)NR⁶⁻²R⁶⁻³, -NR⁶⁻²C(=O)R⁶⁻² or -NR⁶⁻²C(=O)NR⁶⁻²R⁶⁻³, wherein R⁶⁻² and R⁶⁻³ are each independently selected from H, C₁₋₈ alkyl or C₃₋₈ cycloalkyl, wherein the C₁₋₈ alkyl or the C₃₋₈ cycloalkyl is optionally substituted with one or more substituents independently selected from H, halogen, CN, oxo, -NR⁶⁻¹⁻1R⁶⁻¹⁻², -OR⁶⁻¹⁻¹, - SR⁶⁻¹⁻¹, -S(-O)R⁶⁻¹⁻¹ or -S(=O)₂R⁶⁻¹⁻¹, and R⁶⁻¹⁻¹ and R⁶⁻¹⁻² are each independently selected from H, methyl or ethyl;
preferably, R⁶⁻¹ is selected from H, halogen, OH, CN, NO₂, oxo, C₁₋₄ alkyl, C₃₋₆ cycloalkyl, -OR⁶⁻², -SR⁶⁻², -S(=O)R⁶⁻², -S(=O)₂R⁶⁻², NH₂, -NHR⁶⁻², -NR⁶⁻²R⁶⁻³, -C(=O)R⁶⁻², -C(=O)OR⁶⁻², - C(=O)NHR⁶⁻², -C(=O)NR⁶⁻²R⁶⁻³, -NR⁶⁻²C(=O)R⁶⁻² or -NR⁶⁻²C(=O)NR⁶⁻²R⁶⁻³, wherein R⁶⁻² and R⁶⁻³ are each independently selected from H, C₁₋₄ alkyl or C₃₋₆ cycloalkyl, wherein the C₁₋₄ alkyl or the C₃₋₆ cycloalkyl is optionally substituted with one or more substituents independently selected from H, halogen, CN, oxo, -NR⁶⁻¹⁻¹R⁶⁻¹⁻², -OR⁶⁻¹⁻¹, -SR⁶⁻¹⁻¹, -S(=O)R⁶⁻¹⁻¹ or -S(=O)₂R⁶⁻¹⁻¹, and R⁶⁻¹⁻¹ and R⁶⁻¹⁻² are each independently selected from H, methyl or ethyl;
more preferably, R⁶⁻¹ is selected from H, halogen, OH, CN, NO₂, oxo, C₁₋₄ alkyl, C₃₋₆ cycloalkyl or C₁₋₄ alkoxy, wherein the C₁₋₄ alkyl, the C₃₋₆ cycloalkyl or the C₁₋₄ alkoxy is optionally substituted with one or more substituents independently selected from H, halogen, CN or oxo;
more preferably, R⁶⁻¹ is selected from H, halogen, OH, CN, NO₂, oxo, methyl, ethyl, CF₃, methoxy, trifluoromethoxy or cyclopropyl; and further preferably, R⁶⁻¹ is selected from H, F, Cl, OH, CN, NO₂, methyl, ethyl, CF₃, methoxy, trifluoromethoxy or cyclopropyl.

8. The compound or the pharmaceutically acceptable form thereof according to any one of claims 2-7, wherein,
R¹ is selected from H, halogen, CN, -C(=O)OR⁶⁻², C₁₋₄ alkyl, C₃₋₈ cycloalkyl or 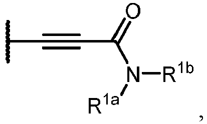 wherein the C₁₋₄ alkyl or the C₃₋₈ cycloalkyl is optionally substituted with one or more substituents independently selected from CN, OH, halogen or CF₃,
R^{1a} and R^{1b} are each independently selected from H, C₁₋₈ alkyl, C₂₋₈ alkenyl, C₂₋₈ alkynyl, C₃₋₈ cycloalkyl, 3- to 8-membered heterocyclyl, 5- to 10-membered heteroaryl or C₆₋₁₀ aryl; R⁶⁻² is selected from H, C₁₋₈ alkyl or C₃₋₈ cycloalkyl,
preferably, R¹ is selected from H, halogen, CN, -C(=O)OR⁶⁻², C₁₋₄ alkyl, C₃₋₆ cycloalkyl or 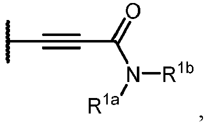 wherein the C₁₋₄ alkyl or the C₃₋₆ cycloalkyl is optionally substituted with one or more substituents independently selected from CN, OH, halogen or CF₃,
and R^{1a} and R^{1b} are each independently selected from H, C₁₋₄ alkyl, C₃₋₆ cycloalkyl or 3- to 6-membered heterocyclyl; R⁶⁻² is selected from H, C₁₋₄ alkyl or C₃₋₆ cycloalkyl,
more preferably, R¹ is selected from H, halogen, CN, methyl, ethyl, cyclopropyl, -CH₂OH, -C(=O)OCH₃, CF₃, 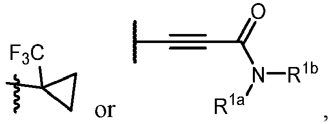 and R^{1a} and R^{1b} are each independently selected from H, methyl or ethyl;
and further preferably, R¹ is selected from H, F, Cl, CN, methyl, ethyl, cyclopropyl, - CH₂OH, -C(=O)OCH₃, CF₃,

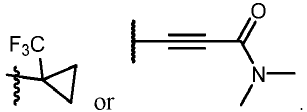

9. The compound or the pharmaceutically acceptable form thereof according to any one of claims 2-8, wherein,
R^{2a} is H,
R^{4a} is H,
R^{2b} and R^{4b}, together with the atoms to which they are attached, form a monocyclic 5-membered heteroaryl, a monocyclic 4-, 5-, 6- or 7-membered heterocyclyl, a bicyclic 6- to 12-membered heteroaryl or a bicyclic 6- to 12-membered heterocyclyl, wherein the heteroaryl or the heterocyclyl each comprises 1 N atom and optionally 1 or 2 additional heteroatoms independently selected from N, O or S, wherein on one or more C atoms, the heteroaryl or the heterocyclyl is optionally substituted with 1, 2 or 3 substituents independently selected from OH, CN, halogen, R⁷, -OR⁷, -S(=O)₂R⁷ or -C(=O)R⁷; wherein on an optional additional N atom, the heteroaryl or the heterocyclyl is optionally substituted with 1 or 2 substituents independently selected from R⁷, -S(=O)₂R⁷ or -C(=O)R⁷,
R⁷ is independently selected from C₁₋₄ alkyl or C₃₋₆ cycloalkyl, wherein the C₁₋₄ alkyl or the C₃₋₆ cycloalkyl is optionally substituted with one or more substituents independently selected from halogen, OH, -O-C₁₋₄ alkyl or CN;
preferably, R^{2b} and R^{4b}, together with the atoms to which they are attached, form a monocyclic 4-, 5-, 6- or 7-membered heterocyclyl, wherein the heterocyclyl comprises 1 N atom and optionally 1 or 2 additional heteroatoms independently selected from N, O or S, wherein on one or more C atoms, the heterocyclyl is optionally substituted with 1, 2 or 3 substituents independently selected from OH, CN, halogen, R⁷, -OR⁷, -S(=O)₂R⁷ or -C(=O)R⁷; wherein on an optional additional N atom, the heterocyclyl is optionally substituted with 1 or 2 substituents independently selected from R⁷, -S(=O)₂R⁷ or -C(=O)R⁷,
and R⁷ is independently selected from methyl, ethyl or cyclopropyl;
more preferably, R^{2b} and R^{4b}, together with the atoms to which they are attached, form a monocyclic 6-membered heterocyclyl, wherein the heterocyclyl comprises 1 N atom and optionally 1 or 2 additional heteroatoms independently selected from N, O or S, wherein on one or more C atoms, the heterocyclyl is optionally substituted with 1, 2 or 3 substituents independently selected from OH, CN, halogen, methyl, ethyl or cyclopropyl; wherein on an optional additional N atom, the heterocyclyl is optionally substituted with 1 or 2 substituents independently selected from methyl, ethyl or cyclopropyl;
particularly preferably, R^{2b} and R^{4b}, together with the atoms to which they are attached, form morpholinyl, piperidinyl or piperazinyl;
or, R^{2a} is H, R^{2b} is H, R^{4a} is selected from H, C₁₋₄ alkyl or C₃₋₆ cycloalkyl, and R^{4b} is selected from H, C₁₋₄ alkyl or C₃₋₆ cycloalkyl; preferably, R^{4a} is methyl, and R^{4b} is methyl;
or, R^{2a} is H, R^{2b} is H, and R^{4a} and R^{4b}, together with the N atom to which they are attached, form a 4- to 12-membered heterocyclyl, wherein the heterocyclyl comprises 1 N atom and optionally 1 or 2 additional heteroatoms independently selected from N, O or S, wherein on one or more C atoms, the heterocyclyl is optionally substituted with 1, 2 or 3 substituents independently selected from OH, CN, halogen, R⁷, -OR⁷, -S(=O)₂R⁷ or -C(=O)R⁷; preferably, R^{4a} and R^{4b}, together with the N atom to which they are attached, form a pyrrolidinyl, wherein on one or more C atoms, the pyrrolidinyl is optionally substituted with 1, 2 or 3 substituents independently selected from OH, CN, halogen, R⁷, -OR⁷, -S(=O)₂R⁷ or -C(=O)R⁷; and more preferably, R^{4a} and R^{4b}, together with the N atom to which they are attached, form

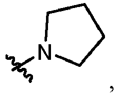

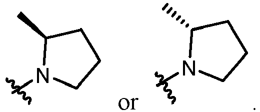

10. The compound or the pharmaceutically acceptable form thereof according to any one of claims 2-9, wherein,
R³ is selected from CN, halogen, Het^{a}, Het^{b}, -CH₂-Het^{a}, -CH₂-Het^{b}, -CH(R⁷⁻¹)-He^{a}, - CH(R⁷⁻¹)-Het^{b}, -P(=O)-(C₁₋₄ alkyl)₂, -S(=O)₂-C₁₋₄ alkyl, C₁₋₆ alkyl or C₃₋₆ cycloalkyl, wherein the C₁₋₆ alkyl or the C₃₋₆ cycloalkyl is optionally substituted with 1 or 2 substituents independently selected from halogen, OH, CN or -O-C₁₋₄ alkyl,
Het^{b} is selected from a monocyclic 4-, 5-, 6- or 7-membered heterocyclyl or a bicyclic 6- to 12-membered heterocyclyl, wherein the heterocyclyl comprises 1, 2 or 3 heteroatoms independently selected from N, O or S, wherein on one or more C atoms, the heterocyclyl is optionally substituted with 1, 2 or 3 substituents independently selected from OH, CN, halogen, methyl or ethyl,
Het^{a} is 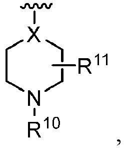 X is selected from N or CH, R¹⁰ and R¹¹ are each independently selected from H, C₁₋₈ alkyl, C₂₋₈ alkenyl, C₂₋₈ alkynyl or C₃₋₈ cycloalkyl,
R⁷⁻¹ is independently selected from C₁₋₄ alkyl or C₃₋₆ cycloalkyl, wherein the C₁₋₄ alkyl or the C₃₋₆ cycloalkyl is optionally substituted with one or more halogens;
preferably, R³ is selected from CN, halogen, Het^{b}, -CH₂-Het^{b}, -CH(R⁷⁻¹)-Het^{b}, -P(=O)-(C₁₋₄ alkyl)₂, -S(=O)₂-C₁₋₄ alkyl, C₁₋₄ alkyl or C₃₋₆ cycloalkyl, wherein the C₁₋₄ alkyl or the C₃₋₆ cycloalkyl is optionally substituted with 1 or 2 substituents independently selected from halogen, OH, CN or -O-C₁₋₄ alkyl,
Het^{b} is selected from a monocyclic 6-membered heterocyclyl, the heterocyclyl comprising 1, 2 or 3 heteroatoms independently selected from N, O or S, wherein on one or more C atoms, the heterocyclyl is optionally substituted with 1, 2 or 3 substituents independently selected from OH, CN, halogen, methyl or ethyl,
R⁷⁻¹ is independently selected from C₁₋₄ alkyl or C₃₋₆ cycloalkyl, wherein the C₁₋₄ alkyl or the C₃₋₆ cycloalkyl is optionally substituted with one or more halogens;
more preferably, R³ is selected from CN, halogen, Het^{b}, -CH₂-Het^{b}, -P(=O)-(C₁₋₄ alkyl)₂, - S(=O)₂-C₁₋₄ alkyl, C₁₋₄ alkyl or C₃₋₆ cycloalkyl, and Het^{b} is selected from a monocyclic 6-membered heterocyclyl, wherein the heterocyclyl comprises 1, 2 or 3 heteroatoms independently selected from N, O or S;
particularly preferably, R³ is selected from CN, halogen, 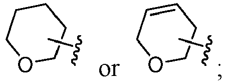 and further preferably, R³ is selected from CN, F, Cl,

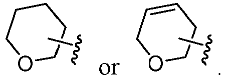

11. The compound or the pharmaceutically acceptable form thereof according to any one of claims 1-10, being a compound having a structure of formula I-1 or formula I-2 or a pharmaceutically acceptable form thereof: 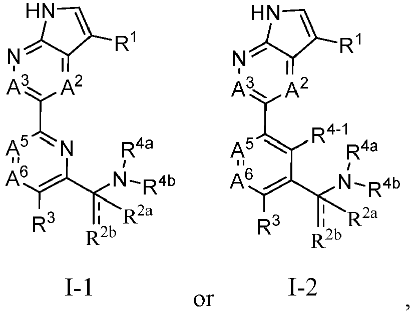
wherein A², A³, A⁵, A⁶, R¹, R⁴⁻¹, R^{2a}, R^{2b}, R^{4a}, R^{4b} and R³ are as defined in claims 1-10,
provided that when R¹ is selected from H, halogen, CN, -NR^{6e}R^{6f}, -C(=O)NR^{6g}R^{6h}, C₁₋₄ alkyl or C₃₋₈ cycloalkyl and when R⁴⁻¹ is H, A², A³, A⁵ and A⁶ are not all CH and A² is not N.

12. The compound or the pharmaceutically acceptable form thereof according to any one of claims 1-10, being a compound having a structure of formula I-3 or formula I-4 or a pharmaceutically acceptable form thereof: 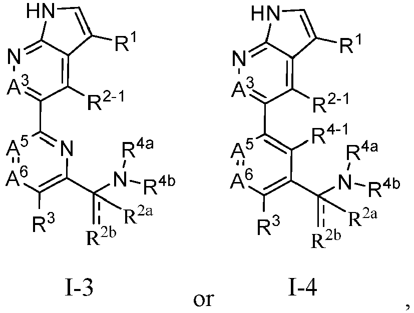
wherein A³, A⁵, A⁶, R¹, R²¹, R⁴⁻¹, R^{2a}, R^{2b}, R^{4a}, R^{4b} and R³ are as defined in claims 1-10,
provided that when R¹ is selected from H, halogen, CN, -NR^{6e}R^{6f}, -C(=O)NR^{6g}R^{6h}, C₁₋₄ alkyl or C₃₋₈ cycloalkyl and when R⁴⁻¹ is H, A³, A⁵ and A⁶ are not all CH, or R²⁻¹ is not H.

13. The compound or the pharmaceutically acceptable form thereof according to any one of claims 1-10, being a compound having a structure selected from formula I-5 to formula I-7 or a pharmaceutically acceptable form thereof: 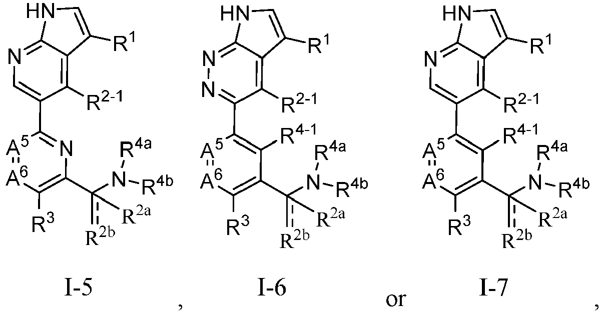
wherein A⁵, A⁶, R¹, R²⁻¹, R⁴⁻¹, R^{2a}, R^{2b}, R^{4a}, R^{4b} and R³ are as defined in claims 1-10,
provided that in formula I-7, when R¹ is selected from H, halogen, CN, -NR^{6e}R^{6f}, - C(=O)NR^{6g}R^{6h}, C₁₋₄ alkyl or C₃₋₈ cycloalkyl and when R⁴⁻¹ is H, A⁵ and A⁶ are not all CH, or R²⁻¹ is not H.

14. The compound or the pharmaceutically acceptable form thereof according to any one of claims 1-10, being a compound having a structure selected from formula I-8 to formula I-10 or a pharmaceutically acceptable form thereof: 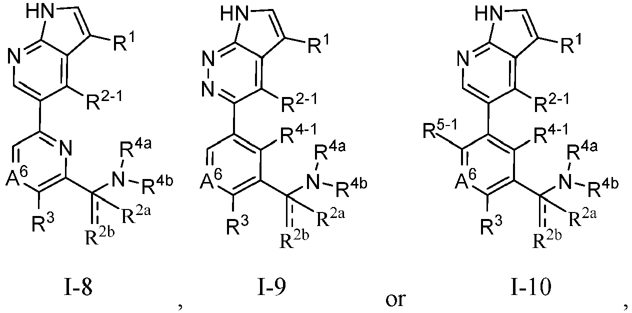
wherein A⁶, R¹, R²⁻¹, R⁴⁻¹, R⁵⁻¹, R^{2a}, R^{2b}, R^{4a}, R^{4b} and R³ are as defined in claims 1-10,
provided that in formula I-10, when R¹ is selected from H, halogen, CN, -NR^{6e}R^{6f}, - C(=O)NR^{6g}R^{6h}, C₁₋₄ alkyl or C₃₋₈ cycloalkyl and when R⁴⁻¹ is H, A⁶ is not CH, or R²¹ is not H, or R⁵⁻¹ is not H.

15. The compound or the pharmaceutically acceptable form thereof according to any one of claims 1-10, being a compound having a structure selected from formula I-11 to formula 1-13 or a pharmaceutically acceptable form thereof: 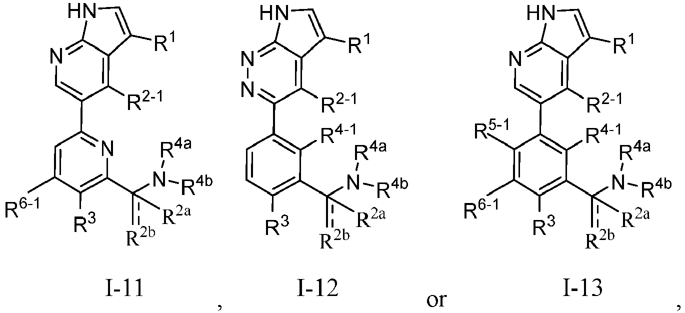
wherein R¹, R²⁻¹, R⁴⁻¹, R⁵⁻¹, R⁶⁻¹, R^{2a}, R^{2b}, R^{4a}, R^{4b} and R³ are as defined in claims 1-10,
provided that in formula I-13, when R¹ is selected from H, halogen, CN, -NR^{6e}R^{6f}, - C(=O)NR^{6g}R^{6h}, C₁₋₄ alkyl, or C₃₋₈ cycloalkyl and when R⁴⁻¹ is H, R²¹ is not H, or R⁵⁻¹ is not H, or R⁶⁻¹ is not H.

16. The compound or the pharmaceutically acceptable form thereof according to any one of claims 1-10, being a compound having a structure of formula I-13-1 or a pharmaceutically acceptable form thereof: 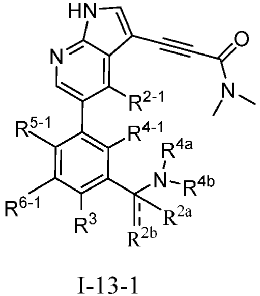 wherein R²⁻¹, R⁴⁻¹, R⁵⁻¹, R⁶⁻¹, R^{2a}, R^{2b}, R^{4a}, R^{4b} and R³ are as defined in claims 1-10.

17. The compound or the pharmaceutically acceptable form thereof according to any one of claims 1-10, being a compound having a structure selected from formula I-14 to formula I-16 or a pharmaceutically acceptable form thereof: 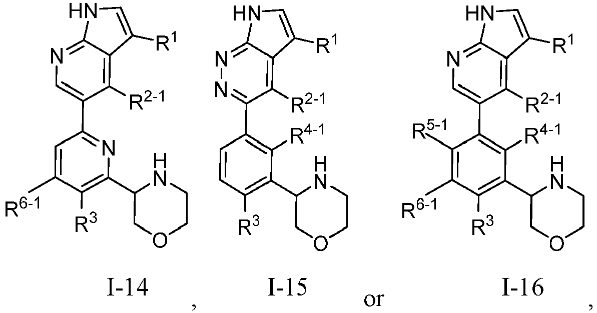
wherein R¹, R²⁻¹, R⁴⁻¹, R⁵⁻¹, R⁶⁻¹ and R³ are as defined in claims 1-10,
provided that in formula I-16, when R¹ is selected from H, halogen, CN, -NR^{6e}R^{6f}, - C(=O)NR^{6g}R^{6h}, C₁₋₄ alkyl, or C₃₋₈ cycloalkyl and when R⁴⁻¹ is H, R²⁻¹ is not H, or R⁵⁻¹ is not H, or R⁶⁻¹ is not H.

18. The compound or the pharmaceutically acceptable form thereof according to any one of claims 1-10, being a compound having a structure selected from formula I-17 to formula I-20 or a pharmaceutically acceptable form thereof: 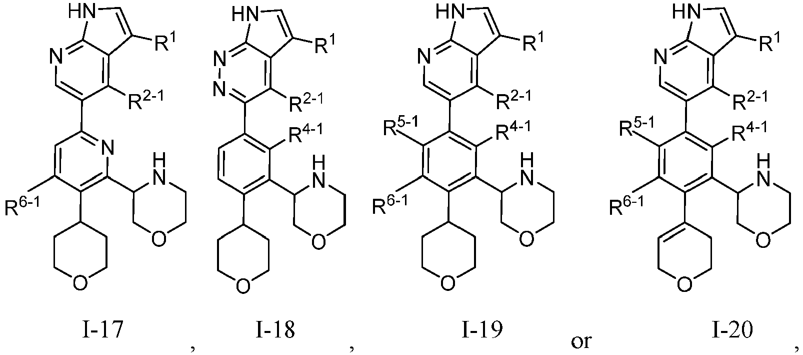
wherein R¹, R²⁻¹, R⁴⁻¹, R⁵⁻¹ and R⁶⁻¹ are as defined in claims 1-10,
provided that in formula I-19 and formula I-20, when R¹ is selected from H, halogen, CN, -NR^{6e}R^{6f}, -C(=O)NR^{6g}R^{6h}, C₁₋₄ alkyl or C₃₋₈ cycloalkyl and when R⁴⁻¹ is H, R²⁻¹ is not H, or R⁵⁻¹ is not H, or R⁶⁻¹ is not H.

19. The compound or the pharmaceutically acceptable form thereof according to any one of claims 2-18, selected from the following compounds or pharmaceutically acceptable forms thereof: 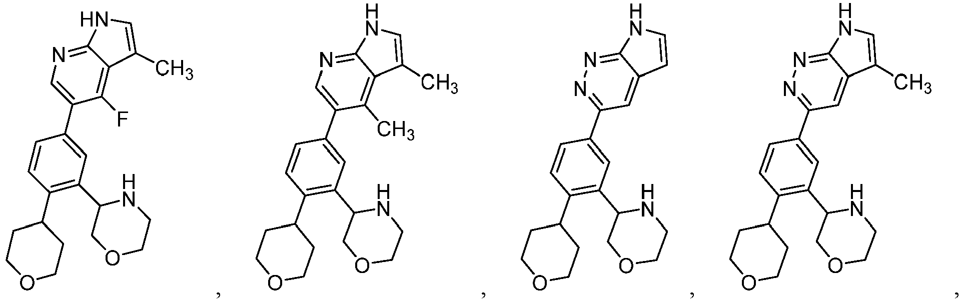 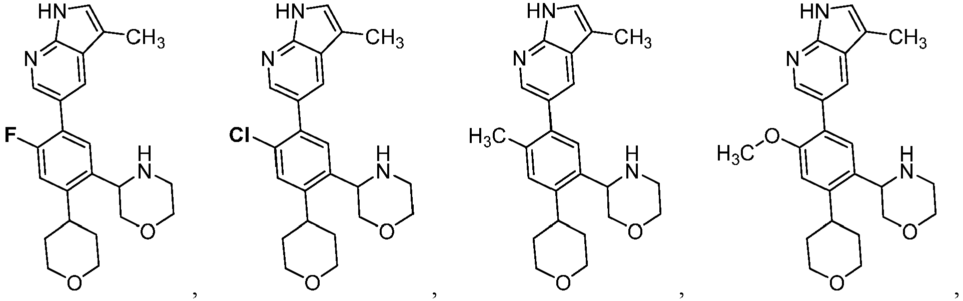 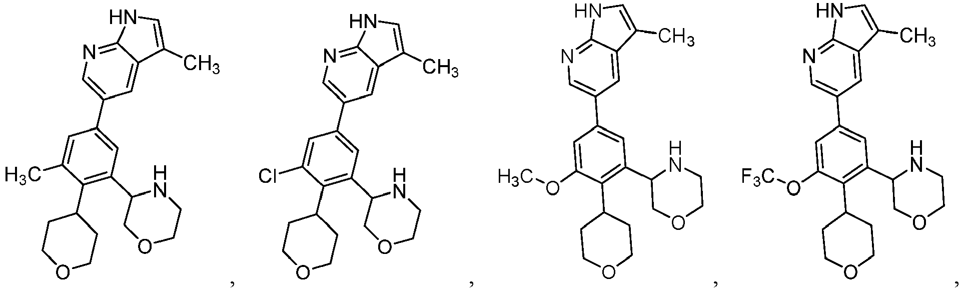 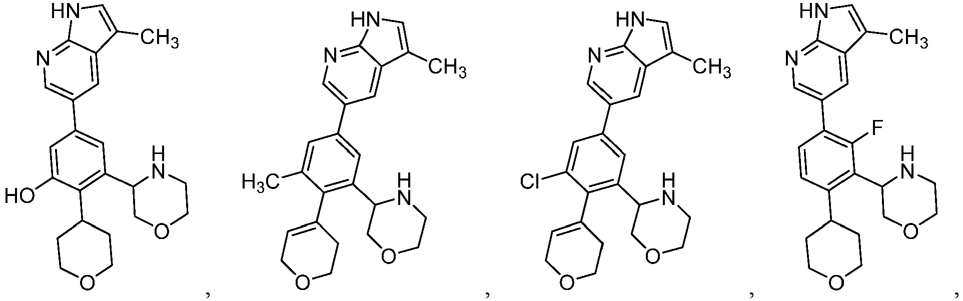 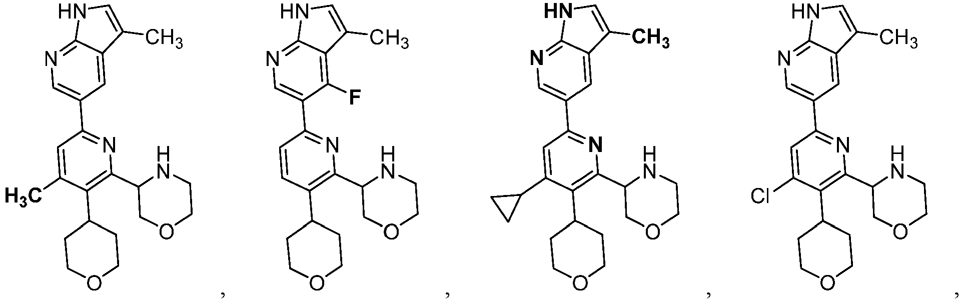 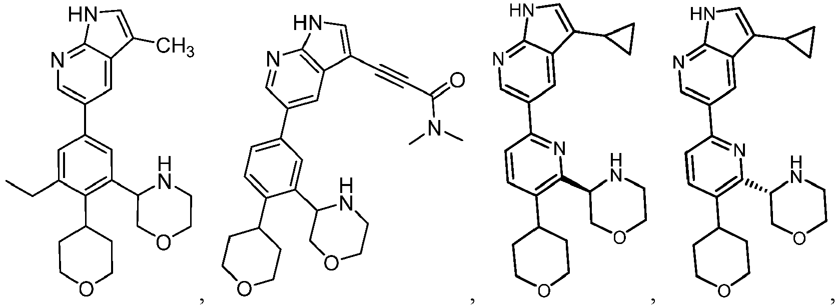 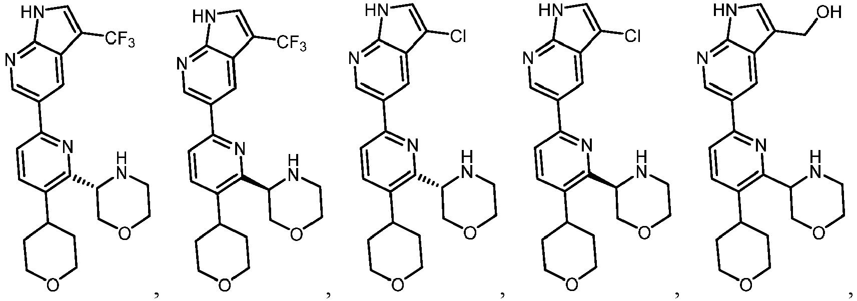 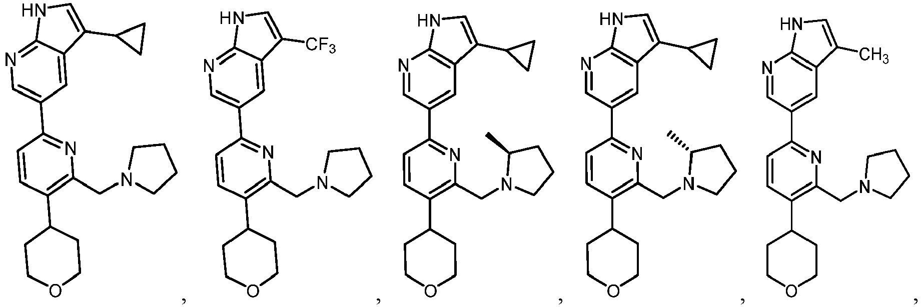 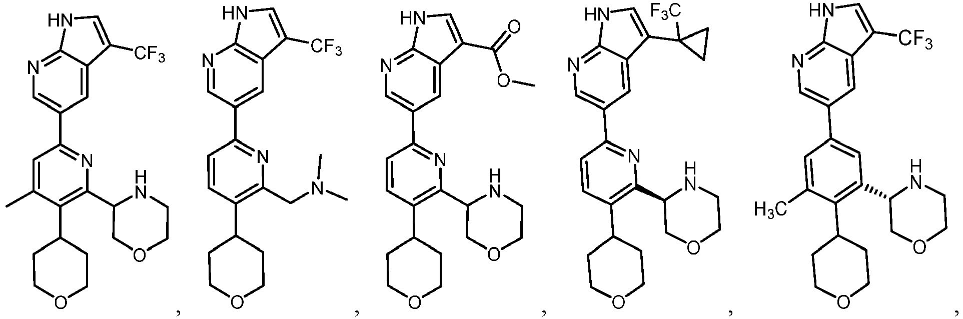 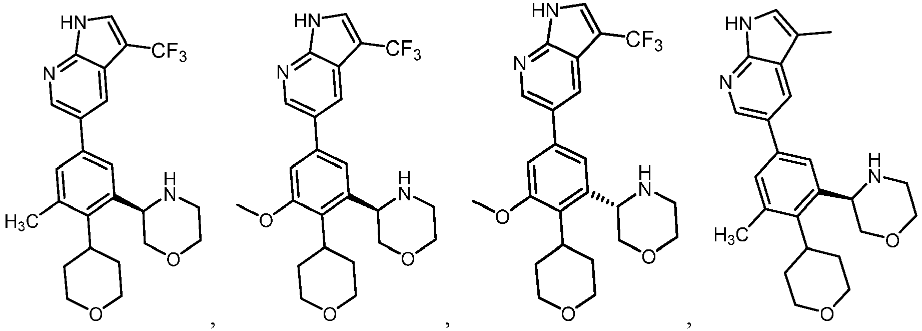 or

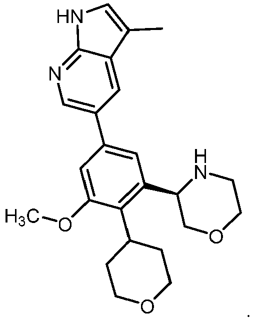

20. A pharmaceutical composition, comprising the compound or the pharmaceutically acceptable form thereof according to any one of claims 1-19, and one or more pharmaceutically acceptable carriers.

21. Use of the compound or the pharmaceutically acceptable form thereof according to any one of claims 1-19 or the pharmaceutical composition according to claim 20 in preparing a medicament for preventing and/or treating a disease or disorder mediated at least in part by HPK1.

22. The use according to claim 21, wherein the disease is a cancer.

23. The use according to claim 21, wherein the disease is selected from non-small cell lung cancer, small cell lung cancer, squamous cell carcinoma, head and neck cancer, oral cancer, pharyngeal cancer, thyroid cancer, esophageal cancer, stomach cancer, gastrointestinal stromal tumor, liver cancer, colon cancer, rectal cancer, villous adenoma of large intestine, breast cancer, breast ductal carcinoma, ovarian cancer, peritoneal cancer, endometrial cancer, corpus uteri cancer, cervical cancer, kidney cancer, renal pelvis cancer, prostate cancer, bladder cancer, neurofibromatosis, osteocarcinoma, brain cancer, testicular cancer, glioma, skin cancer, melanoma, cytoma and sarcoma, multiple myeloma, leukemia, non-Hodgkin's lymphoma or myelodysplastic syndrome.
